# EUROPEAN PATENT SPECIFICATION

(11) **EP 1 028 734 B1**
(45) Date of publication and mention of the grant of the patent: **22.02.2006**
(21) Application number: 98944936.8
(22) Date of filing: 28.09.1998
(51) Int. Cl.: A61K 31/58, C07J 71/00

(54) **USE OF SPECIFIC STEADY-STATE R-TYPE CALCIUM CHANNEL BLOCKERS**
VERWENDUNG VON SPEZIFISCHEN 'STEADY-STATE' CALCIUM-R-KANALBLOCKERN
UTILISATION DES INHIBITEURS SPECIFIQUES ET DE TYPE 'STEADY-STATE' DES CANAUX CALCIQUES DE TYPE R

(30) Priority: 30.09.1997 CA 2217088
(43) Date of publication of application: 23.08.2000
(73) Proprietor: Université de Sherbrooke, Sherbrooke, Québec J1K 2R1 (CA)
(72) Inventor: BKAILY, Ghassan, Sherbrooke, Québec J1J 3X6 (CA); D'ORLEANS-JUSTE, Pedro, Sherbrooke, Québec J1J 2H7 (CA); CALIXTO, Joao, B., CEP-88000-120 Florianopolis, SC (BR); YUNES, Rosendo, A., CEP-88025-400 Florianopolis, SC (BR)
(74) Representative: Boulinguiez, Didier
(86) International application number: PCT/CA1998/000908
(87) International publication number: WO 1999/016449

(56) References cited:
- EP-A- 0 359 310
- NEVES P C; NEVES M C; CRUZ A B; SANT'ANA A E; YUNES R A; CALIXTO J B: "Differential effects of Mandevilla velutina compounds on paw oedema induced by phospholipase A2 and phospholipase C. " EUROPEAN JOURNAL OF PHARMACOLOGY, vol. 243, no. 3, 1993, pages 213-9, XP002092176
- BENTO, EDSON S.; CALIXTO, JOAO B.; HAWKES, GEOFFREY E.; PIZZOLATTI, MOACIR G.; SANT'ANA, ANTONIO E. G.; YUNES, ROSENDO A.: "The structure of velutinol A is (15R,16R,20S)-14,16:15,20:16,21-triepoxy-1 5,16-seco-14.beta.,17.alpha.-pregn-5-ene-3 .beta.,15-diol. A combined quantitative Overhauser effect and molecular modeling study" J. CHEM. SOC., PERKIN TRANS. 2, no. 7, 1996, pages 1359-1366, XP002092177
- BEYER: LEHRBUCH DER ORGANISCHEN CHEMIE, 1981, page 658 XP002092178

## Description

### FIELD OF THE INVENTION

The present invention relates to Ca²⁺ channel blockers and more particularly to the R-type Ca²⁺ channel blockers. More specifically, the invention relates to Ca2+ channel blockers activity of *Mandevilla velutina* and *Mandevilla illustris.* The present invention also relates to the treatment of several pathologies that involve the nifedipine-insensitive but isradipine sensitive steady-state R-type Ca²⁺ channel and the use of steady-state R-type Ca²⁺ channel blockers in the treatment of these pathologies.

### BACKGROUND OF THE INVENTION

Sustained increase of intracellular Ca²⁺ or sustained Ca²⁺ overload (cytosolic, nuclear and mitochondrial) is known to be associated with many abnormal cell function including hypertension, artherosclerosis, hyperinsulinemia, diabetes Melitus type II, abnormal cell proliferation, cell-cell interactions, necrosis, ischemia/reperfusion, anythmias, platelet activation and aggregation as well as inflammation and asthma (Bkaily, 1994, Medical Intelligence Unit, CRC Press, Austin; Bkaily and Jacques, 1994, Kluwer Academic Publ., Boston; Bkaily et al:, 1994, Kluwer Academic Publ., Boston; Bkaily et al., 1997, Can. J. Physiol. Pharmacol. 75:652-666; Bkaily et al., 1997, Mol. Cell. Biochem. 172:171-194; Bkaily et al., 1997b, Drug Devel. Res. 42:211-222; Sowers et al., 1993, Am. J. Hypert. 6:302-307; 1994; Hurwitz et al., 1991, CRC Press, Boca Raton, Ann Arbor; Nagano et al., 1992, Kluwer Academic Publ., Boston; Anand et al., 1989, Kluwer Academic Publ. Boston; Dhalla et al., 1996, Kluwer Academic Publ. Boston; Karmazyn, 1996, Birkhauser Verlag. Basel, Boston; Curtis, 1993, Academic Press. London, San Diego; De Brum et al., 1996, Br. J. Pharmacol. 118:1597-1604; Foreman, 1993, Academic Press, London, San Diego; Furberg et al., 1993, Am. J. Hypert. 6:24S-29S; Holgate et al., 1993, Academic Press, London, Boston; Jacobs et al., 1993, Hypertension 21:308-314; Johnson et al., 1993, J. Clinic. Pharmacol. 35:484-492; Levy et al., 1994, Am. J. Med. 96:260-273; Raman et al., 1995, Am. J. Hypert. 8:197-200; Sperelakis et al., 1984, Martinus Nijhoff Publ. Boston; Standley et al., 1993; Wray et al., 1989, The New York Academy of Sciences, New York. Vol. 560). A wide variety of drugs has been tested against different types of Ca²⁺ channels (P, N, T and L) and the development of Ca²⁺ blockers has been concentrated on the L-type Ca²⁺ channel, which has never been shown to undergo any abnormal function in many diseases implicating sustained increase of intracellular Ca²⁺ ([Ca]ᵢ) or Ca²⁺ overload. Also, these drugs with the exception of isradipine (PN200-110, Lomir/Dynacirc), failed to block or prevent the sustained increase of [Ca]ᵢ, Ca²⁺ overload and necrosis. Recently, the presence of a steady-state nifedipine (L-type blocker)-insensitive but isradipine sensitive (dual L and R-type blocker) R-type (resting-type) Ca²⁺ channel that is voltage and Ligand-G protein-dependent has been reported (Bkaily et al., 1991, Elsevier, New York; Bkaily et al., 1992a, Am. J. Physiol. 262:H463-471; 1993, Br. J. Pharmacol. 110:519-520; 1993a, J. Mol. Cell. Cardiol. 25:1305-1316; 1995, J. Cardiovascul. Pharmacol. 26:303-306: 1996, Mol. Cell. Biochem. 154:113-121; 1997, Drug Develop. Res. 42:211-222; 1997a, Mol. Cell. Biochem. 172:171-194; 1997b, Can. J. Physiol. Pharmacol. 75:652-666; 1997d, Mol. Cell. Biochem, 170:1-8; 1997d, Mol. Cell. Biochem. 176:199-204; 1998, Mol. Cell. Biochem., 183:39-47; Bkaily, 1994a, In: Ionic channels in vascular smooth muscle. G. Bkaily edt. Molecular Biology Intelligence Unit, R.G. Lands Co. Austin.). This channel was responsible for maintaining the resting cytosolic and nuclear Ca²⁺ levels and its overstimulation by sustained depolarization or by permanent presence of some hormones such as insulin, ET-1, PAF, TNFα, PDGF, Bradykinin, or IL-1 induced sustained increase of [Ca]_{c} and [Ca]ₙ. (Bkaily et al., 1991, Elsevier, New York; 1993, *supra;* 1995, *supra;* 1996, *supra,* 1997a, *supra;* 1997b, *supra;* 1997c. *supra;* 1997d, *supra;* 1998, *supra;* Bkaily, 1994a, *supra;* Bkaily, 1994b, In: Membrane physiopathology. G. Bkaily edt. Kluwer Acad. Publ. Boston; Taoudi et al., 1995, J. Cardiovasc Pharmacol. 26:300-302). The important features that distinguish this channel from other Ca²⁺ channels are the sustained activity (as long as a depolarization or the pharmacological and physiological agonist is present) and the large number of disparate agonists that indirectly (via receptor-G proteins coupling) stimulate the channel.

Several reviews described the presence of various types of voltage-dependent Ca²⁺ channels in many cell types including heart, vascular smooth muscle (VSM) and vascular endothelial (VE) cells (Godfraind and Govoni, 1995; Bkaily, 1994b, *supra;* Orallo, 1996, Bkaily et al.. 1997a, *supra).* Among these different types of Ca²⁺ channels, the resting membrane potential steady-state voltage-dependent R-type (for resting) Ca²⁺ channel was first reported by the group of Bkaily et al. (Bkaily et al., 1991, *supra;* 1992, *supra;* Bkaily, 1994, *supra).* Later, the group of Tsien (Zhang et al., 1993, Neuropharmacol 32:1075-1088; Randall et al., 1997, Neuropharmacol 36:879-893) described a dihydropyridine resistant type Ca²⁺ channel also named R-type (for resistant).

The steady-state R-type Ca²⁺ channel in human VSM and VE cells was reported to possess a nearly 24 pS single channel conductance (in 110 mM Ca²⁺) (Bkaily et al., 1997a, *supra).* This type of channel was shown to be responsible for determining, under normal conditions, the resting tension of VSM cells and secretions by VE cells (Bkaily et al., 1991, *supra;* Bkaily et al., 1992, *supra;* Bkaily et al., 1993, *supra;* Bkaily et al., 1995, *supra;* Bkaily et al., 1996; Bkaily et al., 1997a; Bkaily et al., 1997b; Claing et al., 1994, Br. J. Pharmacol. 112:1202-1208; Taoudi-Benchekroun et al., 1995, J. Cardiovasc. Pharmacol. 26:300-302). This type of Ca²⁺ channel is known to be insensitive to nifedipine and inorganic L-type Ca²⁺ channel blockers such as cobalt, cadmium and Mn²⁺ and the T-type Ca²⁺ blocker, nickel (Bkaily, 1994, *supra).* However, it is blocked by PN200-110 (isradipine) which is also known to block the L-type Ca²⁺ channel (Bkaily et al., 1992, *supra;* Bkaily et al., 1997a, *supra*)*.* Unlike T and L-type Ca²⁺ channels, the R-type Ca²⁺ channel was not regulated by second messengers such as cAMP, cGMP and protein kinase C and neither by ATP (Bkaily, 1994, *supra).* This type of channel was reported to be indirectly stimulated by insulin, PAF, ET-1 and bradykinin via stimulation of a PTX and CTX sensitive G-protein(s) (Bkaily, 1994a, *supra;* 1991, *supra;* 1992, *supra;* 1995; 1996, *supra;* 1997a, *supra;* 1997b, *supra;* 1997c, *supra;* 1998, *supra)* and to contribute to a sustained elevation of cytosolic ([Ca]_{c}) and nuclear ([Ca]ₙ) Ca²⁺. These indirect R-type Ca²⁺ channel stimulators such as PAF induced elevation of [Ca]_{c}, and [Ca]ₙ by increasing the probability of opening of the channel, and thus allowing longer influx of Ca²⁺ through the sarcolemmal membrane (Bkaily et al., 1997a, *supra).*

Since PN200-110 was found to be the only available compound to depress the R-type Ca²⁺ channel and since this Ca²⁺ blocker is known to affect other types of Ca²⁺ channels, there remains a need to develop specific and potent steady-state R-type Ca²⁺ channel blockers.

The steady-state R-type Ca²⁺ channels are distributed in a non-homogenous fashion, similarly to some other receptors. This type of channel seems to have no inactivation gate and it is highly selective for Ca²⁺ ions. The R-type Ca²⁺ channel is highly voltage-dependent but could be stimulated by receptors whose activation is coupled to a specific PTX and CTX-sensitive G-protein(s) (Bkaily et al., 1998, *supra).* Thus, if the R-type Ca ²⁺ channel is fully activated via a receptor dependent pathway, it may appear as a receptor operated Ca²⁺ channel. Moreover, if the R-type channel is fully activated by voltage, receptor stimulation does not further modulate its function and appears as a pure voltage-dependent channel (Bkaily, 1994, *supra;* 1997a, *supra;* 1997c, *supra).* Since T- and L-type Ca²⁺ channels are rapidly inactivated during sustained voltage or pharmacological stimulation, these types of channels can only contribute to the inset stimulation. However, the R-type Ca²⁺ channel will contribute to both inset and sustained elevation of cytosolic and nuclear free Ca²⁺, seen in normal and pathological conditions, depending on the function of the studied cell type. Hence, this type of channel, under normal physiological situations contributes to the resting Ca²⁺ influx responsible for determining the resting cytosolic and nuclear Ca²⁺ that modulate resting tension, secretion, protein synthesis and mitosis. In working muscle cells, such as heart cells, the normal physiological function of this channel at the sarcolemmal membrane level, is to maintain normal resting cytosolic Ca²⁺ level. However, at the nuclear membrane levels, this channel seems to be implicated in maintaining normal resting nucleoplasmic Ca²⁺ levels (near 300 nM) (Bkaily et al., 1997a, *supra;* 1997b, *supra).*

During excitation-contraction coupling, the R-type Ca²⁺ channel is implicated in regulatingCa²⁺ wave propagation initiated, by Ca²⁺ influx through the opening of the T- and L-type Ca²⁺ channels and the subsequent large Ca²⁺ release from the SR by attenuating the cytosolic Ca²⁺ wave amplitude, by allowing Ca²⁺ influx through the nuclear membrane and thus permitting a smooth contraction and relaxation. The subsequent release of the uptaken Ca²⁺ permits the maintainance of Ca²⁺ waves and slow relaxation and propagation of the waves to neighboring cells, most likely through gap-junctions and in this manner, allowing synchronization of contraction of ventricular cells (Lopez et al., 1995, Biochem Biophys Res Commun 214:781-787; Bkaily et al., 1996, *supra;* 1997a, *supra).* The fact that cytosolic Ca²⁺ waves cannot be completely absorbed by the nucleus is due to the maximum Ca²⁺ buffering capacity of the nucleus which is shielded from variations in cytoplasmic Ca²⁺, perhaps by gating mechanisms in the perinuclear envelope once its maximum capacity is reached (Bumier et al., 1994, Am J Physiol 266:C1118-C1127; Bkaily, 1994, *supra;* 1996, *supra;* 1997a, *supra,* 1997b, *supra).*

Recent published results also showed that in secretory cells such as VE and VSM cells, tonic secretion or contraction is mainly, if not only due to the activation of sarcolemmal R-type Ca²⁺ channels. It was further shown that in VSM and excitable cells (VE cells do not possess T- or L-type Ca²⁺ channels) the T and/or the L-type Ca²⁺ channel activation, serves as a turbo Ca²⁺ influx mechanism in order to rapidly bring the Ca²⁺ level up to the threshold level for contractile elements and to pre-overload the nucleoplasm with Ca²⁺, enabling cytosolic accumulation of Ca²⁺ and maintain tension. Thus, overstimulation of the R-type Ca²⁺ channels may highly contribute to cytosolic and nuclear Ca²⁺ accumulation that could be considered in many cases as the first and in all cases the final pathological consequence of several diseases such as hypertension, atherosclerosis, abnormal conduction, arythmias, fibrillation, and of remodelling, proliferation and apoptosis. For these reasons, targeting the sarcolemmal nuclear membrane R-type Ca²⁺ channels with a selective depressor blocker, or targeting receptors that indirectly modulate this type of channel at the sarcolemmal, or mainly at the nuclear membrane level, would constitute without any doubt, a major therapeutical pathway for a new generation of Ca²⁺ channel and Ca²⁺ entry blockers.

For example, the sustained activation of the R-type channel by insulin may explain in part "syndrome X ", the hypertension, hyperglycemia, dyslipidemia, vascular.smooth muscle proliferation and end organ damage associated with non-insulin-dependent diabetes mellitus (NIDDM) and obesity-induced hypertension. Also, the sustained increase in [Ca]_{c} and mainly [Ca]ₙ mediated by the stimulation of the R-type Ca²⁺ channel could contribute to the expression of oncogenes and to the proliferation of malignant cells as well as to stimulation of TNFα; PAF which would lead to septic shock. The finding that Lomir/Dynacirc (but none of the other L-type Ca²⁺ antagonists) is unique in depressing the overstimulation of the R-type Ca²⁺ channel permits the identification and characterization of this type of Ca²⁺ channel. Non published results in two human osteoblast cancer lines (MG63 and FAOS-2) clearly showed that Lomir/Dynacirc (10⁻⁸M) reduced spontaneous cell proliferation and blocked hypertension and ET-1 plasma elevation associated with cyclosporin A treatment in allograft transplant. In contrast, an L-type Ca²⁺ blocker, nifedipine (10⁻⁶M) had no effect. A role for the R-type Ca²⁺ channel and Lomir/Dynacirc in human cancer is suggested by the above findings and supported by the finding of reduced cancer rates in the Lomir/Dynacirc treated group of the MIDAS study. The identification of a potent and specific antagonists may hold the possibility of a new therapeutic target for novel medications. The novel R-type Ca²⁺ channel may also prove important in dissecting differential signalling pathways in immune cells. The evaluation of these mechanisms leads to R-type blockade as a therapeutic tool for specific intervention in graft rejection, autoimmune diseases, asthma and septic shock.

A recent report in patients with type I and type II Raynaud's phenomenon (pain and numbness in the fingers, which in some subjects can be complicated by skin ulcers) showed that Lomir/Dynacirc significantly reduced the elevated plasma concentration of ET-1 level, frequency, severity, and disabling nature of acute attacks of Raynaud's phenomenon (La Civita et al., 1996, Clinic. Drugs Invest. 11:S126-31). The decrease of the elevated ET-1 circulating level by Lomir/Dynacirc is due to the blockade of the R-type Ca²⁺ channel which reverses the sustained increase of [Ca]_{c} and [Ca]ₙ, thus, reducing the autocrine and self perpetuating secretion of mitogenic factors such as ET-1, PAF and TNFα. A blockade of the elevated autocrine and self perpetuating secretion of mitogenic factors by cancer cells may in turn contribute to reduction and even blockade of expression of oncogenes and proliferation of these cells.

The use of Sandimmune is known to produce potentially serious side effects such as renal impairment and hypertension. These side effects will restrict Sandiummune's use in autoimmune indications such as psoriasis and rheumatoid arthritis. The renal impairment and hypertension are attributable to altered renal hemodynamics induced by Sandimmune. The L-type Ca²⁺-channel blockers have been used successfully to treat hypertension and renal impairment. The benefits of the Ca²⁺-channel blockers have been attributed to their effects on renal hemodynamics specifically dilation of the afferent renal arteriole.

Data indicate that the dual R-and L-type Ca²⁺ channel blocker isradipine (but not a pure L-type blocker) may correct the vasoconstriction at both the afferent and efferent renal arterioles. The advantage of dilation of the afferent and efferent arterioles is a correction of renal blood flow and glomerular filtration without an increase in filtration fraction. Filtration fraction is an indicator of filtration pressure. An increase in filtration pressure could increase the likelihood of developing glomerulonephritis and eventual renal failure.

The potential benefit of blockade of the R-type Ca²⁺ channel by Lomir/Dynacirc on filtration pressure is supported by the existing literature. For example Grossman et al. (1991, Am. J. Cardiol. 68:65-70) in a 3-month study with Lomir/Dynacirc showed that filtration fraction remained constant. The filtration fraction remained constant despite the increase in glomerular filtration rate and renal blood flow. Vascular resistance was also reduced by the 3-month treatment with Lomir/Dynacirc.

A favourable effect on filtration fraction has been corroborated in transplant patients (Berg et al., 1991, Nephrology, Dialysis, Transplantation. 6:725-30). These investigators showed that filtration fraction was reduced by Lomir/Dynacirc while renal blood flow increased.

The R-type Ca²⁺ channel has also been identified and characterised in vascular smooth muscle cells isolated from human renal arteries (Bkaily et al., 1991, *supra).*

*Mandevilla velutina* is a native Brazilian plant used in folk medicine to treat snake bites and as an anti-inflammatory agent. Some non-peptidic compounds extracted from this plant block bradykinin and related kinins action. It shows potent analgesic and anti-inflammatory activities (Calixto et al., 1987, *supra).*

Since 1985, Calixto's group has worked on extracts of *Mandevilla velutina* (MV) and claimed that some of the extracts (such as MV8608) had antagonistic properties against the effect of bradykinin (BK). The compound MV8608 has been characterized in 1987 (Calixto et al., Br. J. Pharmacol. 91:199-204). It has been found to be selective in its ability to inhibit the contraction of rat uterus induced by BK. The previous work made by Calixto's group as well as others on extracts of Mandevilla species have always focused on compounds which have a presumed action at the BK receptor site.

In a review article published after 1990, Calixto's group (Calixto and Yunes, 1991, Mem. lnst. Oswaldo 86:195-202, supl. 2) mentioned that the compounds MV8608 had a pregnane structure. It is further mentioned that MV8608 is an aglycone compound (without any sugar). No specific structure is shown in this review article concerning MV8612. This review is a compendium of data, characteristics, and properties of MV8608 and MV8612 in numerous systems responding to BK (therefore not limited to the effect of BK on rat uterus). Again, it may be deducted from this publication that the Calixto group of researchers have focused their study on the search of a ligand which is a BK receptor antagonist. MV8612 has been retained as a good candidate because it best corresponds to established receptor classification criteria (a fairly good pA2, competition curve whose slope does not differ from one and selectivity). Of note, Calixto publication (Calixto and Yunes, 1991, *supra)* does not teach or suggest that MV8612 may have an action which is aimed at the receptor directly. Although on certain systems the effect of MV8612 has been shown to be non-selective, no explanation on this lack of selectivity toward BK has been provided. Therefore, this publication does not teach or suggest any role of MV8608 and MV8612 as calcium channel blockers.

Other compounds isolated from *Mandevilla Pentlandia,* have also claimed an anti-BK activity, (patent application of Proctor and Gamble Co., EP 0/359310). Furthermore, other *Mandevilla* extracts, particularly from *Mandevilla Illustris* have been shown to have physiological antagonist activity against BK. Indeed, all the compounds obtained from *Mandevilla* species are described as compounds having anti-BK activity. Strikingly, all such descriptions fail to teach or mention the specific site of action of these compounds, and while they lack selectivity, they are deemed to be useful for treating pathologies and conditions involving bradykinin (inflammation, smooth muscle contraction, pain, hypotension, etc.).

The art teaches that a non-specific inhibitor of a calcium channel such as isradipine, which has an effect on calcium channel types L and R, reduces or abolishes the effect of hormones like insulin and PAF (platelet-activating factor), ET-1 and BK which effect is absent when using nifedipine (a L-channel blocker). Nevertheless, the art is indicative of the contribution of the Retype calcium channel in the effect of insulin, PAF, ET-1 and BK.

There thus remains a need to assess the specificity of MV8608 and MV8612 by identifying their direct or indirect effects on Ca²⁺ homeostasis. More broadly, there remains a need to verify whether MV8608 and MV8612 are as non-specific as isradipine. More particularly, there remains a need to assess the activity of these compounds on the R-type Ca²⁺ channel, as well as T, L Ca²⁺ channels and the fast Na⁺ and delayed outward K⁺ channels.

In spite of the recent discovery of the R-type Ca²⁺ channel, there is a definite need for a new generation of class of drugs to treat overstimulation of R-type Ca²⁺ channel-associated diseases for the following reasons:
1. There is no drug approved for the treatment of diseases or conditions in which a sustained elevation of [Ca]_{c'} [Ca]ₙ or R-type Ca²⁺ channell blocking is encountered; and
2. There remains a definite need for the identification of drugs which are more specific, show less side effects and have a wider therapeutic value, for the treatment of hypertension, artherosclerosis, inflammation, septic shock, arthritis, asthma, cancer, pain, diabetes type II and ischemia-reperfusion, hyperventilation and high circulating ET-1 level.

The present invention seeks to meet these and other needs.

### SUMMARY OF THE INVENTION

The invention concerns the pharmaceutical use of saponin-like compounds isolated from *Mandevilla* species which act as specific R-type Ca²⁺ channel blockers. The structure of these compounds is shown in claim 1. The R-type Ca²⁺ channel blocker are obtained from *Mandevilla* species and more particularly from *Mandevilla Velutina* and *Mandevilla Illustris.* In a preferred embodiment, the invention relates to the use of specific R-type Ca²⁺ channel blockers MV8608 and MV8612.

The invention in addition relates to pharmaceutical compositions comprising specific R-type Ca²⁺ channel blockers obtained from *Mandevilla* species to treat and/or prevent diseases or conditions associated with a sustained elevation of [Ca]_{c} , [Ca]ₙ , or R-type Ca²⁺ channel bloking and/or cytosolic and nuclear Ca²⁺ accumulation, together with a suitable pharmaceutical carrier. More specifically, the present invention relates to such pharmaceutical compositions to treat or prevent hypertension, artherosclerosis, hyperinsulinemia, diabetes Melitus type II, abnormal cell proliferation, cell-cell interactions, necrosis, ischemia/reperfusion, arrhythmias, platelet activation and aggregation, inflammation, asthma, abnormal conduction, fibrillation, remodelling, proliferation, antibacterial proliferation, septic shock, apoptosis, hyperglycemia, dyslipidemia, vascular smooth muscle proliferation and end organ damage associated with non-insulin-dependent diabetes mellitus (NIDDM), and obesity-induced hypertension, cancer, renal impairment, renal failure, arthritis pain, hyperventilation, and high circulating ET-1 level.

The invention further relates to the use of a family of R-type Ca²⁺ channel blockers which virtually only affect the overstimulation thereof, without significantly affecting the basal activity thereof.

In addition, the invention relates to the use of a family of specific R-type Ca²⁺ channel blockers which reduce the over-basal frequency of the R-type Ca²⁺ channel.

The R-type CA²⁺ channel blockers may be used in methods of preventing or treating a warm blooded animal having a disease or condition demonstrating a sustained elevation of calcium through an effect on a R-type Ca²⁺ channel, comprising an administration of an effective amount of specific R-type Ca²⁺ channel blocker, in accordance with the present invention, together with a pharmaceutically acceptable carrier. The invention also relates to pharmaceutical compositions for such methods of prevention or treatment.

The invention further enables methods of treatment of a warm blooded animal in need of this treatment comprising an administration of a therapeutically effective amount of a R-type Ca²⁺ channel blocker obtained from *Mandevilla* species, together with a pharmaceutically acceptable carrier. More specifically, the present invention enables a treatment of a warm blooded, animal demonstrating a sustained elevation of [Ca]_{c}, [Ca]ₙ, R-type Ca²⁺ channel blocking, and/or cytosolic and nuclear Ca²⁺ accumulation, comprising an administration of a therapeutically effective amount of a R-type Ca²⁺ channel blocker obtained from *Mandevilia* species, together with an acceptable pharmaceutical carrier.

Before the present invention, the properties of the MV compounds towards the calcium channel type R disclosed had not been taught or suggested.

Furthermore, before the present invention, it was unknown that in organ transplants, in an animal model such as rabbit, that there is an increase of circulating ET-1 and a decrease of blood flow that were not prevented by cyclosporin-A and by the pure L-type blocker nifedipine. In contrast, the dual R- and L-type Ca²⁺ blocker Lomir/Dynacirc (isradipine or PN200-110), restored ET-1 and blood flow levels in cyclosporin-A treated and transplanted animals. Thus, the present invention shows that some undesired side effects associated with cyclosporin-A treatment for example, are attributable to an overstimulation of R-type Ca²⁺ channels.

While a blockade of the elevated autocrine and self perpetuating secretion of mitogenic factors by cancer cells could have been suggested from the results by La Civita et al. (1996, *supra),* prior to the present invention, it was unknown whether this proliferative effect was related to the R-type Ca²⁺ channel and whether it was a common mechanism in cancer and tumor cells. In accordance with the present invention, preliminary results using several types of human cancer cell lines seem to highly suggest that the proliferative effect of overstimulation acts through the R-type Ca²⁺ channel and is indeed a common mechanism in cancer and tumor cells. Thus supporting the results for the R-type Ca²⁺ channel and its blockade by Lomir/Dynacirc in human cancer as mentioned above in the Lomir/Dynacirc group of the MIDAS cohort (a non-specific R-type and C-type channel blocker). Thus, the present invention relates to a method of decreasing proliferation of cancer and tumor cells comprising an incubation thereof with an effective amount of a R-type Ca²⁺ channel blocker. More particularly, the present invention relates to a method of decreasing proliferation of cancer and tumor cells comprising an incubation thereof with an effective amount of a R-type Ca²⁺ channel blocker obtained from *Mandevilla* species and even more particularly of MV8608 and MV8612. In addition, the present invention provides a method for preventing non-properly regulated autocrine secretion using the specific R-type Ca²⁺ channel blockers of the present invention (and pharmaceutical compositions therefor).

Further, prior to the present invention, it was unknown that a combination therapy of certain drugs which display potentially serious side effects such as renal impairment and hypertension (i.e. Sandimmune) with R-type Ca²⁺ channel blockers could block or relieve these side effects. Furthermore, data using the rabbit (see example 9) indicate the presence of the R-type Ca²⁺ channel in both the afferent and efferent renal arterioles. It remains to be seen whether the R-type Ca²⁺ channel is present in renal arterioles. The instant invention along with that of all the published papers since 1991 by Bkaily et al. provides the rationale for an advantage of R-type Ca²⁺ channel blocking agent such as Lomir/Dynacirc over other pure L-type Ca²⁺ channel blockers in the long-term protection of renal function. The contribution of endothelin to the Sandimmune induced side effects of renal impairment and hypertension has now been assessed. The results highly suggest that blockade of the R-type Ca²⁺ channel by Lomir/Dynacirc blocks the elevated circulating endothelin level induced with the Sandimmune drugs (Bkaily et al., data not shown).

The compounds of the present invention, in contradistinction to nifedipine and isradipine, do not induce hypertension in a normal patient.

From the wide variety of L-type Ca²⁺ channel blockers such as nifedipine, nicardipine, Diltiazem, Clentiazem, Verapamil, D600 and D888 none were found to block the R-type Ca²⁺ with the exception of isradipine (Lomir/Dynacirc) (data not shown). This later compound was found to block the R-type Ca²⁺ channel with an ED₅₀ near (10⁻⁸ M), the T-type Ca²⁺ channel with an ED₅₀ near (10⁻⁷M), the L-type Ca²⁺ channel with an ED₅₀ near (10⁻⁶M) and the fast Na⁺ channel with an ED₅₀ near (10⁻⁵M) (data not shown). Thus, although the R-type Ca²⁺ channel blocker isradipine seems to be a potent R-type Ca²⁺ blocker, it is not highly specific. On a clinical point of view, this drug seems to be more potent and to possess less side effects than other dihydropyridines (DHP) derivative L-type Ca²⁺ channel blockers. The difference between isradipine and other DHPs compounds could be due to the potential of the former, to act as a R-type Ca²⁺ blocker.

It has now been demonstrated that similarly to PAF, insulin and ET-1, bradykinin (BK) also induced a sustained increase of [Ca]ᵢ which was mainly nuclear and was due to the stimulation of the R-type Ca²⁺ channel in human aortic endothelial and vascular smooth muscle, as well as chick and heart ventricular cells. The stimulation of R-type Ca²⁺ channel by BK was due to the kinin activation of the B₁-receptor.

In accordance with the present invention, there is therefore provided a pharmaceutical use of a compound having the general formula of MV8612 analogs VIIA and VIIB, saponin-like derivatives thereof and pharmaceutically acceptable salts thereof.

The saponin-like compound has the general formula EST wherein E and S define a saponin oligosugar portion and T defines a steroid-like portion; wherein T is a pregnane-3β-ol derivative.

In accordance with the present invention there is also provided a pharmaceutical composition for treating or preventing overstimulation of R-type Ca²⁺ channels associated disease or condition in a warm blooded animal comprising at least one compound of general formula EST, together with a pharmaceutically acceptable carrier.

As well, in accordance with the present invention, there is also provided a pharmaceutical composition for blocking or relieving side effects of a drug which overstimulate R-type Ca²⁺ channels comprising at least one compound of general formula EST, together with a pharmaceutically acceptable carrier.

The present invention, enables a method for specifically inhibiting overstimulation of a R-type Ca²⁺ channel in a warm blooded animal comprising an administration of an effective amount of a compound of general formula EST,together with a pharmaceutically acceptable carrier.

### BRIEF DESCRIPTION OF THE DRAWINGS

Having thus generally described the invention, reference will now be made to the accompanying drawings, showing by way of illustration a preferred embodiment thereof, and in which:
Figure 1 shows the structure of compound of MV8608 and its isomers; as well as the structure of 5-pregnane-3β-ol-20 (compound V);
Figure 2 shows the structure of MV8612 (analogs VIIA and VIIB);
Figure 3 shows ¹HNMR of compound MV8612 isolated from *Mandevilla Velutina;*
Figure 4 shows ¹³CNMR of compound MV8612 isolated from *Mandevilla Velutina;*
Figure 5 shows COSY (correlated spectroscopy) of compound MV8612 isolated from *M. Velutina;*
Figure 6 shows HETCOR of compound MV8612 isolated from *M. Velutina;*
Figure 7 shows HPLC chromatogram of compound MV8608 isolated from *M. Velutina;*
Figure 8 shows HPLC chromatogram of compound MV8612 isolated from *M. Velutina;*
Figure 9 shows GC chromatogram of compound MV8608 isolated from *M. Velutina;*
Figure 10 shows GC chromatogram of compound illustrol isolated from *M. Illustris;*
Figure 11 shows the absence of the effect of (10⁻⁷M) of MV8608 on the TTX-sensitive fast Na⁺ current in single heart cell;
Figure 12 shows the relative weak depressing effect of (10⁻⁹M) and (10⁻⁷M) of MV8608 on the T-type Ca²⁺ current in heart cells;
Figure 13 shows the relative weak depressing effect of (10⁻⁹M) and 5 x (10⁻⁷M) of MV8608 on the L-type Ca²⁺ current in heart cells;
Figure 14 shows that intra patch pipette application of (10⁻⁷M) of MV8608 decreased the R-type Ca²⁺ channel amplitude and probability of opening and that extra patch pipette application of MV8608 (10⁻⁷M) increased the time of opening duration followed by transient decrease of the amplitude of the R-type Ca²⁺ channel;
Figure 15 shows the blockade by MV8608 (10⁻⁹M) of the sustained depolarization induced by sustained increase of total [Ca]ᵢ via activation of the R-type Ca²⁺ channels in chick and human heart cells;
Figure 16 shows the absence of the effect of nifedipine (10⁻⁶M) on ET-1 (10⁻⁹M), sustained depolarization (KCI, 30 mM) and PAF (10⁻⁹M) induced sustained increase of [Ca]ᵢ via the activation of the R-type Ca²⁺ channel and the blockade of this sustained increase by MV8608 (10⁻⁹M) in heart cells;
Figure 17 shows the blockade by MV8608 (10⁻⁹M) and the absence of the effect of nifedipine (10⁻⁶M) on the sustained depolarization (30 mM), and PAF (10⁻⁹M) induced sustained increase of [Ca]ᵢ via activation of the R-type Ca²⁺ channel in human heart cells;
Figure 18 represents histograms showing MV8608 (10⁻⁹M) blockade of sustained increase of [Ca]ᵢ (in presence of nifedipine (C+N) induced by sustained depolarization (KCI, 30 mM) and PAF (10⁻⁹M) stimulation of the R-type Ca²⁺ channel in human heart cells;
Figure 19 represents histograms illustrating MV8608 (10-⁹M) blockade of the sustained increase of [Ca]ᵢ, induced by sustained depolarization (in presence or absence of nifedipine), PAF and ET-1 stimulation of R-type Ca²⁺ channel in chick heart cells;
Figure 20 shows the blockade by MV8608 (10⁻⁹M) of bradykinin (10⁻⁶M) induced sustained increase of [Ca]ᵢ (in presence of (10⁻⁶M) nifedipine) via activation of the R-type Ca²⁺ channels in chick heart cells, human heart cells and rabbit aortic vascular smooth muscle cells;
Figure 21 represents histograms showing the MV8608 (10⁻⁹M) blockade of bradykinin (BK,10⁻⁶M) and PAF (10⁻⁹M) induced sustained increase of [Ca]ᵢ via activation of the R-type Ca²⁺ channels in rabbit aortic vascular smooth muscle cells;
Figure 22 shows a typical example of the decrease of basal sustained increase of [Ca]ᵢ by MV8608 (10⁻⁹M) in freshly isolated human aortic endothelial cells and the blockade by MV8608 (10⁻⁹M) of PAF (10⁻⁹M) induced sustained increase of [Ca]ᵢ, via activation of the R-type Ca²⁺ channels in freshly isolated human aortic vascular smooth muscle cells;
Figure 23 represents histograms showing that increasing the concentration of PAF (10⁻⁷M) required high concentration of MV8608 (10⁻⁶M) for blockade of PAF induced sustained increase of [Ca]ᵢ via activation of the R-type Ca²⁺ channels in human aortic vascular smooth muscle cell lines;
Figure 24 represents histograms showing that in double-perfused mesenteric bed of the rat, MV8608 (1 µM) but not Illusteol (1 µM) blocked PAF but not ACh and Angll induced arterial vasodilatation and venconstriction;
Figure 25 shows the time course decreases of the TTX-sensitive fast Na⁺ current in chick heart cells by (10⁻⁸M) of MV8612;
Figure 26 shows the time course blockade of the L-type Ca²⁺ current by high concentration (10⁻⁷M) of MV8612 in human heart cells;

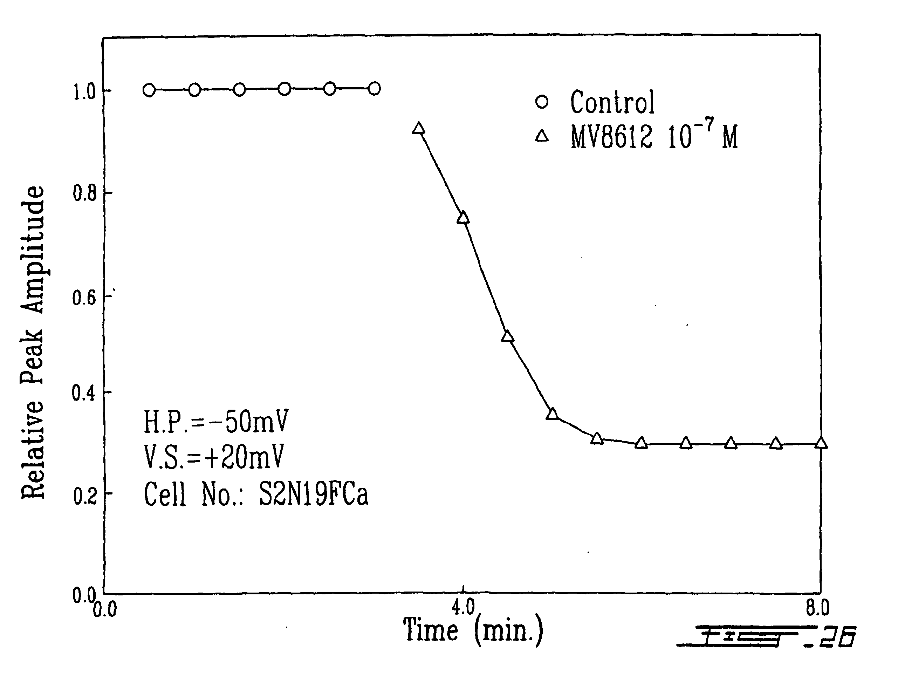

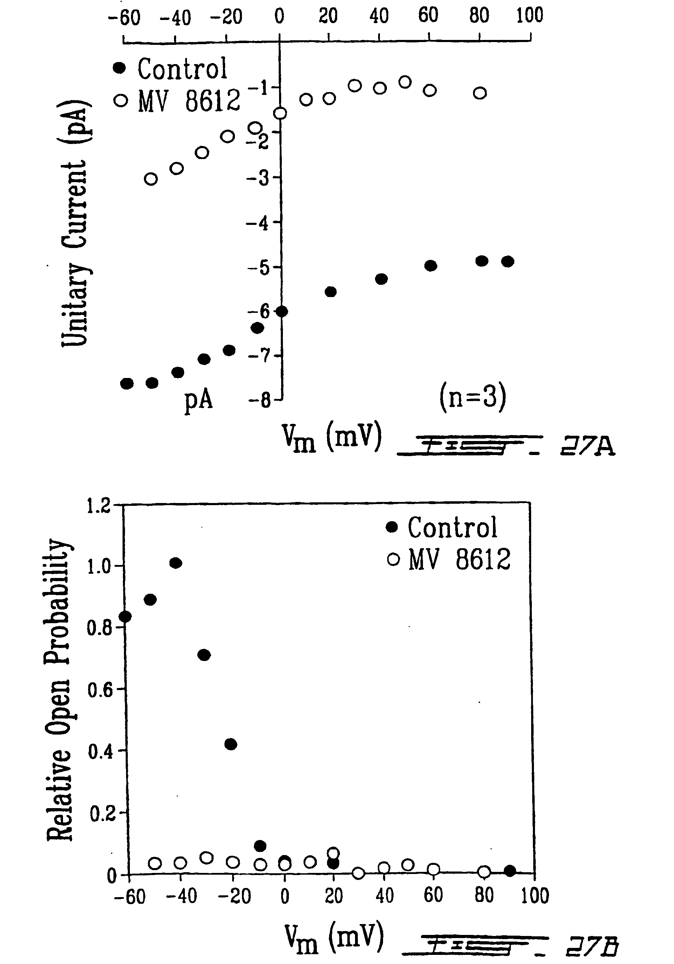

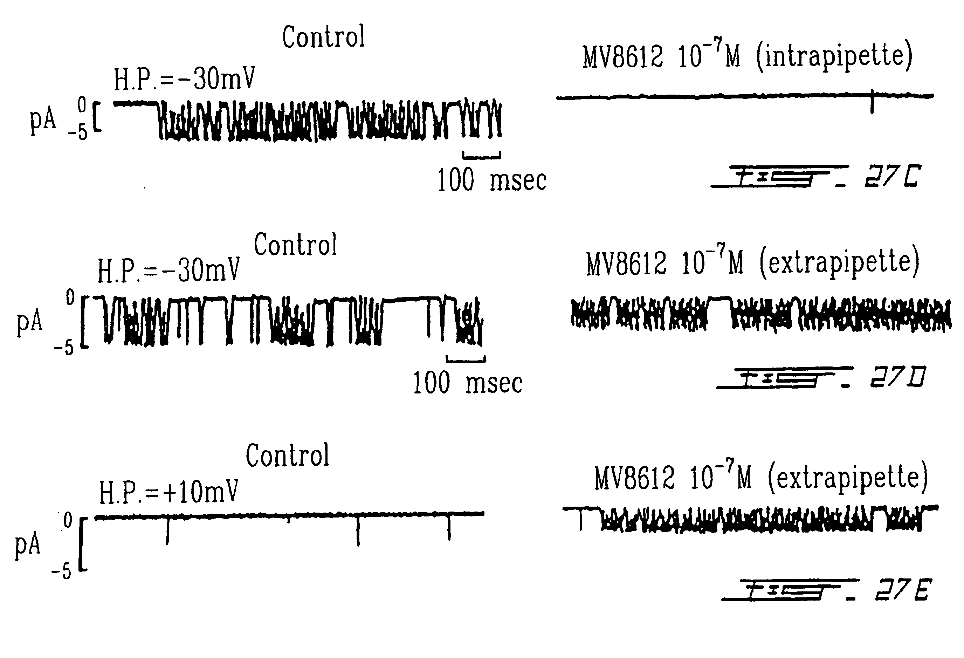

Figure 27 shows graphs and cell attached single R-type Ca²⁺ channel recording (in presence of (10⁻⁶M) nifedipine) showing the decrease of the single channel current amplitude (panel A, current voltage relationship, n=3), probability of opening (panel B, open probability-voltage relationship, n=3) by 10⁻⁷M MV8612 application in the patch pipette and panel C, example of single channel current traces. Panels D-E show that application of MV8612 (10⁻⁹M) to extrapipette solution only induced a slight decrease of the R-type Ca²⁺ channel amplitude and largely increased the probability of opening of the channel. This demonstrates that MV8612 does penetrate to the cytosol and its effect at the cytosolic side of the channel is different from that at the outer side;
Figure 28 represents graphs showing that both MV8608 (10⁻⁸M) and MV8612 (10⁻⁸M) but not nifedipine (10⁻⁷M) significantly decreased the spontaneous proliferation of human aortic vascular smooth muscle cell line;

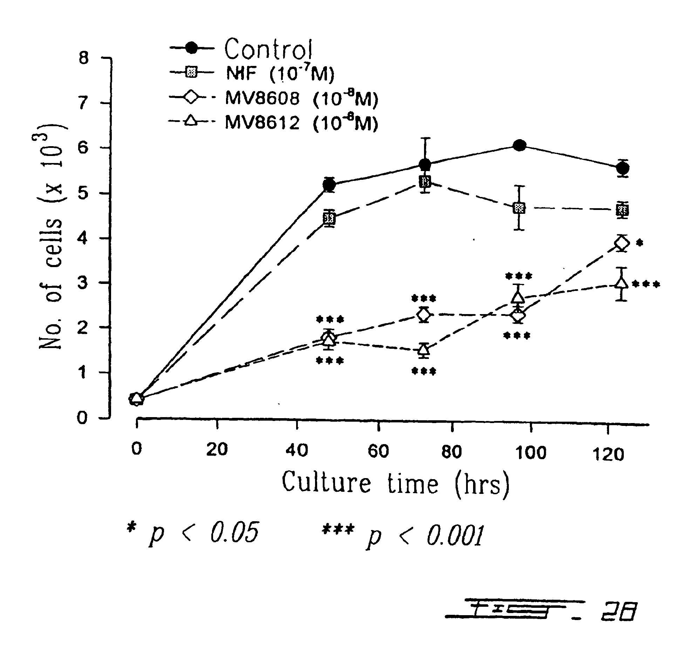

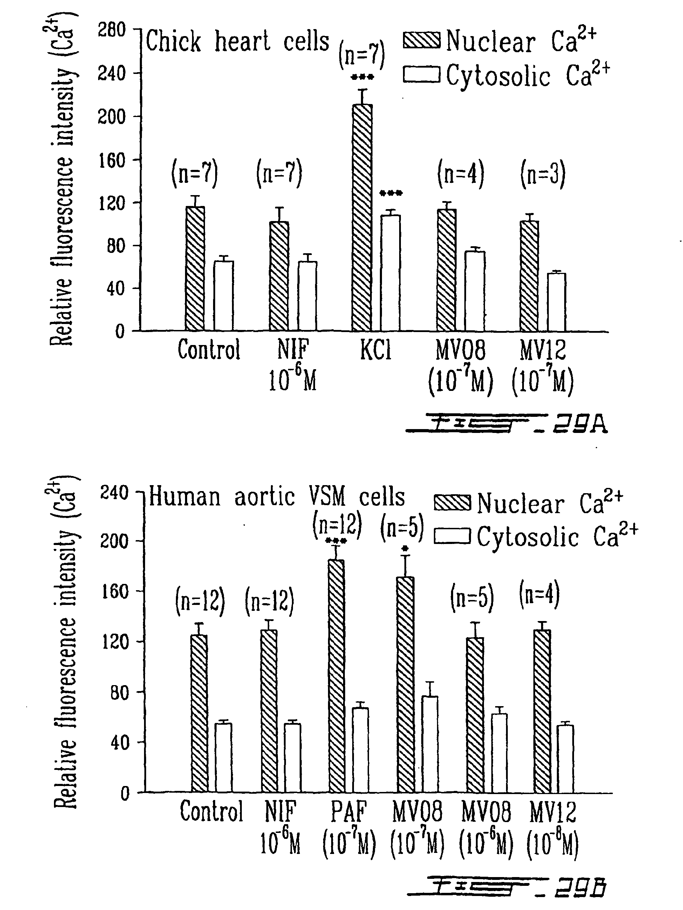

Figure 29 represents histograms showing that the L-type Ca²⁺ blocker, nifedipine did not affect basal cytosolic ([ ]_{c}) and nuclear ([ ]ₙ) free Ca²⁺ as well as the sustained depolarization and high PAF induced sustained increase of [Ca]_{c} and [Ca]ₙ. However, MV8608 and MV8612 blocked completely the sustained depolarization induced sustained increase of [Ca]_{c}, and [Ca]ₙ (panel A) in heart cells. Panel B shows that high concentration of PAF (10⁻⁷M) induced sustained increase of [Ca]_{c} and [Ca]ₙ is blocked by high concentration of MV8608 (10⁻⁶M) but normal concentration of MV8612 (10⁻⁸M);
Figure 30 is a 3-dimensional reconstitution showing the absence of effect of nifedipine and the blockade of the sustained increase of [Ca]_{c} and [Ca]ₙ induced by sustained depolarization and high PAF (10⁻⁷M) in chick heart (A) and human aortic vascular smooth muscle cell line (B);
Figure 31 represents histograms showing the preventive effect by MV8608 and MV8612 of sustained depolarization and high PAF (10⁻⁷M) induced sustained increase of [Ca]_{c} and [Ca]ₙ via stimulation of the R-type Ca²⁺ channel in chick heart cells and human aortic vascular smooth muscle cell line;
Figure 32 represents histograms showing the preventive effect by MV8612 (10-⁸M) of sustained depolarization (KCl 30 mM) and high PAF (10⁻⁷M) induced sustained increase of [Ca]ᵢ, via activation of the R-type Ca²⁺ channel in human aortic vascular smooth muscle cell line;
Figure 33 represents histograms showing the blockade by MV8612 (10⁻⁹M) of sustained depolarization and ET-1 (10⁻⁹M) induced sustained increase of [Ca]ᵢ via activation of the R-type Ca²⁺ channels in rabbit aortic vascular smooth muscle;
Figure 34 represents histograms showing the blockade by 10⁻⁹M MV8612 of the sustained depolarization, low PAF (10⁻⁹ M) and ET-1 (10⁻⁹M) induced sustained increase of [Ca]ᵢ via the activation of the R-type Ca²⁺ channels in chick heart cells;
Figure 35 shows the marked intrinsic hypotensive properties of the dual L- and R-type Ca²⁺ channel blocker isradipine (panel B) when compared to the pure L-type Ca²⁺ channel blocker, nifedipine (Panel A);
Figure 36 shows that pretreatment with MV8612 abolishes the bronchoconstrictive responses and the hypotensive effect of PAF in the anaesthetized guinea pig model;
Figure 37 shows the effect of subplantar injection of compound MV 8608 isolated from *Mandevilla velutina* on paw oedema caused by subplantar injection of des-Arg⁹-bradykinin (DABK) (A) in rats treated 24 h prior with LPS; bradykinin (BK, B) and for des-Arg⁹-bradykinin (C) and for bradykinin (D) in animals treated 30 days prior with BCG. Each group represents the mean of 4-5 animals and the vertical bars the S.E.M. The asterisks indicate the significance levels: * P < 0.05;
Figure 38 shows the effect of subplantar injection of MV 8608 isolated from *Mandevilla velutina* on paw oedema caused by subplantar injection of prostaglandin E₂ (PGE₂) (A), PAF- acether (PAF) (B), substance P (SP) (C) and ovalbumin (OVO) (D). Each group represents the mean of 4-5 animals and the vertical bars the S.E.M. The asterisks indicate the significance levels: * P < 0.05. Each group represents the mean of 4-5 animals and the vertical bars the S.E.M. The asterisks indicate the significance levels: *P < 0.05;
Figure 39 shows the effect of subplantar injection of MV 8608 isolated from *Mandevilla velutina* on paw oedema caused by subplantar injection of low dose of des-Arg⁹-bradykinin plus PAF (A) or prostaglandin E₂ (PGE₂) (B). Each group represents the mean of 4-5 animals and the vertical bars the S.E.M. The asterisks indicate the significance levels: *P < 0.05;
Figure 40 shows the effect of subplantar injection of MV 8608 isolated from *Mandevilla velutina* on paw oedema caused by subplantar injection of carrageenan (Cg) (A), dextran (DEX) (B), histamine (HIST) (C) and for serotonin (5-HT) (D). Each group represents the mean of 4-5 animals and the vertical bars the S.E.M. The asterisks indicate the significance levels: *P < 0.05;
Figure 41 shows the effect of subplantar injection of MV 8612 isolated from *Mandevilla velutina* on paw oedema caused by subplantar injection of bradykinin (BK) (A and C), des-Arg⁹-bradykinin (DABK) (B) and for tyr⁸-bradykinin (D). Experiments for DABK were carried out in animals treated with LPS 24 h prior. Each group represents the mean of 4-5 animals and the vertical bars the S.E.M. The asterisks indicate the significance levels: *P < 0.05;
Figure 42 shows the effect of subplantar injection of MV 8612 isolated from *Mandevilla velutina* on paw oedema caused by subplantar injection of prostaglandin E₂ (PGE₂ (A), PAF acether (PAF) (B), carrageenan (Cg) (C) and substance P (SP) (D). Each group represents the mean of 4-5 animals and the vertical bars the S.E.M. The asterisks indicate the significance levels: *P < 0.05. Each group represents the mean of 4 - 5 animals;
Figure 43 shows the effect of subplantar injection of MV 8612 isolated from *Mandevilla velutina* on paw oedema caused by subplantar injection of low dose of bradykinin plus CGRP (A), BK plus prostaglandin E₂ (PGE₂) (B); BK plus PAF C) or BK plus PGl₂ (D). Each group represents the mean of 4-5 animals and the vertical bars the S.E.M. The asterisks indicate the significance levels: *P < 0.05;
Figure 44 shows the effect of an intraperitoneal injection of MV 8612 isolated from *Mandevilla velutina* on rat paw oedema caused by subplantar injection of bradykinin in animals treated with cyproheptadine (A) or compound 48/80 (B). Each group represents the mean of 4 animals and the vertical bars the S.E.M. The asterisks indicate the significance levels: * P < 0.05;
Figure 45 shows a time-dependent antioedematogenic effect caused by subplantar injection of compound MV 8612 on bradykinin-induced rat paw oedema. Each group represents the mean of 4-5 animals and the vertical bars the S.E.M. The asterisks indicate the significance levels: *P < 0.05;
Figure 46 shows a dose-dependent antioedematogenic effect caused by co-injection of compound MV 8612 on bradykinin (BK, A), carrageenan (B). PAF (C and serotonin (D)-induced mouse paw oedema. Each group represents the mean of 7 animals and the vertical bars the S.E.M. The asterisks indicate the significance levels:* P < 0.05; ** P < 0.01;
Figure 47 shows a dose-dependent antioedematogenic effect caused by intraperitoneal injection of compound MV 8612 on bradykinin (BK, A), cellulose sulphate (B)-serotonin (5-HT, C) and histamine (Hist, D)-induced mouse paw oedema. Each group represents the mean of 5-6 animals and the vertical bars the S.E.M. The asterisks indicate the significance levels: **P < 0.01;
Figure 48 shows a dose-dependent antioedematogenic effect caused by intraperitoneal injection of compound MV 8608 on histamine (Hist, A) -serotonin (5-HT, B) and bradykinin (BK, C)-induced mouse paw oedema. Each group represents the mean of 5-6 animals and the vertical bars the S.E.M. The asterisks indicate the significance levels: *P <0.05; ** P < 0.01;
Figure 49 shows the effect of compound MV 8612 given intraperitoneally on carrageenan (1mg/site)-induced pleurisy in mice. Each group represents the mean of 8 to 10 animals and the vertical bars the S.E.M. The asterisks indicate the significance levels: *P <0.05; **P < 0.01;
Figure 50 shows the effect of compound MV 8608 given intraperitoneally on carrageenan (1mg/site)-induced pleurisy in mice. Each group represents the mean of 8 to 10 animals and the vertical bars the S.E.M. The asterisks indicate the significance levels: ** P < 0.01;
Figure 51 shows the effect of compounds MV 8608 and MV 8612 given intraperitoneally on PAF-acether (1 g/site)-induced pleurisy in mice. Each group represents the mean of 10 animals and the vertical bars the S.E.M. The asterisks indicate the significance levels: **P < 0.01;
Figure 52 shows a dose-dependent inhibition of bradykinin-induced skin vascular permeability in rats caused by intraperitoneal injection of MV 8612 and MV 8608. Each group represents the mean of 8 animals and the vertical bars the S.E.M. The asterisks indicate the significance levels: *P <0.05; **P < 0.01;
Figure 53 shows a concentration-dependent inhibition of human lymphocyte proliferation caused by compounds MV 8612 and MV 8608. Each group represents the mean of 6-7 experiments and the vertical bars the S.E.M;
Figure 54 shows a dose-related antinociceptive effect caused by intraperitoneal injection of compounds MV 8612 and MV 8608 against acetic-acid-induced writhe responses in mice. Each group represents the mean of 8 animals and the vertical bars indicate the S.E.M;
Figure 55 shows a dose-related antinociceptive effect caused by intraperitoneal injection of compounds MV 8612 and MV 8608 against acetylcholine-induced writhe responses in mice. Each group represents the mean of 8 animals and the vertical bars indicate the S.E.M;
Figure 56 shows a dose-related antinociceptive effect caused by intraperitoneal injection of compounds MV 8612 and MV 8608 against kaolin-induced writhe responses in mice. Each group represents the mean of 8 animals and the vertical bars indicate the S.E.M; and
Figure 57 shows a dose-related antinociceptive effect caused by i.c.v. injections of compounds MV 8612 and morphine against acetic acid-induced writhe responses in mice. Each group represents the mean of 8 animals and the vertical bars indicate the S.E.M.

Other objects, advantages and features of the present invention will become more apparent upon reading of the following non-restrictive description of preferred embodiments with reference to the accompanying drawing which is exemplary and should not be interpreted as limiting the scope of the present invention.

### DESCRIPTION OF THE PREFERRED EMBODIMENT

The present invention thus concerns the pharmaceutical use of saponin-like compounds isolated from *Mandevilla* species having the general formula EST as defined above with specific R-type calcium channel blocking properties which display the remarkable property of virtually only affecting the overstimulation of R-type Ca²⁺ channels, without significantly affecting the basal activity thereof. In addition, these compounds, and especially MV8608 and MV8612, display the remarkable property of blocking or relieving the side effects associated with other drugs or compounds. Furthermore, the R-type Ca²⁺ channel blockers show significant activity in a number of applications ranging from a control of cellular proliferation to pain control.

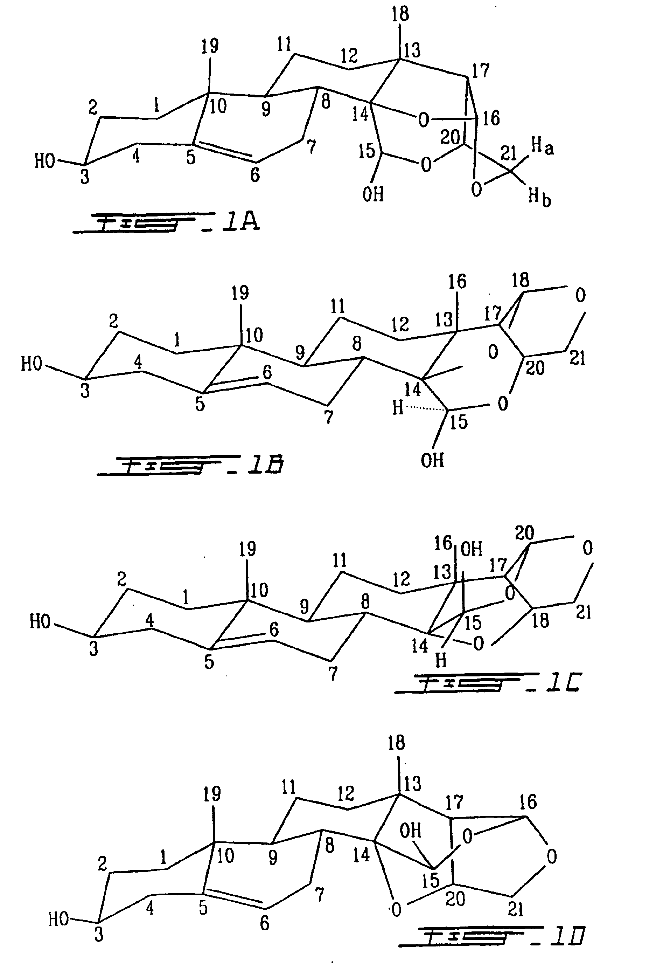

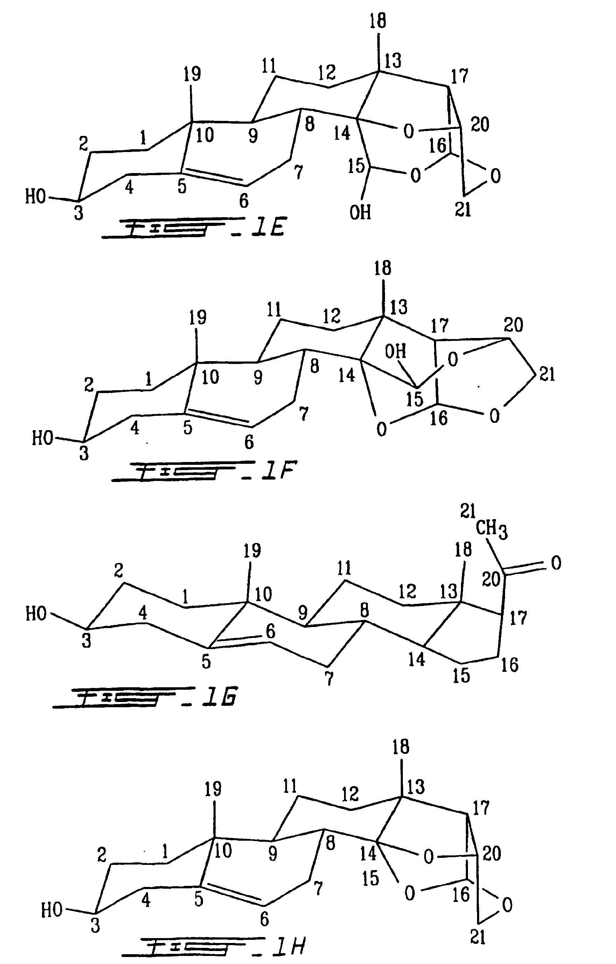

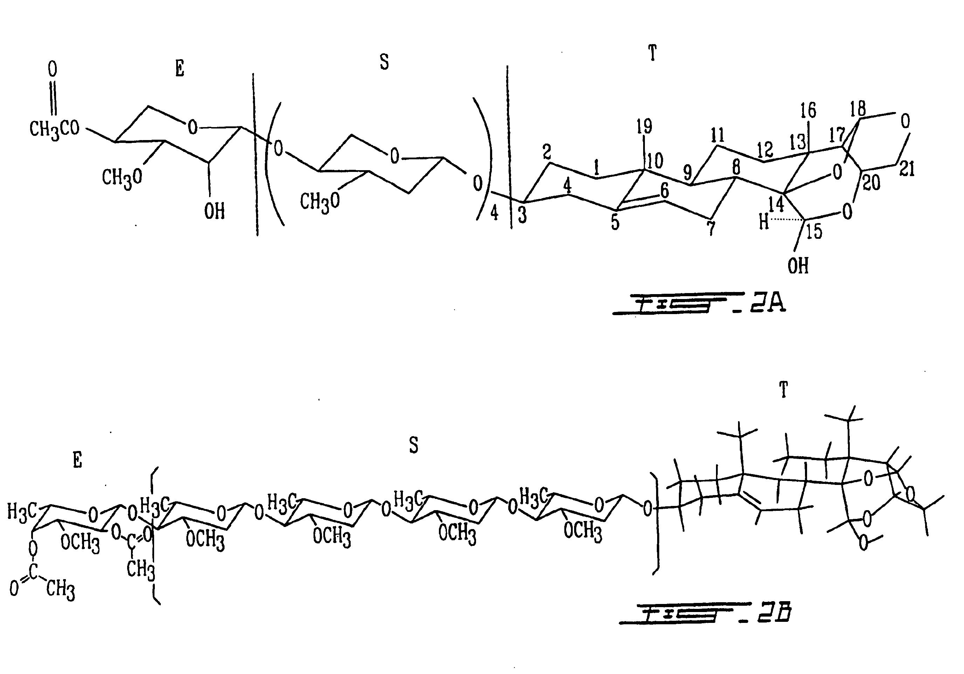

The freshly collected rhizomes of *Mandevilla velutina* were extracted with ethyl acetate and then fractionated by column chromatrography on silica gel with methylene chloride and ethyl acetate as solvents giving 20 components. Two of these fractions showed indirect bradykinin blocking action. As mentioned previously, one of them named Velutinol (MV8608) shows that the structure comprises a pregnane skeleton. The structure of Velutinol A was determined (Yunes et al., 1993, Phythochemistry. 34:787-790: 1993, Phytochemical Analysis 4:76-81: 1993, Phytochem. Anal. 4:76-81; Bento, 1996, J. Chem. Soc. Perkin Trans 2:1359-1366; Yunes et al., 1996) as : 3-β-hydroxipregna-5-one derivatives (see Figure 1, compounds I, IA and IB) and it was suggested to be a (15R, 16R, 20S)-14, 16:15,20:16,21-triepoxi-15,16-seco 14β, 17α-pregn-5-ene-3β, 15-diol. Figure 1, (compounds I, IA and IB).

Pregnane derivatives have been reported to be present in several species (Abe et al., 1976, Phytochemistry 15:1745-1748; 1978, Chem. Pharm. Bull. 26 (10):3023; 1979, Chem. Pharm. Bull. 27 (7):1604-1610; 1981, Chem. Pharm. Bull. 29(2):416; 1987, Chem. Pharm. Bull. 35 (10):4087; 1988, Chem. Pharm. Bull. 36 (2):612; 1988, Chem. Pharm. Bull. 36 (10):3811). In any event, others isomers could also exist as are shown by structures II, 111, IV, V and VI (Fig. 1). According to the Calixto group, these isomers have also been shown to have activity through the bradykinin receptor.

For comparison structure of 5-pregnane-3β-ol-20-one is shown in Figure 1 (compound V).

Surprisingly, it was discovered that compound MV8608 had an inhibitory activity on the R-type calcium channel. Furthermore, this inhibitory activity was shown to be specific to the R-type calcium channel.

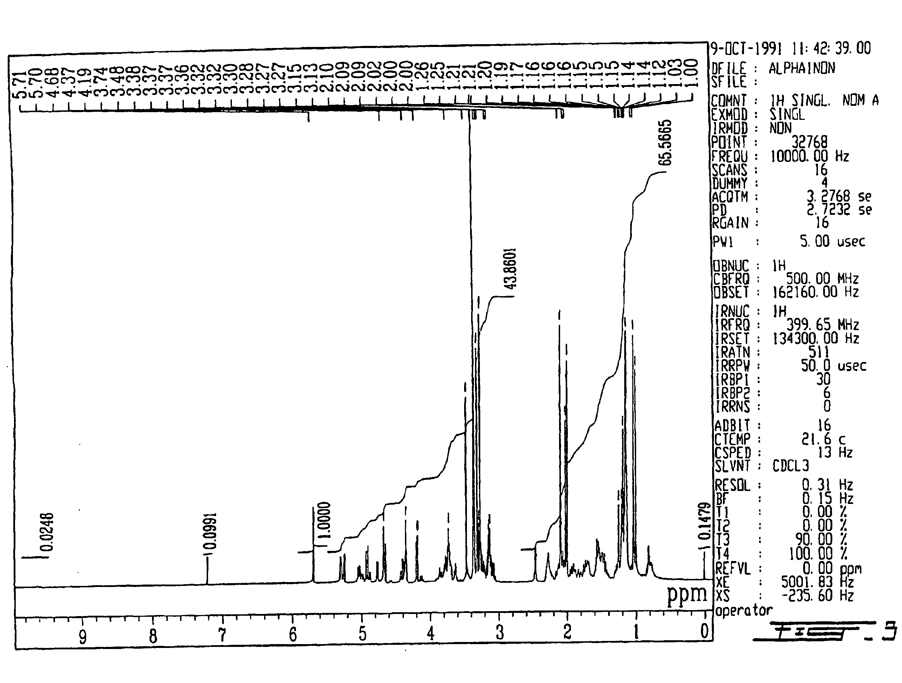

Isolated from the same *Mandevilla velutina* rhizomes an other compound MV8612 has a very specific inhibitory activity on the steady-state calcium channel type R. The structure of MV8612 was determined. The invention also relates to the structure of MV8612 (analogs A and B compound, Figure 2) and its saponin-like derivatives displaying an inhibitory activity of the steady-state R-type calcium channel. The primary structure of such compounds is shown in Figure 2.

It is important to note that the molecule of the present invention consists of a classical saponin oligosugar part (designated as "ES" in Figure 2) and a steroid ("T") portion. The structure of the steroid (T) component of the molecule is based on a 5 pregnane-3β-ol derivative with a tricyclic oxygenated ring system or illustrol isomer as shown in Figure 1 (compounds V and VI). However, as will be recognized by a person of ordinary skill to which the instant invention pertains, derivatives of these compounds can possess inhibitory activity on the Ca²⁺ influx into the cytosol, the nucleus, the mitochondria as well as the (SR) sarcoplasmic reticulum and (ER) endoplasmic reticulum, in the EST combination as shown in Fig. 2. The structure of "T" is preferably a 5-pregnane-3β-ol oxytricyclo 15-ol as shown in Fig. 2, although a 5-pregnane-3β-ol-20-one, cholesterol, cholic acid, ergosterol, stigmasterol, androstenon, digitoxygenin, β-sitostenol, uvaol, ursolic acid, sarsasapogenin, 18, β-glycyrrhetinic acid, betulin, betulinic acid, oleanoic acid, podocarpic acid are also encompassed as being within the scope of the present invention.

In the EST formula (Figure 2, analog A), S is preferably α(1-4) (2-deoxy, 3-methoxy) -L-lyxotetrose, α(1-4) (2-deoxy, 3-methoxy) L-xylotetrose, α(1-4) (2-deoxy, 3-methoxy)-L-arabinotetrose, α(1-4) (2-deoxy, 3-methoxy)-L-xylotetrose, α(1-4) (2-deoxy, 3-methoxy-L-ribopyranotetrose, α(1-4) (2-deoxy, 3 methoxy-L-sorbotetrose, α(1-4)-L-lyxotetrose, α(1-4)-L-xylotetrose, α(1-4)-L-arabinotetrose, α(1-4)-L-xylotetrose, α(1-4)-3. 4 methoxy-L-lyxotetrose, α(1-4)-3, 4 methoxy-L-xylotetrose, α(1-4)-3,4 methoxy-L-arabinotetrose, α(1-4)-3,4 methoxy-L-xylotetrose, α(1-4)-3,4 methoxy-L-ribopyranotetrose, α(1 -4)-3,4 methoxy-L-sorbopyranotetrose, α(1-4)-L-lyxotetrose, α(1-4)-L-xylotetrose, α(1-4)-L-arabinotetrose, (1-4)-L-riboyranoetrose,α(1-4)-L-sorbotetrose.

The MV8612 analog A has a monomeric to oligomeric of mentioned sugar derivatives, and has preferably a tetra sugar derivative. The terminal E of the analog A part is preferably 4-acetoxy-3 methoxy-L-α-lyxose, 4-acetoxy-3-methoxy-L-α-xylose, 4-acetoxy-3-methoxy-L-α-arabinose, 4-acetoxy-3-methoxy-L-α-xylose, 4-acetoxy-3-methoxy-L-α-ribopyranose, 4-acetoxy-3-methoxy-L-α-sor-bose-acetoxy.

The compound of formula I (I, IA and IB) and MV8612 analogs VIIA and VIIB could be modified into peptidic analogs (deprotection reaction of amines functions in peptidic syntheses, acid treatment or catalytic hydrogenation depending on the nature of the ES) in order to obtain peptidic analogs of compounds of formula I as commonly known to a person of ordinary skill. The compounds of formula I (IA and IB) could be, if necessary purified using classical technique such as crystallisation and/or silice column chromatography.

As used herein, "chemical derivatives" is meant to cover additional chemical moieties not normally part of the subject matter of the invention. Such moieties could affect the physico-chemical characteristic of the derivative (i.e. solubility, absorption, half life and the like, decrease of toxicity). Such moieties are examplified in Remington's Pharmaceutical Sciences (1980). Methods of coupling these chemical-physical moieties to a polypeptide are well known in the art.

As used herein, the terms "molecule", "compound" or "ligand" are used interchangeably and broadly to refer to natural, synthetic or semisynthetic molecules or compounds. The term "molecule" therefore denotes for example chemicals, macromolecules, cell or tissue extracts (from plants or animals) and the like. Non limiting examples of molecules include nucleic acid molecules, peptides, antibodies, carbohydrates and pharmaceutical agents. The agents can be selected and screened by a variety of means including random screening, rational selection and by rational design using for example protein or ligand modelling methods such as computer modelling. The terms "rationally selected" or "rationally designed" are meant to define compounds which have been chosen based on the configuration of the interaction domains of the present invention. As will be understood by the person of ordinary skill, macromolecules having non-naturally occurring modifications are also within the scope of the term "molecule". For example, peptidomimetics, well known in the pharmaceutical industry and generally referred to as peptide analogs can be generated by modelling as mentioned above. Similarly, in a preferred embodiment, the polypeptides of the present invention are modified to enhance their stability. It should be understood that in most cases this modification should not alter the biological activity of the interaction domain. The molecules identified in accordance with the teachings of the present invention have a therapeutic value in diseases or conditions in which the physiology or homeastasis of the cell and/or tissue is compromised by a defect in Ca²⁺ homeostasis. Alternatively, the molecules identified in accordance with the teachings of the present invention find utility in the development of more efficient compounds to reduce or prevent overstimulation of R-type Ca²⁺ channel associated diseases. As exemplified herein, the present invention provides numerous assay systems to test the effect of these molecules.

They can be also ionized with an acceptable pharmaceutical acid, or if it is possible and if it desired with an acceptable pharmaceutical base.

The necessary crude materials used in the processes described herein are either commercially available or easily accessible to a person of ordinary skill to which the present invention pertains knowledgeable of the instant invention and of the procedures available in the literature.

In comparison with the dual L and R-type Ca²⁺ channel blocker, israpidine (PN200-110), the compounds of the present invention, presents a highly superior *in vivo* as well as *in vitro* specificity and potency as well as protective and therapeutics cellular activities against Ca²⁺ overload in all cell types. Non-limiting examples of such cell types include heart, vascular smooth muscle, vascular and non vascular endothelial cells, bone cells, T lymphocytes, monocytes, smooth muscle cells, nerve cells, cerebral cells, and non-differentiated cells of anaplasic or neoplasic origins.

The tests realized *in vitro* on VSMC, VEC, bone cells, blood immune cells and heart cells in culture, placed in several pathological, electrical and hormonal conditions showed that the compounds of the present invention protected and blocked in a remarkable way and more potently than isradipine, cell integrity and Ca²⁺ overload as well as Ca²⁺-dependent over stimulation of hormone secretion and abnormal excitation-contraction coupling and conduction. Other tests carried out *in vitro,* using abnormal proliferation of T-lymphocytes as well as VSM, VEC and osteoblast cells, demonstrated that the compounds of the present invention significantly and remarkably protected the cells from proliferation, significantly largely decreased their capacity to undergo spontaneous proliferative processes and retained their normal integrity and function. The effects of the compounds of the present invention were largely superior to that of isradipine.

The tests *in vivo,* using rats and rabbits as well as guinea pigs as model systems for warm blooded animals demonstrated that the compounds of the present invention significantly prevented and blocked vasoconstriction, hypotension and airways hypereactivity induced by PAF, ET-1 and organ transplantation without any side effect. On note, the activity of the compounds of the present invention was shown to be superior to be largely superior to that of isradipine which was, in addition and in contradistinction to the compounds of the instant invention, the dual L-and R-type channel blockers isradipine, exhibited significant side effects.

The remarkable properties of the compounds of the present invention make them valuable compounds in treatment of numerous diseases and conditions in which R-type Ca²⁺ channel blocking is beneficial. Non-limiting examples of such diseases or conditions include diseases of the cerebral, cardiac and vascular systems, and the immune system, for the treatment and prevention of cerebral and cardiac ischemia, vascular contraction, oedema, post-surgery and post-transplantation hyper-immune activities and related pathologies and septic shock.

In general, the protective effects of the compounds of the present invention and in particular of MV8612 find utility in the treatment of, for example, cardiac, vascular and cerebrovascular accidents of different origin, post-surgical traumas, encephalopathy, neuro-degenerative pathology, hypertrophy, cancer, diabetes type II, hyperthyroidism, osteoporosis, arrythmia, fibrillation as well as osteoporosis..

The property of the compounds of the present invention to protect cells during hypoxia and ischemia as well as remodelling also permits their use in the treatment and prevention of ischemia of peripheral tissues, mainly in cardiology for myocardial ischemia and coronary ischemia and their different clinical expressions such as for example angina, myocardial infarct, arrhythmias, vasospasms, heart failure, fibrillation; as well as in ophthalmology and in oto-rhino-laryngology during chorio-retinial vascular damage, vertigo of vascular origin, vertigo de Meuniere or d'acouphenes as well as digitalis intoxication.

The invention concerns also the addition of salts to the compounds of the present invention and in particular to the compounds of formula I (I, IA and IB) and MV8612 analogs VIIA and VIIB obtained with a mineral or organic pharmaceutically acceptable salt.

The pharmaceutically acceptable acids that can be used to obtain a salt, by addition to the compounds of the present invention, are well known to the person of ordinary skill and taught for example in Remington Pharmaceutical Sciences (1980). Non-limiting examples of pharmaceutically acceptable acids include chlorhydric acid, phosphoric acid, tartaric acid, malic acid, fumaric acid, oxalic acid, methanesulfonic acid, ethanesulfonic acid, camphoric acid, citric acid, etc.

Non-limiting examples of pharmaceutical bases which can saltify the compounds of the present invention and in particular the compounds of formula 1 (I, IA and Ib) and analogs VIIA and VIIB, include sodium, potassium, calcium, aluminium hydroxyl, carbonates of acaly metals or alkalinoterrus or organic bases such as triethylamine, benzylamine, diethanolamin, tertbutylamin, dicyctohexylamin, arginine, etc.

The present invention also relates to the pharmaceutical compositions including as an active ingredient, a saponin-like compound of the present invention. More particularly, a compound of the present invention having the EST structure as shown in Figure 2 and, even more particularly, a compound of formula 1 (I, IA and IB) and MV8612 analogs VIIA and VIIB or their salt derivatives (by addition for example of a mineral or organic base or acid) together with a pharmaceutically acceptable carrier, as well known in the art. Non-limiting examples of such pharmaceutically acceptable carriers include inert excipients, non toxic covenants for pharmaceutical use and/or an agents attaching an aromatic agent, a delitement agent, edulcorant agent, lubrificant agent as well as a liquid and semi-liquid vehicle adapted for different modes of administration such as for example sterile epirogenic water for intravenous administration (see for example Remington Pharmaceutical Sciences (1980)).

Non-limiting examples of pharmaceutical compositions according to the invention include, in particular, those adapted for oral, parental, ocular, per or transcutan, nasal, rectal, perlingual administrations such as ocular or nasal drops, pills, sublingus pills, capsules, tablets, suppositories, cremes, pomades, gels, and the like (see for example Remington Pharmaceutical Sciences (1980)).

The compositions of the present invention are generally presented in a dose form and can contain dependent on the patient treated, age and sex of the patient, from 0.1 to 500 mg of the active principle.

It can, depending on the route of administration be delivered at a dose of 0.1 to 500 mg of one or several times a day.

The terminology "pharmaceutical" is used herein in a broad sense to cover veterinary uses. The compositions will be readily adapted by the skilled artisan for the treatment of particular warm blooded animals to which the instant invention pertains.

From the specification and appended claims, the term therapeutic agent should be taken in a broad sense so as to also include a combination of at least two such therapeutic agents. Further, compounds according to the present invention can be introduced into warm blooded animals including human patients in a number of ways, as well known in the art. Erythropoietic cells can be isolated from the afflicted individual, transformed with a DNA construct according to the invention and reintroduced to the afflicted individual in a number of ways, including intravenous injection. Altematively, the DNA construct can be administered directly to the afflicted individual, for example, by injection in the bone marrow. The DNA construct can also be delivered through a vehicle such as a liposome, which can be designed to be targeted to a specific cell type, and engineered to be administered through different routes.

For administration to humans, the prescribing medical professional will ultimately determine the appropriate form and dosage for a given patient, and this can be expected to vary according to the chosen therapeutic regimen (i.e. DNA construct, protein, cells), the response and condition of the patient as well as the severity of the disease.

Composition within the scope of the present invention should contain at least one of the active agents in an amount effective to achieve the desired therapeutic effect while avoiding adverse side effects. Typically, the compounds in accordance with the present invention (i.e. MV8608 or MV8612) can be administered to warm blooded animals (i.e. humans) in doses ranging from 0.005 to 1 mg per kg of body weight per day of the warm blooded animal which is treated. Pharmaceutically acceptable preparations and salts of the active agent are within the scope of the present invention and are well known in the art (Remington's Pharmaceutical Science, 16th Ed., Mack Ed.). The dosage will be adapted by the clinician in accordance with conventional factors such as the extent of the disease and different parameters from the patient. Typically, 0.001 to 50 mg/kg/day will be administered to the warm blooded animal.

The present invention is illustrated in further detail by the following non-limiting examples.

### EXAMPLE 1

### Procedure for isolation and purification of compound MV8612 (Figure 2)

The rhizomes of *Mandevilla velutina* were grounded into small pieces and extracted repeteadly with ethyl acetate. The extract was filtered and evaporated to yield a brown powder that accounts for 9% of the rhizomes. The extract was fractioned by silica gel column chromatography with a methylene chloride system containing increasing amounts of ethyl acetate.

Fractions were collected and monitored by thin layer chromatography (TLC; Silica Gel G), eluted with toluene-EtoAc-MeOH (55:45:5) and visualized with short and long wavelength u.v. light or with an arylaldehyde-AcOH-MeOH-H₂SO₄ (0:5:10:85:5) spray.

Fractions rich in velutinol glycoside MV8612 were rechromatographed in the same manner several times. Further purification by TLC yields the pure compound VIIB (Fig. 2) crystallized in ethanol.

Compound VI (0.0001% of dry weight) mp 148-150°C, white needles from ethanol responded positively to the Lieberman Burchard (Abisch et al., 1960, Helv. Chim. Acta 43:1844), Xanthydrol (Barton et al., 1952, Nature 170:249) and Keller-Kiliane (Nagata et al., 1957, Helv. Chim. Acta 40:41) indicating a stereoidal glycoside of a 2-deoxysugar.

The molecular formula was obtained through elementar analysis [((60-26%), H (7.94%), 0 (31.10%)] [Cale:((60.80%) H(8.01%), O(31.10%) and fast atom bombardment (FAB) mass spectrum (MS) that afforded a molecular peak at m/z 1205 (M + Na⁺); 1221 (M + K⁺) and 1200 (M + NH₄⁺) suggesting to be C₆₀H₉₄O₂₃. The lR spectrum showed peaks (KBr) at CM⁻¹: 3450 (-OH), 1745 (-COCH₃), 2920, 1440 (OCH₃), 1230, 1160, 1100, 1080, 1050 (O-C-O). Its methanolic solution is transparent in the UV visible region. In the mass spectrum the loss of fragment 45 from the aglicone, following by the loss of a fragment of 244, were indicative of a one terminal sugar with two acetyl and one methoxyl groups and suggesting a straight chain of sugars. The positive ionization fast atom bombardment mass spectra (FAB-MS) confirmed the result with peaks at m/z (%) 1137 [M-Co-OH]⁺ (66) 893 [1137-C₁₁H₁₇O₆]⁺(10) 749[893-C₇H₁₂O₃)]⁺ (21), 605 [749-C₇H₁₂O₃⁺(25); 462 [605-(H₇ O₁₁]₃ (10), 318[462-C₇H₁₂O₃]⁺ (15) and suggested that there are four dideoxy sugars in the molecule.

### EXAMPLE 2

### NMR Experiments

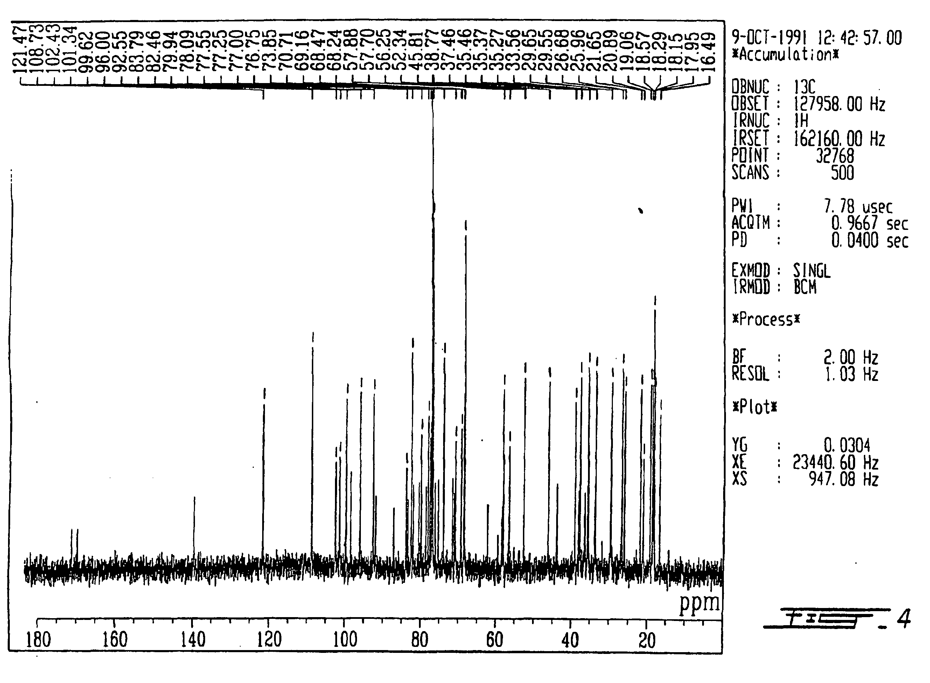

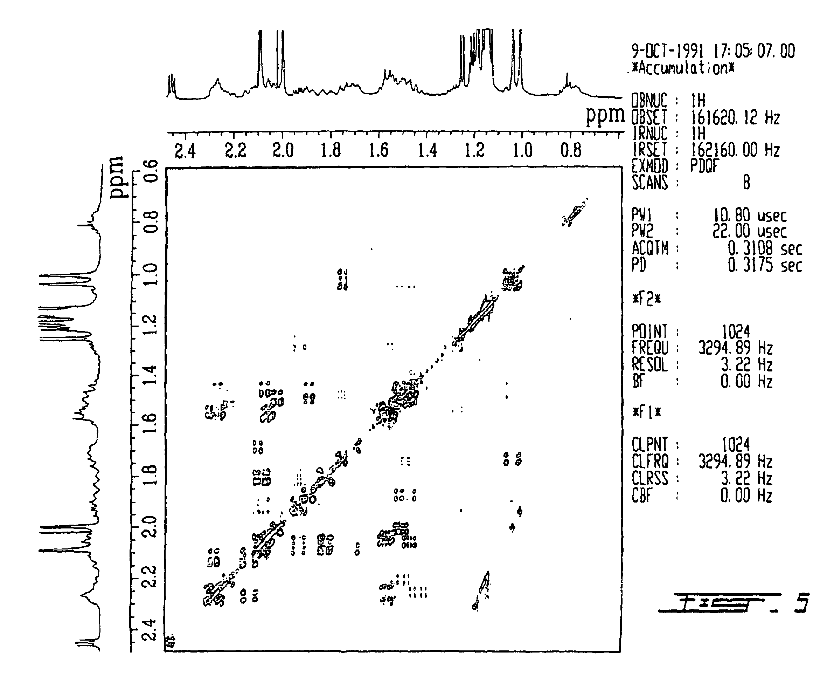

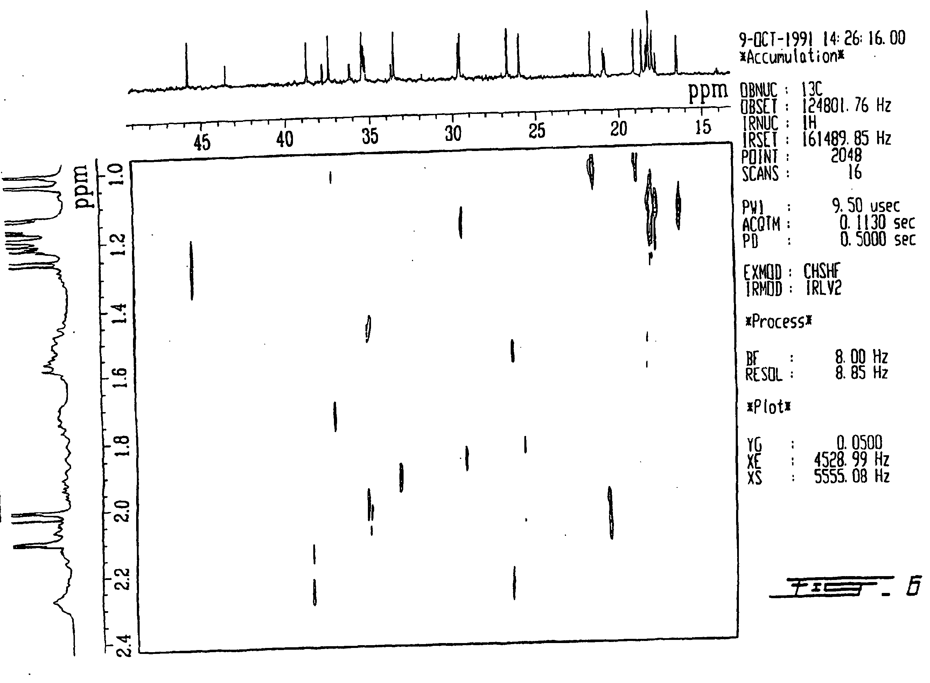

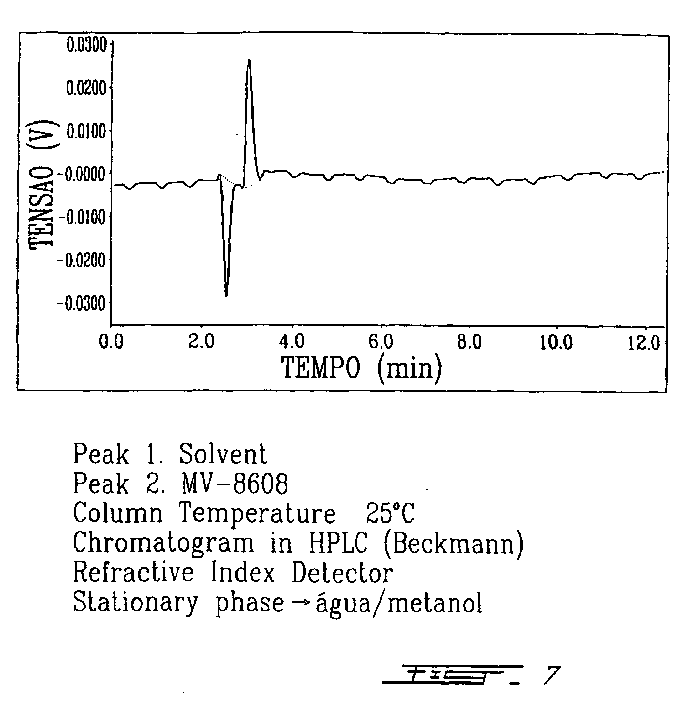

The results of the NMR experiments are shown in Figures 3-6.

### 1) 1D ¹HNMR Spectrum

The analysis of the 600 Mhz ¹H NMR spectrum can be divided into three distinct regions. The first region [5.78 ppm-4.28 ppm] with the best resolved signals, corresponds to the five anomeric protons and protons at C2 and C4 of the sugar ring 5, besides protons 16, 6, 15 (H), 15(OH), 10 and 21 b of the genin part (velutinol).

For the second region [3.93 ppm - 3.15 ppm], a very crowded region, the integration is proportional to 31 protons and were assigned as protons 3 and 21 a of the genin part, five methoxy and fourteen methine protons of the sugar rings.

The integration of the last region [2.53 - 1.08 ppm] showed the presence of fifty protons; five secondary methyl, eight methylenic, two methyl from acetyl groups and twenty-one protons from the genin part.

### 2) 1D selective TOCSY

The 1D selective TOCSY was used to define each one of the five spin systems for the sugar rings attached to the genin part. Selective irradiation of an isolated spin multiplet yields a subspectrum of all hydrogens directly or indirectly scalar, coupled to the irradiated resonance, if the mixing time is long enough to allow complet transfer of magnetization. A description of the results is given in Table 1.

**TABLE 1 1D selective TOCSY**

| **Irradiated proton** | **Observed signals (8, ppm)** | | | | | |
|---|---|---|---|---|---|---|
| | **H1** | **H2** | **H3** | **H4** | **H5** | **H6** |
| H1,R1 δ=4.45ppm | X | Ax:1.58 | 3.81 | 3.22 | 3.85 | 1.22 |
| | | Eq:2.08 | | | | |
| H1,R2 δ=4.76ppm | X | Ax:1.64 | 3.78 | 3.19 | 3.34 | 1.21 |
| | | Eq:2.11 | | | | |
| H2,R3(ax) δ=1.57ppm | 4.45 | Ax:1.57 | 3.40 | 3.15 | 3.28 | 1.29 |
| | | Eq:2.29 | | | | |
| H1,R4 δ=4.96ppm | X | Ax:1.55 | 3.77 | 3.19 | 3.92 | 1.21 |
| | | Eq:2.16 | | | | |
| H2,R5 δ=5.12ppm | 4.43 | 5.12 | 3.33 | 5.33 | 3.70 | 1.21 |

The four anomeric protons of 2,6-dideoxyhexopyranose appeared as a doublet of doublets at 4.96; 4.85; 4.76 and 4.45, with J = 10 and 2 Hz. A fifth anomeric proton appeared as a doublet (J = 10 Hz), 4.43 and was assigned to the normal hexapyranose unit. The large value of the coupling constant of these anomeric protons were typical of the axial configuration of the hexopyranoses in the C-1 (D) conformation indicating that these sugars were joined through (1 → 4)-glycosidic linkages.

The spectrum also contained five methoxy groups which appear as singlets and were observed at 3.37(3H), 3.44(3H), 3.39(3H), and 3.34(6H); five secondary methyl groups appear as doublets and were observed at 1.21 (9H), 1.22(3H) and 1.29(3H), (J = 6.0 Hz); two tertiary methyl groups singlets were observed at 1.08 and 1.11 and two methyl from acetyl groups were observed at 2.07 and 2.18.

The eight C-2 methylene protons of four 2-deoxy sugar units appeared as two sets of four protons multiplets in the regions 2.29-2.08 and 1.64-1.55 for the equatorial and axial protons respectively (Abe et al., 1988, Chem. Pharm. Bull. 36 (2):612; Abe et al., 1987, Chem. Pharm. Bull. 36 (10):3382-3389). There is also a doublet of doublets at 5.12 (J = 10 and 8 Hz) attributed to the C-2 proton of the acetylated sugar, it couples with both the signals at 4.43 (anomeric proton) and 3.33 credited to a C-3 proton. The C-3 signal, part of a multiplet, is coupled with a doublet of doublets at 5.33 (J=3.0 and 2.0 Hz) attributed to the C-4 proton of a diacetyl sugar, which in turn is coupled with a doublet of doublets at 3.70 (J=6.0 and 2.0 Hz) attributed to the C-5 proton. This is in turn coupled to a doublet (J=6.0 Hz) at 1.21, attributed to the secondary methyl. The chemical shift for C-2 and C-4 is in accordance with an acetyl sugar derivatives.

### 3c) COSY spectrum

The J coupling relationship described above was also determined from the COSY spectrum. The complete ¹H NMR assignment of all the protons in the five sugar rings is given below:
**a)** H1, 4.43 H2, 5.12 H3, 3.33 H4, 5.33 H5, 3.70 CH3, 1.21.
**b)** H1, 4.96 H2, 1.55 and 2.16 H3, 3.77 H4, 3.19, H5, 3.92 CH3, 1.21.
**c)** H1, 4.45 H2, 1.57 and 2.29 H3, 3.40 H4, 3.15 H5, 3.28 CH3, 1.29
**d)** H1, 4.76 H2, 1.64 and 2.11 H3, 3.78 H4, 3.19 H5, 3.34 CH3, 1.21.
**e)** H1, 4.85 H2, 1.58 and 2.08 H3, 3.81 H4, 3.22 H5, 3.85 CH3, 1.22.

### 4) ¹³C spectrum, DEPTs and CH correlations

The carbon 13 nuclear magnetic resonance spectra (¹³C NMR) indicated the presence of six quaternary carbons, fourteen methyl, eleven methylene, twenty nine methine and two carbonyl groups (Breitmaier et al., 1987, Third Edition VCH Veriagsgprollachaft mbH weinheim RFG, Germany). The carbon signal were assigned on the ¹H-¹³C COSY (correlated spectroscopy) one-bond spectrum except for the quaternary ones. The long-range correlations data was used to assign these and the multiplicity of the protonated one was determined from the DEPT (distortion enhancement by polarization transfer) spectral data. The ¹³C assignments of the sugars are indicated in Table 2.

**TABLE 2 ¹³C assignments (sugars) (δ, ppm)**

| | **C1** | **C2** | **C3** | **C4** | **C5** | **-CH3** | **-Omc** |
|---|---|---|---|---|---|---|---|
| R1 | 96.11 | 35.63 | 68.38 | 82.59 | 70.87 | 18.25* | 56.74 |
| R2 | 99.73 | 36.15 | 77.10 | 83.91 | 71.19 | 18.27* | 58.33 |
| R3 | 101.45 | 36.35 | 78.75 | 82.15 | 71.54 | 18.41 | 56.39 |
| R4 | 98.47 | 35.37 | 76.39 | 83.81 | 69.29 | 18.06 | 58.03 |
| R5 | 102.55 | 70.72 | 80.34 | 68.45 | 71.02 | 16.58 | 57.81 |

| | | | | | | | |
|---|---|---|---|---|---|---|---|
| (*) These assignments can be interchangeable | | | | | | | |

| **CH3-CO** | | | |
|---|---|---|---|
| **CH3** | | **CO** | |
| 20.87** | C2-R5 | 170.6 | C4-R5 |
| 20.97** | C4-R5 | 172.4 | C4-R5 |

| | | | |
|---|---|---|---|
| (**) These assignments can be interchangeable | | | |

In agreement with the previous determination (ref. of velutinol) of the genin part (Velutinol A), the proton nuclear magnetic resonance spectra (¹H NMR) showed a characteristic signal due to the C-6 olefinic proton (5.38, m), as well as those due to C-19 and C-18 (1.08 and 1.11, s) methyl protons, C-3 methine proton (3.53, t,t), C-9, C-8, C-17, C-20, C-15 and C-16 methine protons (1.36, m; 2.01, td; 2.53, d,d; 4.44, d; 5.01, d; and 5.78,d, respectively) and C-21 methylene protons (, 3.81 and 4.28, d) (Abe et al., 1988, Chem. Pharm. Bull. 35 (10):4081-4087; Chen et al., 1987, Phytochem. 26 (8) :2351) (Tables 3 and 4).

### 5) CH long range correlations

The analysis of the ¹H⁻¹³C long-range data gave the following correlations which provide evidence for an attachment of the genin and the sugar residue, as well as for the following sequence of the sugar rings:
(Table 5)
**a)** H3(3.35, V) C1(96.11, R1) and H1(4.85, R1) C3(77.64, V) define the linkage point between the genin and the sugar residue;
**b)** H4(3.22, R1) C1(99.73, R2) and H1(4.76, R2) C4(82.59, R1) define the connection between the first and second sugar rings;
**c)** H4(3.19, R2) C1(101.45, R3) and H1(4.45, R3) C4(83.91, R2) define the connection between sugar rings 2 and 3;
**d)** H4(3.15, R3) C1(98.47, R4) and H1(4.96, R4) C4(82.15, R3) define the attachment of sugar ring 3 to sugar ring 4;
**e)** H4(3.19, R4) C1(102.55, R5) and H1(4.43, R5) C4(83.81, R4) define the linkage point between the last two sugar rings 4 and 5.

**TABLE 3 ¹³C assignments for the genin part (velutinol) in compound -12 (δ, ppm)**

| **Position** | **Velutinol*** | **Compound-12** |
|---|---|---|
| 1 | 37.4 | 37.57 |
| 2 | 31.4 | 29.67 |
| 3 | 71.3 | 77.64 |
| 4 | 42.0 | 38.89 |
| 5 | 139. 3 | 140.49 |
| 6 | 121.4 | 121.56 |
| 7 | 25.9 | 26.07 |
| 8 | 33.5 | 33.69 |
| 9 | 45.8 | 45.94 |
| 10 | 37.6 | 37.96 |
| 11 | 18.6 | 18.68 |
| 12 | 26.6 | 26.80 |
| 13 | 43.5 | 43.70 |
| 14 | 87.1 | 87.40 |
| 15 | 92.5 | 92.67 |
| 16 | 108.7 | 108.84 |
| 17 | 52.3 | 52.47 |
| 18 | 21.6 | 21.74 |
| 19 | 19.1 | 19.17 |
| 20 | 73.8 | 73.96 |
| 21 | 78.0 | 78.19 |

| | | |
|---|---|---|
| *** Breitmaier, E. et al.,** 1987, Carbon 13 NMR Spectroscopy-High Resolution Methods and Applications in Organic Chemistry and Biochemistry, Third Edition VCH Veriagsgprollachaft mbH weinheim RFG, Germany. | | |

**TABLE 4 ¹H NMR assignments of Velutinol and of the aglycone of 8612 compound**

| **Position** | **ppm** | |
|---|---|---|
| | **δ.-**^{**1**}**H** | |
| | Velutinol | Compound 12 |
| 1 | 1.12(a):1.82(b) | 1.14(a)qd:1.82(b)dt.J=13.5:3.5 |
| 2 | 1.85(a):1.50(b) | 1.95(a):1.56(b) |
| 3 | 3.53(H.a):1.80(OH) | 3.53(tt.J=11.5:4.5) |
| 4 | 2.30(a): 2.23(b) | 2.34(a): 2.23(b) |
| 5 | -- | -- |
| 6 | 5.38 | 5.38(m) |
| 7 | 2.16(a): 1.89(b) | 2.16(a): 1.90(qd.J=18.0:5.0:2.5) |
| 8 | 2.01 | 2.01 (td.J=11.5:5.5) |
| 9 | 1.36 | 1.36(dd) |
| 10 | -- | -- |
| 11 | 1.64(a.b) | 1.64(a.b)m |
| 12 | 2.35(a):1.65(b) | 2.35(a):1.65(b) |
| 13 | -- | -- |
| 14 | -- | -- |
| 15 | 5.01(H):4.75(OH) | 5.01(H)d.j=12.0:4.75(OH)d.J=12 |
| 16 | 5.78 | 5.78(d.J=4.5) |
| 17 | 2.53 | 2.53(dd.J=6.0:4.5) |
| 18 | 1.11 | 1.11(s) |
| 19 | 1.09 | 1.08(s) |
| 20 | 4.45 | 4.44(dd.J=6.0:3.5) |
| 21 | 3.81 (A) 4.28(B) | 3.81 (A)dd.J=10.0:3.5 4.28(B)d.J=10.0 |

**TABLE 5 ¹³C-¹H correlation long range**

| **C-1 (δ, ppm)** | | **H (δ, ppm)** | |
|---|---|---|---|
| R-1 | 96.11 | 3.53 | H3(Vel.) |
| R-2 | 99.73 | 3.22 | H4-R1 |
| R-3 | 101.45 | 3.19 | H4-R2 |
| R-4 | 98.47 | 3.15 | H4-R3 |
| R-5 | 102.55 | 3.19 | H4-R4 |

| **C (δ, ppm)** | | **H-1 (δ, ppm)** | |
|---|---|---|---|
| C-3 (velutinol) | 77.64 | 4.85 | R-1 |
| C4-R1 | 82.59 | 4.76 | R-2 |
| C4-R2 | 83.91 | 4.45 | R-3 |
| C4-R3 | 82.15 | 4.96 | R-4 |
| C4-R4 | 83.81 | 4.43 | R-5 |

### 6) NOESY spectra

The analysis of the cross peaks in the NOESY (2D n.O.e) spectra also provides evidence for the above mentioned connections between the sugar rings and the genin. The most important interactions are the following:

| | | | |
|---|---|---|---|
| H1(4.43, R5) | H4(3.19, R4); | H1(4.96, R4) | H4 (3.15, R3); |
| H1(4.45, R3) | H4(3.19, R2); | H1(4.76, R2) | H4(3.22, R1); |
| H1(4.85, R1) | H3(3.53, V); | H1(4.85, R1) | H4 (2.34, V). |

The analysis of the NOESY spectra also showed that in each of the sugar rings, proton at C-3 couple with proton in the C1, and this is in turn spatially coupled to proton at C-5 position. Such proximities can occur if these protons are all axial. This thus provides evidence that the methoxy and the secondary methyl groups are located at equatorial positions.

The values for the coupling constants (J) for protons 2 (10 and 8 Hz) and 4 (3.0 and 2.0 Hz) of the last sugar ring (5.12 and 5.33, respectively) indicate their position as axial configuration at C-2 and an equatorial configuration at C-4. The acetoxy groups are at equatorial and axial positions, respectively. In addition, the long range CH correlation spectra showed coupling between the two carbonyl at 170.6 ppm (acetyl at C-2) and 172.4 ppm (acetyl at C4), with the methyl signals at 2.08 ppm and 2.17 ppm, respectively.

The analysis of NMR spectra, specially long range CH correlations and cross peaks in the NOESY spectra, provides information for the connection between the sugar rings and the genin. The data from NMR confirm the sequence of sugars indicated by FAB-MS with the 6-deoxy-2,4-acetoxy-3-O-methyl hexopyranose as the terminal of the sugar chain.

### EXAMPLE 3

### Structure of the compounds

In the formula EST for analog VIIB (Fig. 2) the structure of the T part of the molecule is a 5-pregnane-3 -ol-20-one, cholesterol, cholic acid, ergosterol, stigmasterol, androstenon, digitoxygenin, -sitosterol, uvaol, ursolic acid, sarsasapogenin, 18, -glycylrhetinic acid, betulin, betulinic acid, oleanoic acid, padocarpic acid, and preferably 5-pregnane-3 -ol oxytricyclo 15-ol as shown in Fig. 1 (I, IA, IB, II, III, IV).

**S** for analog B is preferably (1-4) (2-deoxy, 3-methoxy, 5-methyl) -L-lyxotetrose, (1-4) (2-deoxy, 3-methoxy) L-xylotetrose, (1-4) (2-deoxy, 3-methoxy)-L-arabinotetrose, (1-4) (2-deoxy, 3-methoxy)-I-xylotetrose, (1-4) (2-deoxy, 3-methoxy-L-ribopyranotetrose, (1-4) (2-deoxy, 3 methoxy-L-sorbotetrose, (1-4)-L-lyxotetrose, (1-4)-L-xylote-trose, (1-4)-L-arabinotetrose, (1-4)-L-xylotetrose, (1-4)-3,4 methoxy-L- lyxotetrose, (1-4)-3,4 methoxy-L-xylotetrose, (1-4)-3,4 methoxy-L- arabinotetrose, (1-4)-3,4 methoxy-L-xylotetrose, (1-4)-3,4 methoxy-L- ribopyranotetrose, (1-4)-3,4 methoxy-L-sorbopyranotetrose, (1-4)-L- lyxotetrose, (1-4)-L-xylotetrose, (1-4)-L-arabinotetrose, (1-4)-L- sorbotetrose.

**E** for analog VIIB is preferably diacetylfucose but also 4-acetoxy-3 methoxy-L-lyxose, 4-acetoxy-3-methoxyl-L-xylose, 4-acetoxy-3-methoxylL-arabinose, 4-acetoxy-3-methoxy-L-xylose, 4-acetoxy-3-methoxy-L-ribopyranose, 4-acetoxy-3-methoxy-L-sorbose-acetoxy.

**MV-8608** - Has only one sugar bonded to the genin part T (Fig. 2).

**MV-8609** - Has in the part S of Fig. 2 only one sugar.

**MV-8210** - Has on the part S of Fig. 2 two sugars.

**MV-8611** - Has in the part S of Fig. 2 three sugars.

All of these compounds were demonstrated to be active against bradykinin-induced pharmacological effects (Calixto, Yunes, et al., 1987, *supra;* Calixto et al., 1988, Br. J.Pharmacol., 94:1133-1142).

### EXAMPLE 4

### Compounds from Mandevilla illustris

### MI-07 (illustrol)

The structure of illustrol (Fig. 1 compound VI) was determined by the same authors to be a derivative of 14:15-seco-15-norpregnane (Yunes et al., 1993, *supra).*

### MI-15, MI-18 and MI-21

Demonstrated to be active against bradykinin-induced pharmacological effects (Calixto et al., 1991, General Pharmacol. 22:99-101; 1991, Memórias do Instituto Oswaldo Cruz, 86:195-202) are of similar structure to that indicated in Fig. 2, where the Genin (part T) correspond to the illustrol. The S part has one, two three or more sugars and the terminal sugar E is, as was indicated preferably, 6-deoxy-2,4-acetoxy-3-0-methyl hexopyranose.

### EXAMPLE 5

### Experimental procedures

The freshly collected rhizomes of the *Mandevilla* species were cut into small pieces and repeatedly extracted with ethyl acetate at room temperature. The extract was filtered and evaporated under reduced pressure and the crude extract was fractioned by column chromatography on silica gel using methylene chloride with increasing amounts of ethyl acetate as eluent.

After repeated column chromatography of the fractions using hexane-acetone as eluents, it is possible to isolate several compounds that exhibit indirect bradykinin blocking action.

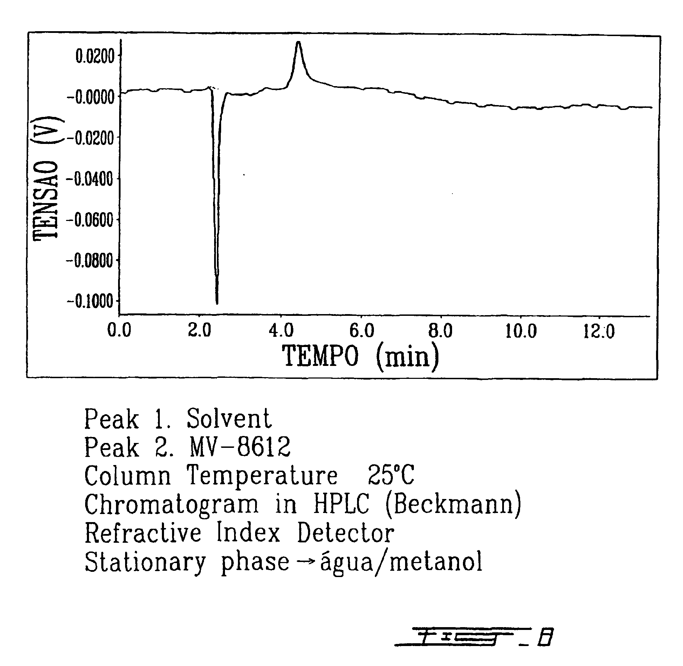

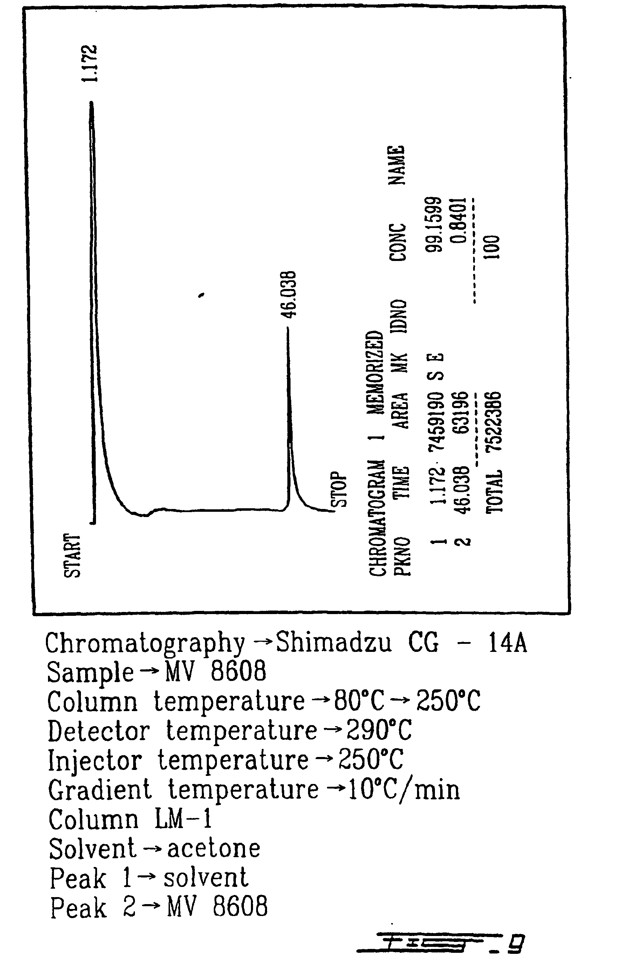

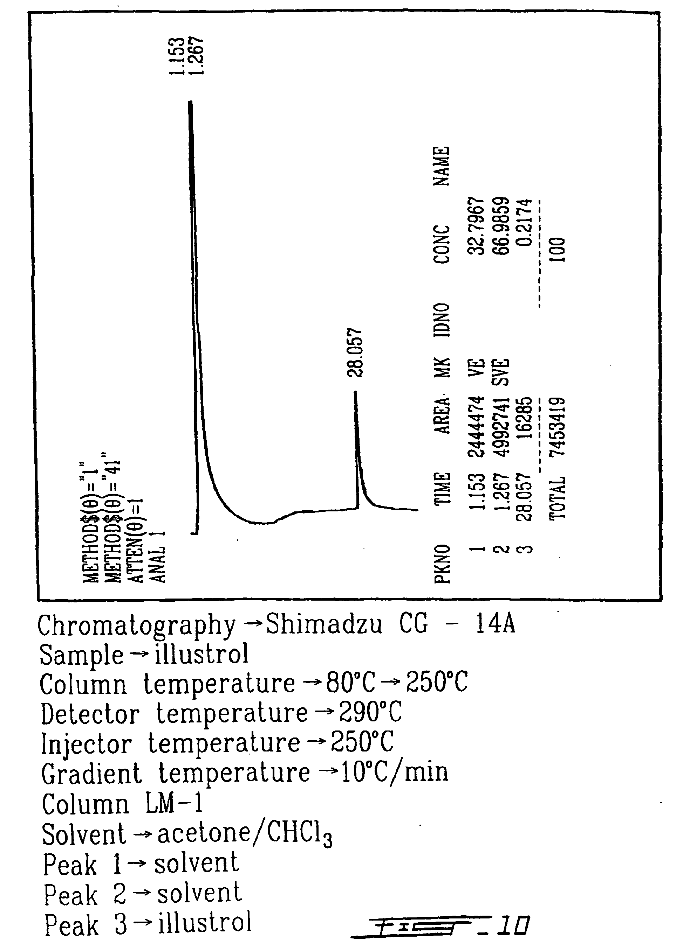

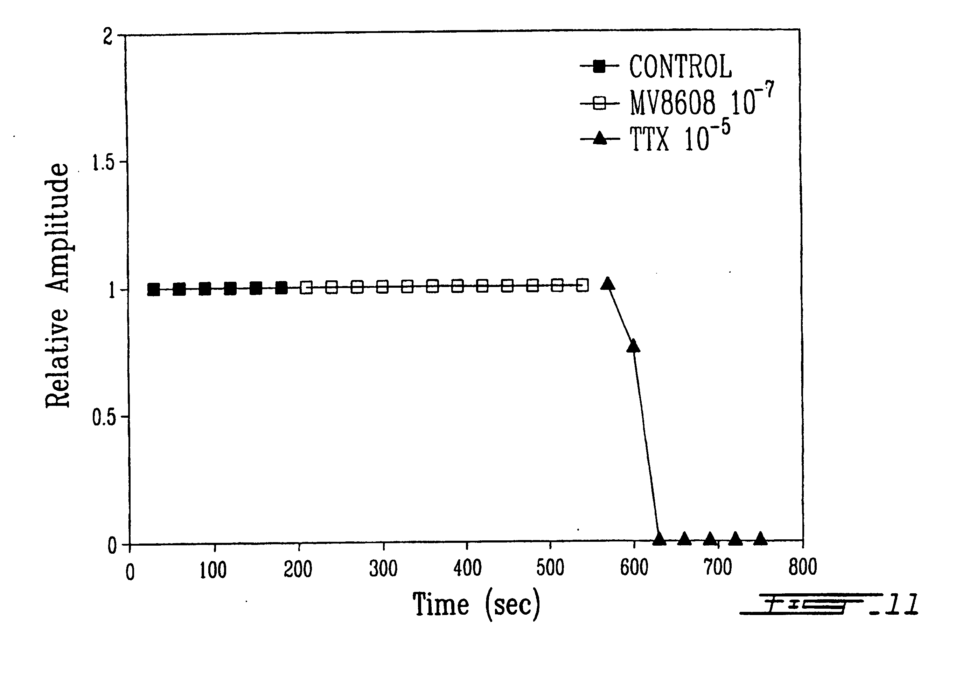

HPLC chromatogram of MV8608 and MV8612 are observed in Figs.7 and 8 and a CG chromatogram of MV8608 in Fig. 9. The CG chromatogram of lllustrol is shown in Fig. 10.

M.ps. (melting point) were determined using a Kofler-stage microscope and are not corrected. Optical rotation were recorded at 18-28° C using a 1 dm cell. IR spectrum was obtained from KBr discs and in CHCl₃ solution. Electron-impact mass spectra were taken on an MS Finnigam model 1020. The FAB-MS were taken on a Fison.

The NMR experiments were made in CDCl₃ solution with TMS as internal standard, using AM 250 and Amx 600 instruments, and a JEOL alpha 500 instrument. One-dimensional ¹H spectram were acquired as 64 K data points with a spectral width of 8.5 ppm (0-8.5). One-dimensional ¹³C spectrum were recorded as 64 K data points with a spectral width of 200 ppm (0-200). The ¹³C DEPT (distortionless enhancement by polorization transfer) experiments (90 and 135) were both recorded on the Bruker AM 250 spectrometer at 62.89 MHz. The 1D selective TOCSY were recorded on the JEOL alpha 500 MHz spectrometer. The phase sensitive DQF-COSY and NOESY (nucleus overhouser effect spectroscopy) were acquired using standard Bruker programs. The hereronuclear (¹H - ¹³C) correlation experiments, both one-bond and long-range correlation, were performed in reverse ¹H detected mode.

TLC was performed on silica gel (Merck Kilselgel 60 F254 0.25 mm layers). Column Chromatography was carried out on silica gel 200-300 mesh).

The plant material of *M.velutina* was collected from Minas Gerais State, Brazil, and was identified by Prof. Ademir Reis and Valério F. Ferreira of the Department of Botany of the Federal University of Santa Catarina. A voucher specimen is deposited in the Herbarium "Flor" of the Department of Botany, Federal University of Santa Catarina, under acession number 17.888-17.892.

### EXAMPLE 6

### Pharmacological study of the compounds of the present invention:

### Principle of in vitro studies

Single cells from heart, VSM and VEC of human and animals in culture constitute a model of choice for looking at the effect of drugs on different types of ionic channels using whole-cell and single channel patch clamp techniques in normal and stimulated conditions (Bkaily et al., 1988, *supra;* 1991, *supra;* 1992, *supra;* 1992a, *supra;* 1993a, *supra;* 1996a-e, *supra;* Bkaily G. 1994a,b, *supra).* Indeed, it is recognized as a model system in analyzing drug ionic channel interactions.

Heart cells as well as VSMC possess fast Na⁺ current, T, L and R-type Ca²⁺ channels as well as different types of K⁺ channels (Bkaily G., 1991, *supra;* 1995, *supra).* However, vascular endothelial cells (VECs) only possess R-type Ca²⁺ channels and different types of K⁺ channels. Thus, the later type of VECs constitute a model of choice for studying the effect of drugs on Ca²⁺ influx due to opening of the R-type Ca²⁺ channels (Bkaily G., 1994, *supra;* 1996, *supra;* 1997a-d, *supra).* Of note, the R-type Ca²⁺ channel has been reported to be responsible for the sustained increase of intracellular calcium and nuclear and cytosolic Ca²⁺ overload that are a result of sustained depolarization of the cell membrane or continual presence of several cardioactive and vasoactive hormones such as ET-1, PAF, bradykinin and insulin (Bkaily G., 1994, *supra;* 1992, *supra; 1993, supra;* 1995, *supra;* 1996*, supra;* 1997a, *supra;* 1997b, *supra;* 1997c, *supra;* 1997d, *supra;* 1998, *supra).*

A sustained increase of cytosolic, nuclear and mitochondrial Ca²⁺, considered as pathological, is a visible and measurable aggression in all types of excitable and non excitable cells such as heart cells, VSMC, VEC, osteoblast cells and immune cells (Bkaily et al., 1996, *supra).*

Herein the effect of the compounds of the present invention were tested on the above-mentioned cell types at whole-cell and cell attached patch clamp configurations, as well as at [Ca]ᵢ, [Ca]_{c} and [Ca]ₙ levels using a standard techniques (Bkaily, 1994a,b, *supra;* 1992, *supra;* 1993, *supra;* 1995, *supra;* 1996, *supra;* 1997a,b,c,d, *supra;* 1998, *supra).*

### Methodology

Single cells of different types in culture were prepared from biopsies of human, chick and rabbit. Known and accepted methods for the isolation of fast Na⁺ current, T, L and R-type Ca²⁺ channels as well as delayed outward K⁺ current were used (Bkaily 1994, *supra).*

The compounds to be tested are added to the appropriate extracellular solution after recording a stable ionic current or normal steady-state level of [Ca]ₙ and [Ca]ₙ The effect of different concentrations of compounds are tested on the different type of current and [Ca]ᵢ of the different cell types. The effect of each concentration of the compounds in function of the time of exposure are then determined. Once the steady-state effect is reached, the second concentration is added, etc.

Also, for the R-type Ca²⁺ channel current, the effect of the compounds are tested on the R-type Ca²⁺ channel amplitude, voltage dependency and probability of opening, by using the cell-attached patch clamp technique (Bkaily 1994, *supra;* 1996, *supra)* and intra and extra patch pipette application of the drug. In all experiment using single channel recording, nifedipine (10⁻⁶M) was present in the control and experimental solutions.

Recent results have recently demonstrated that some cardiogenic and vasoconstrictor hormones such as PAF, ET-1 and bradykinin induced a sustained increase of cytosolic as well as nuclear calcium (data not shown). This sustained increase of Ca²⁺ induced by depolarization of the cell membrane or hormones such as PAF, ET-1 and bradykinin is due to the increase of Ca²⁺ influx through the R-type Ca²⁺ channels at the sarcolemmal membrane and/or the nuclear membrane (Bkaily G., 1994, *supra;* 1996, *supra;* 1997a-d, *supra).* Using Ca²⁺ fluorescence probes Fura-2 or Fluo-3 and 2 and three-dimension Ca²⁺ imaging techniques (Bkaily G., 1994, *supra;* 1996, *supra;* 1997a-d, *supra),* the effect of hormones and drugs could be easily tested. These two methods are used with single cells of different types as described above.

The effects of the compounds of the present invention on cytosolic and nuclear Ca²⁺ in different conditions (K⁺ depolarization, PAF, ET-1, etc.) that increase the probability of opening of the R-type Ca²⁺ channels and induce cytosolic and/or nuclear Ca²⁺ overload (in presence of L-type Ca²⁺ blocker, nifedipine) were tested.

Using Fura-2 or Fluo-3 cytosolic and nuclear Ca²⁺ measurement techniques, the inventors also tested the effect of the compounds of the invention on the spontaneous increase of cytosolic and nuclear Ca²⁺ during spontaneous contraction of ventricular single cells. Single cells from human fetal ventricular cells and chick embryonic cells were bathed in normal Tyrode's solution and spontaneous intracellular Ca²⁺ transient recorded in the absence and the presence of the compounds of the invention.

### EXAMPLE 7

### Effects of MV8608 on TTX-sensitive fast Na⁺ current

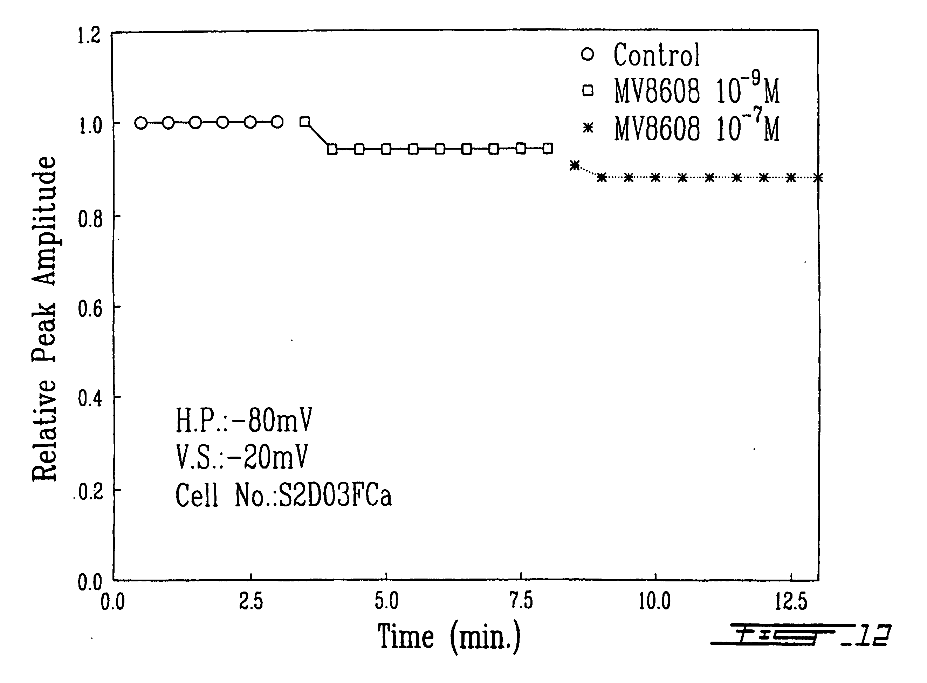

In one series of experiments (n=5), the effect of different concentrations of MV8608 (10⁻¹¹M to 10⁻⁷M) on the TTX-sensitive fast Na⁺ current were tested using the whole-cell voltage clamp technique and experimental conditions reported elsewhere (Bkaily et al., 1988, *supra;* 1993, *supra).* MV8608 was found to have no effect on the TTX-sensitive fast Na⁺ current at all concentrations used and Figure 11 shows an example using a concentration of 10⁻⁷M.

Thus, MV8608 had no effect on the fast Na⁺ current and cannot be used as a depressor or blocker of this channel where its reduction has a therapeutic action such as the case of several local anesthetics and antiarrythmic drugs such as Lidocaine.

### EXAMPLE 8

### Effect of MV8608 on T-type Ca²⁺ current

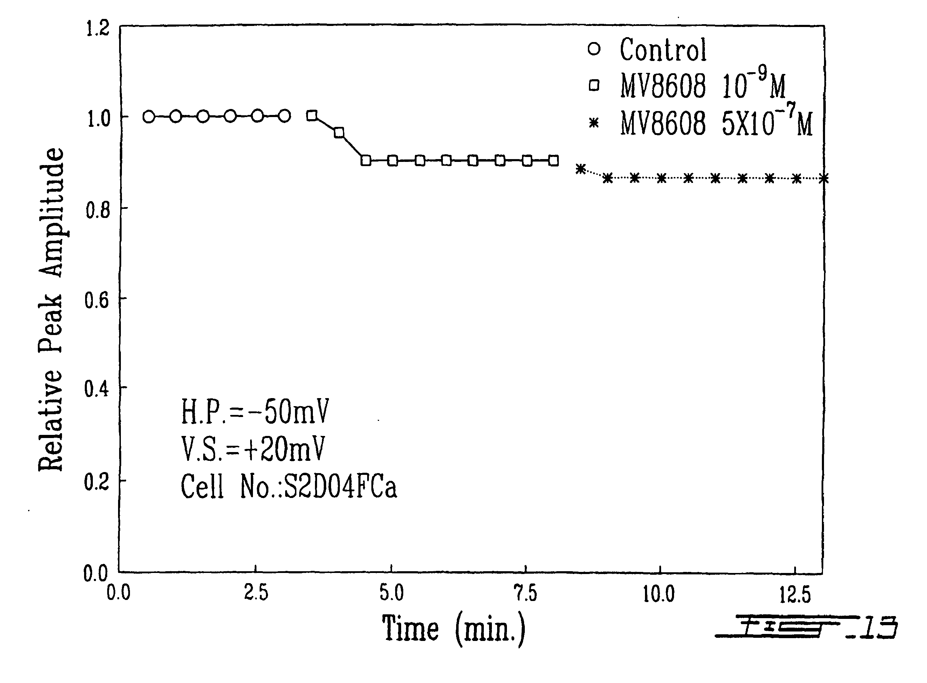

In another series of experiments (n=7), the effect of different concentrations of MV8608 were tested on the T-type Ca²⁺ current (l_{Ca}) using the whole-cell voltage clamp technique and classical experimental conditions described elsewhere by the inventors (Bkaily G. et al., 1991, *supra;* 1992, *supra;* 1993, *supra).* MV8608 had no effect on the T-type I_{Ca} amplitude at a concentration of 10⁻⁹M to 10⁻⁷M and Figure 12 shows an example. As it can be seen in that figure, the inset effect of MV8608 in T-type I_{Ca} is immediate. Thus, the MV8608 was found to be a very weak depressor of the T-type I_{ca}.

These results suggest that MV8608 cannot be used as a potent blocker of the T-type Ca²⁺ channel and in a therapeutic action, wherein its blockade is involved. However, the depressing effect of MV8608 on the T-type I_{Ca} could be useful for example, in combination with other drugs to suppress ventricular tachycardia and fibrillation.

### EXAMPLE 9

### Effect of MV8608 on L-type Ca²⁺ channel

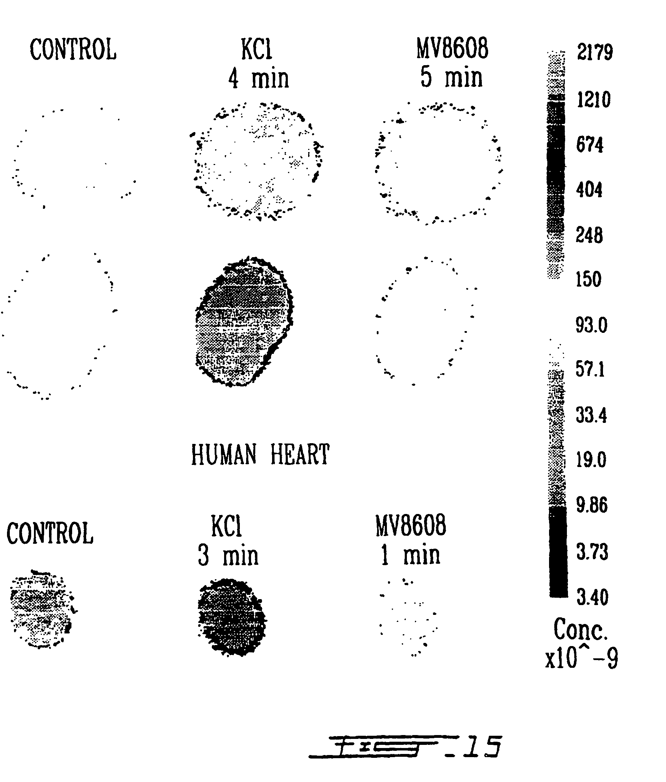

In another series of experiments (n=5), the effect of different concentrations of MV8608 (10⁻¹¹ to 10⁻⁶M) were tested on the L-type I_{Ca} of heart cells of chick embryos using the whole-cell and experimental conditions and protocols described elsewhere by the inventors (Bkaily G. et al., 1993, *supra).* As for the T-type I_{Ca'} the L-type I_{Ca} was not affected by 10⁻¹¹ to 10⁻¹⁰M MV8608. However, increasing the concentration of the compound up to 10⁻⁹M decreased the I_{Ca} amplitude by 10% and a further slight increase was found at a concentration of 5 x 10⁻⁷M of MV8608. Figure 13 shows a typical experiment of the time course effect of the 10⁻⁹ and 5 x 10⁻⁷M concentrations of MV8608.

These results show that MV8608 is a very weak depressor of the L-type Ca²⁺ channel. Thus MV8608 cannot be considered as a high potent antagonist of the L-type Ca²⁺ channel but its depressor effect could be useful when used in combination with known L-type Ca²⁺ antagonist drugs. The weak depressor effect of MV8608 on the T-type Ca²⁺ channel along with the L-type Ca²⁺ channel would be highly beneficial for the treatment of ventricular tachycardia, fibrillation and pathology, where the L-type Ca²⁺ blockers are known and clinicaly used.

### EXAMPLE 10

### Effect of MV8608 on R-type Ca²⁺ channel

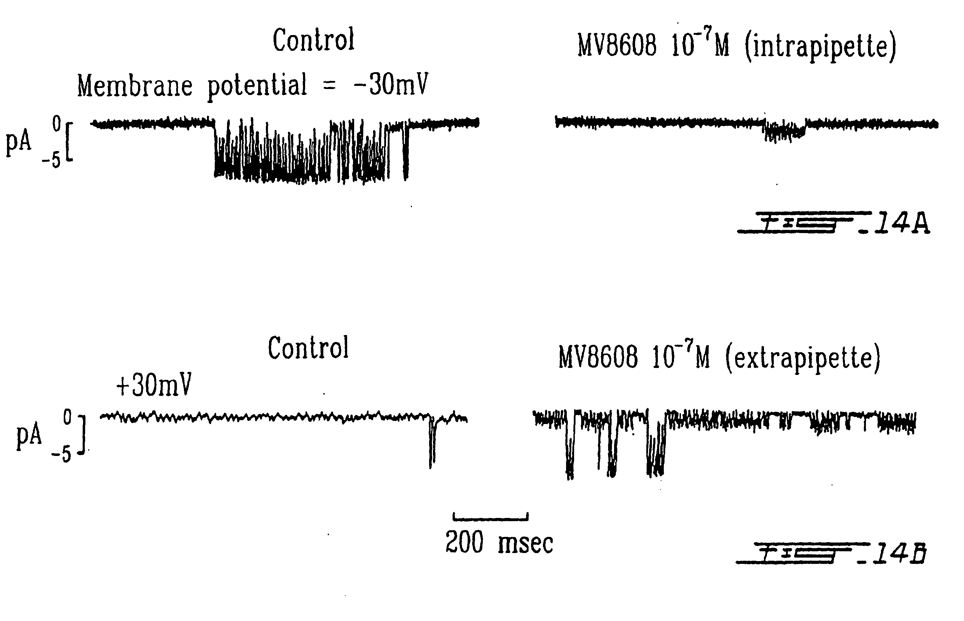

In another series of experiments (n=7), using the cell attached patch clamp technique (Bkaily G. 1994, *supra;* Bkaily G. et al., 1996, *supra),* the effect of 10⁻⁷M of MV8608 on the R-type Ca²⁺ channel in human aortic vascular smooth muscle cell line was tested. In one series of experiments (n=4), in the presence of 10⁻⁶M of nifedipine (10⁻⁶M) in the patch pipette solution containing 110mM Ca²⁺, 10⁻⁷M of MV8608 was applied to the pipette. MV8608 applied in the patch pipette solution decreased the single channel amplitude and probability of opening of the single R-type Ca²⁺ channel under the patch pipette without affecting its single channel conductance. In a second series of experiments (n=3), the patch pipette solution was free of MV8608 and after recording the single channel activities at different voltages, MV8608 (final concentration of 10⁻⁷M) was applied to the extra-patch pipette solution containing 140mM KCI (where the rest of the cell is bathing). This experiment was designed to verify whether MV8608 crossed the cell membrane and if so, whether its effect from the internal side of the channel under the patch is the same as that when applied at the outer side thereof. The results showed that MV8608 did indeed cross the cell membrane. However, instead of decreasing permanently the amplitude and probability of opening of the single R-type channel as did the intra-patch pipette application, the action of MV8608 on the inner side of the membrane increased the probability and duration of opening of the R-type channel. This was accompanied by a spontaneous decrease and release of the blockade of the single channel current. Figure 14 shows a typical single R-type Ca²⁺ channel current in absence and presence of extra-patch pipette 10⁻⁷M of MV8608. Such a pattern of increase of probability of opening and the open duration accompanied with the sporadic decrease of the single channel current amplitude has never been observed when MV8608 was applied at the outer side of the single channel under the patch pipette.

These results highly suggest that the blocking action of MV8608 on R-type Ca²⁺ channel is located mainly at the outer side of the channel. Furthermore, it strongly suggests that its capability of crossing the cell membrane enables the compound to increase fleckering and duration of opening of the channel which in turn makes the external inhibitory site of the channel accessible to the external molecules of MV8608. Taken together, these results highly suggest that the inhibitory action of MV8608 requires the R-type Ca²⁺ channel to be in the open state (overstimulated) and also depends on the frequency of opening of the R-type Ca²⁺ channel. This may explain, at least in part, the preventive as well as therapeutic action of MV8608, on the overstimulation of the R-type Ca²⁺ channel as will be shown below using Fura-2 and Fluo-3 Ca²⁺ measurement techniques.

These results demonstrate that MV8608 does block efficiently the R-type Ca²⁺ at the open state of the channel (i.e. in state of overstimufation). The blockade of the R-type Ca²⁺ by MV8608 should reduce the sustained Ca²⁺ overload that occurred during many abnormal cell function such as for example sustained vasoconstriction and hormone secretion, self-perpetuating hormone secretion of spontaneously active proliferating cells in atherosclerosis, cancer cells proliferation, acute immuno-reaction, arthritis inflammation, pain, ischemia-reperfusion, asthma, acute bronchoconstruction, arrythmia, fibrillation, septic shock and epiptosis.

### EXAMPLE 11

### Effect of MV8608 on R-type Ca²⁺ channel under sustained activation thereof

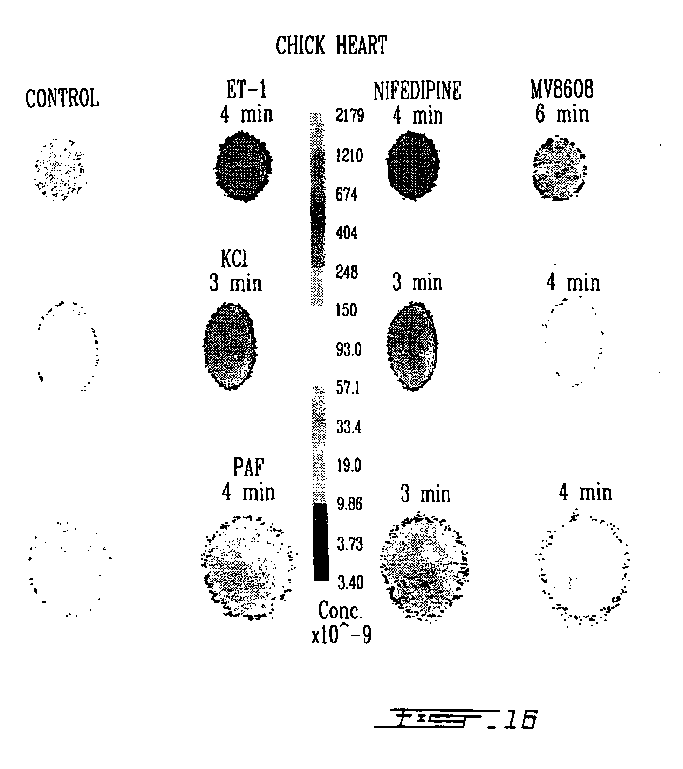

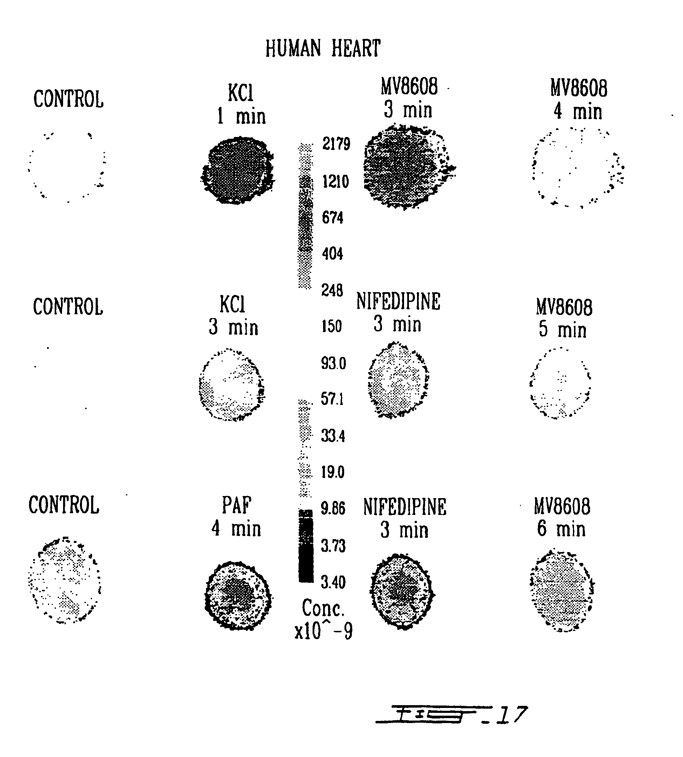

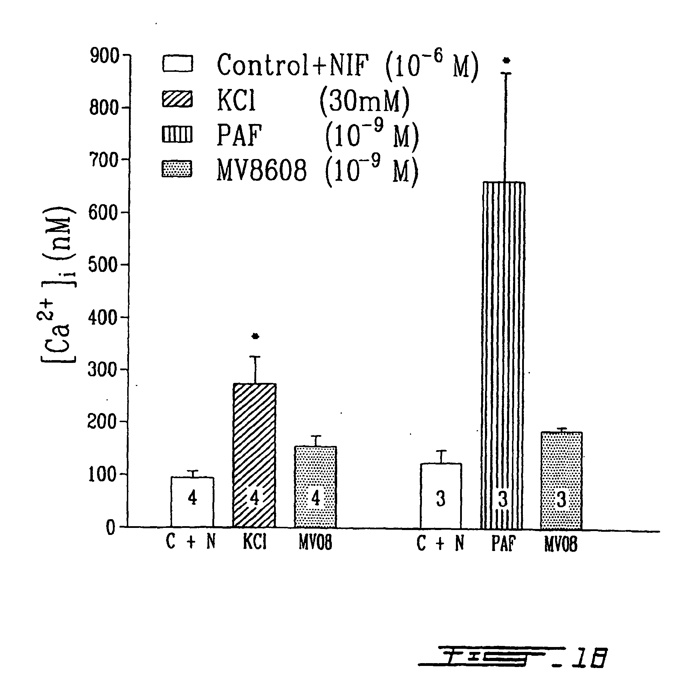

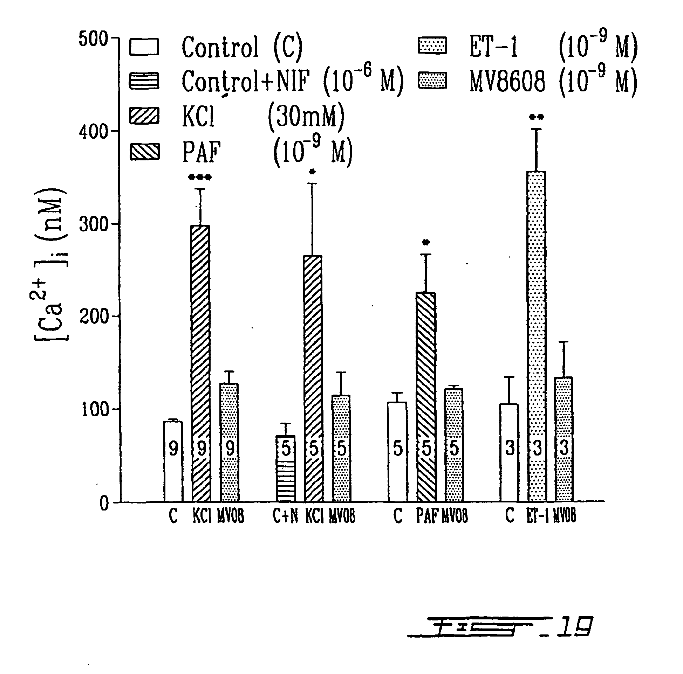

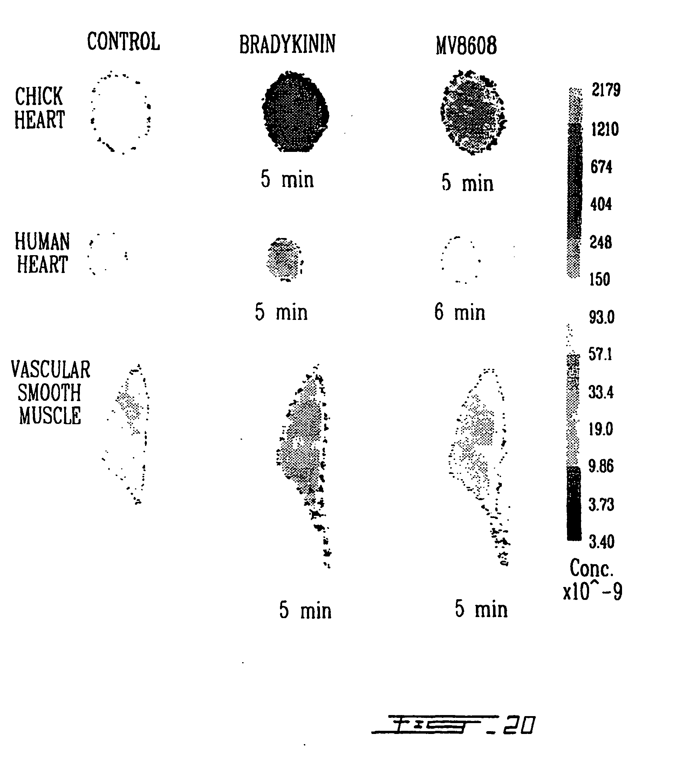

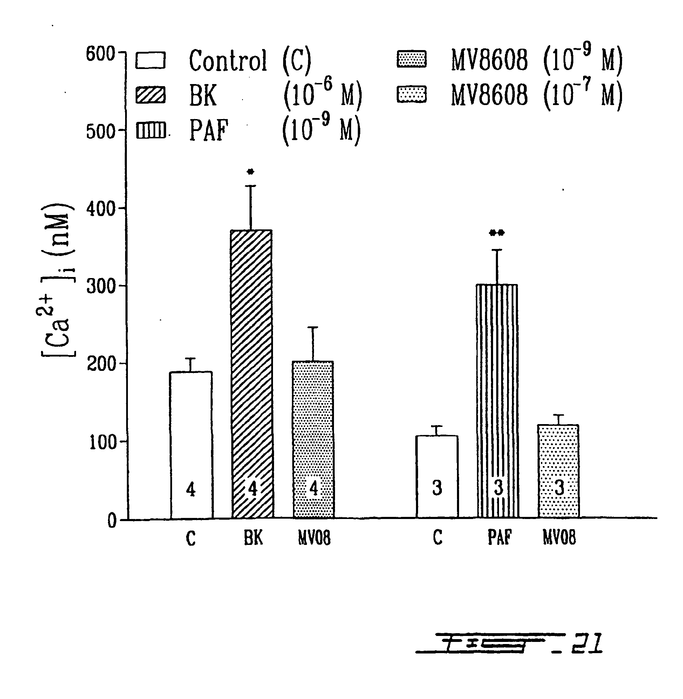

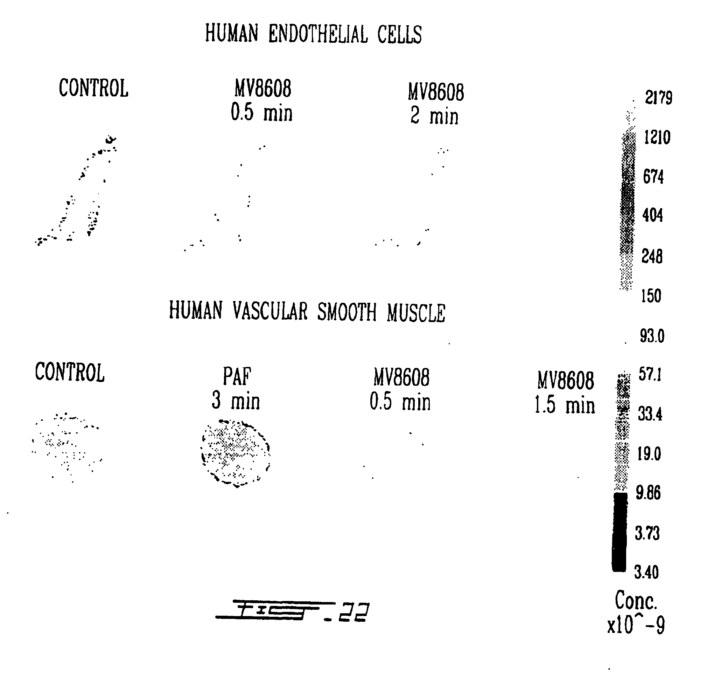

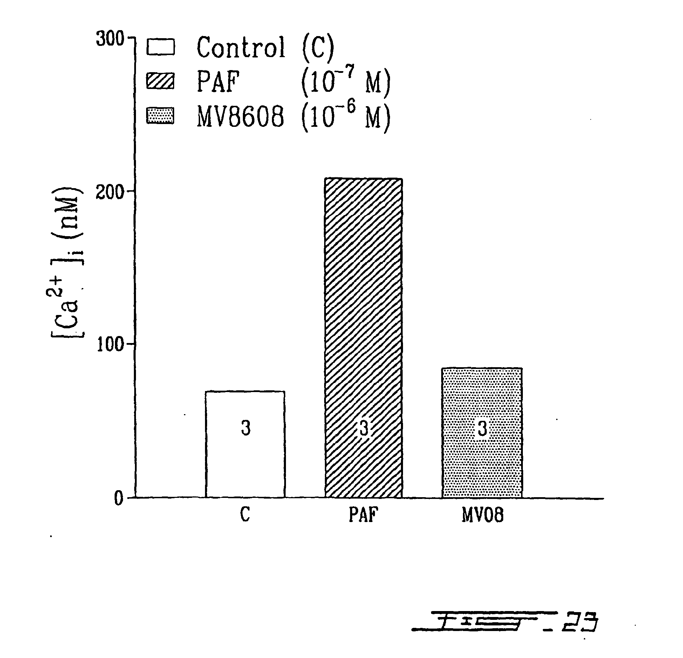

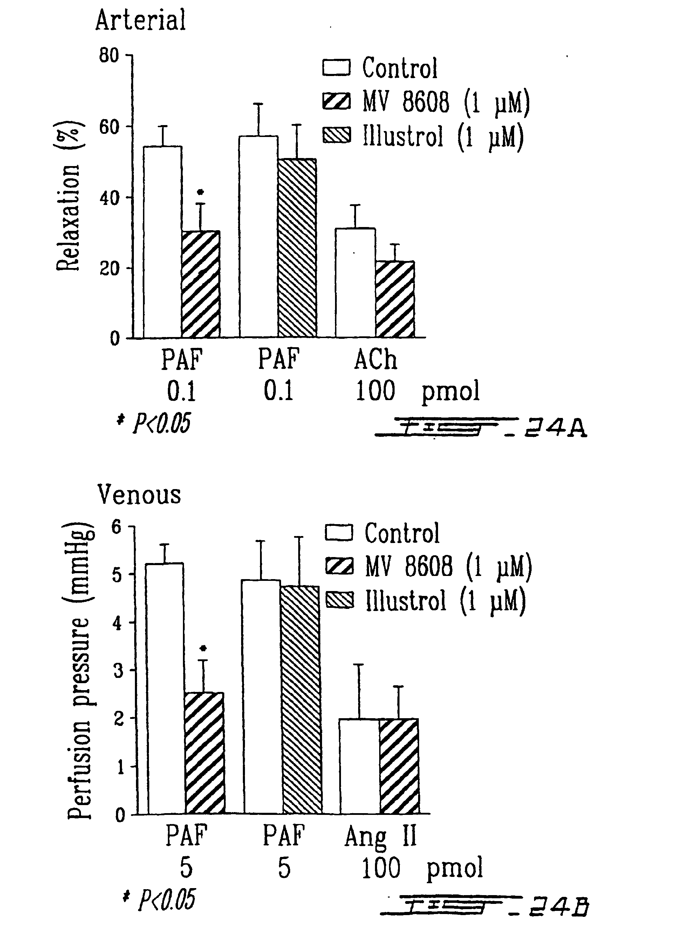

In another series of experiments we tested the effect of MV8608 (10⁻⁹M) on R-type Ca²⁺ channels stimulation-induced sustained increase of total intracellular Ca²⁺ by sustained depolarization (Figures 15 to 19), by PAF (10⁻⁹M) (Figures 16 to 19 and 21 to 22), by ET-1 (10⁻⁹M) (Figures 16 and 19) and bradykinin (BK, 10⁻⁶M) (Figures 20 and 21), in embryonic chick heart cells (Figures 15, 16, 19 and 20), 19-week-old human fetal heart cells (Figures 15,17,18 and 20), rabbit aortic vascular smooth muscle (VSM) cells (Figures 20 and 21), human aortic VSM cell-line (Figure 23) and freshly isolated (Figure 22) as well as in freshly isolated aortic endothelial cells (Figure 22).

As can be seen in these results, sustained activation of the R-type Ca²⁺ channel induced by a sustained increase of [Ca]ᵢ induced by a sustained depolarization or sustained superfusion with a relatively low concentration of a hormone such as ET-1 (10⁻⁹M), PAF (10⁻⁹M) and high concentration BK (10⁻⁶M), was completely blocked by 10⁻⁹M of MV8608 and this effect occurred within 4 to 5 min in the presence of the R-type Ca²⁺ blocker. In addition, these results showed that the pure L-type blocker, nifedipine (10⁻⁷M to 10⁻⁵M) had no effect on the R-type Ca²⁺ channel or on the sustained increase of [Ca]ᵢ - induced stimulation of the R-type Ca²⁺ channel (Figures 16 to 23). Furthermore, the pure L-type Ca²⁺ channel blocker did not prevent MV8608 from blocking the stimulation of R-type Ca²⁺ channel induced sustained increase of [Ca]ᵢ (Figures 18 and 19). In some experiments, the stimulation of the R-type Ca²⁺ channel was elevated by increasing the concentration of PAF from 10⁻⁹M up to 10⁻⁷. Under such conditions, 10 M⁹ of MV8608 failed to significantly decrease the sustained increase of [Ca]ᵢ induced by 10⁻⁷M PAF. Only a concentration of 10⁻⁶M of MV8608 was able to block the high PAF effect on the sustained increase of [Ca]ᵢ (Figure 23).

Taken together, these results demonstrate that MV8608 blocked the R-type Ca²⁺ channel in all cell types used including the human osteoblast cancer cell lines (MG63 and FAOS-2), human VSM cells isolated from atherosclerotic patients, arterial and venous endothelial cells, endocardiac endothelial cells, T-lymphocytes and platelets (not shown) and spontaneously proliferative human aortic vascular smooth muscle cells, AOSMC-9 (Figure 28).

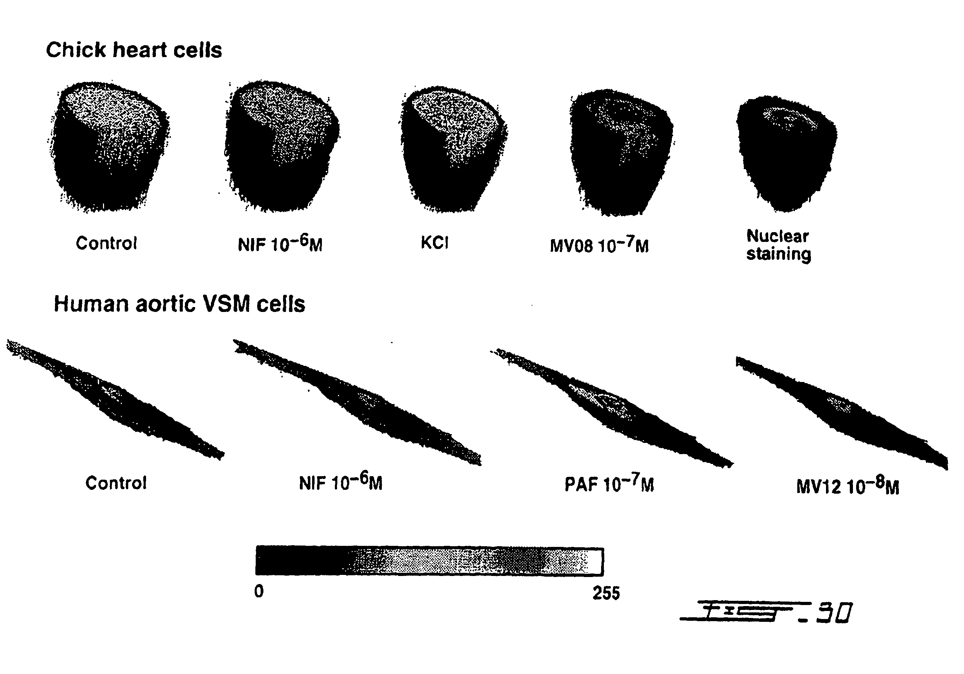

In another series of experiments confocal microscopy was used with Fluo-3, 3-dimension Ca²⁺ measurement techniques in order to verify if the blockade of the R-type Ca²⁺ channel by MV8608 could block both the sustained increase of cytosolic ([ ]_{c}) and (nuclear ([ ]ₙ) free Ca²⁺ induced by the stimulation of the R-type Ca²⁺ channels induced by a sustained increase of [Ca]ᵢ. As can be seen in Figures 29 and 30, and as previously reported (Bkaily et al., 1996a, *supra),* in presence of nifedipine, sustained depolarization with 30mM KCI and PAF induced a sustained increase of both [Ca]_{c} and [Ca]ₙ (largely nuclear). The MV8608 blocked both the cytosolic and nuclear Ca²⁺ sustained overload with a concentration of 10⁻⁷M. As was shown using the Fura-2 total [Ca]ᵢ measurement technique, only at 10⁻⁶M did the compound significantly decrease the sustained increase of [Ca]_{c} and [Ca]ₙ induced with high concentration of PAF (10⁻⁷M) back to the control level (Figure 29B). In addition to its blocking of the depolarization induced sustained increase of [Ca]ᵢ , MV8608, at a concentration of 10⁻⁸ M and 10⁻⁷ M, prevented the stimulation of the R-type Ca²⁺ channel by the sustained depolarization in a dose-dependent fashion, thus decreasing back the [Ca]_{c} and [Ca]ₙ to the control level (Figure 29A). Extracellular applications of the Ca²⁺ chelator EGTA further decreased the [Ca]ᵢ mainly at the nucleus level.

These results demonstrate that blockade of the R-type Ca²⁺ by MV8608 blocked both [Ca]_{c} and [Ca]ₙ sustained overload. In contradistinction to what was shown for high PAF (10⁻⁷ M) induced sustained increase of [Ca]ᵢ, [Ca]_{c} and [Ca]ₙ, MV8608 succeeded in preventing the high PAF action at a concentration of 10⁻⁹M. Thus, this compound seems to be equally effective in acute sustained Ca²⁺ overload, however, it is more effective as a preventive blocker of the R-type Ca²⁺ channel in chronic overstimulation of the channel.

Taken together, these results showed that MV8608 is a more effective R-type Ca²⁺ channel blocker in acute rather than in chronic situations. However, it seems to be more effective in preventing rather than treating a chronic stimulation of Ca²⁺ influx through the R-type Ca²⁺ channel. Non-limiting examples of acute situations include septic shock, acute asthma attacks and bronchospasm. Non-limiting examples of chronic situations include cystic fibrosis, rheumatoid arthritis, pulmonary oedema and hypertension caused by arteriosclerosis.

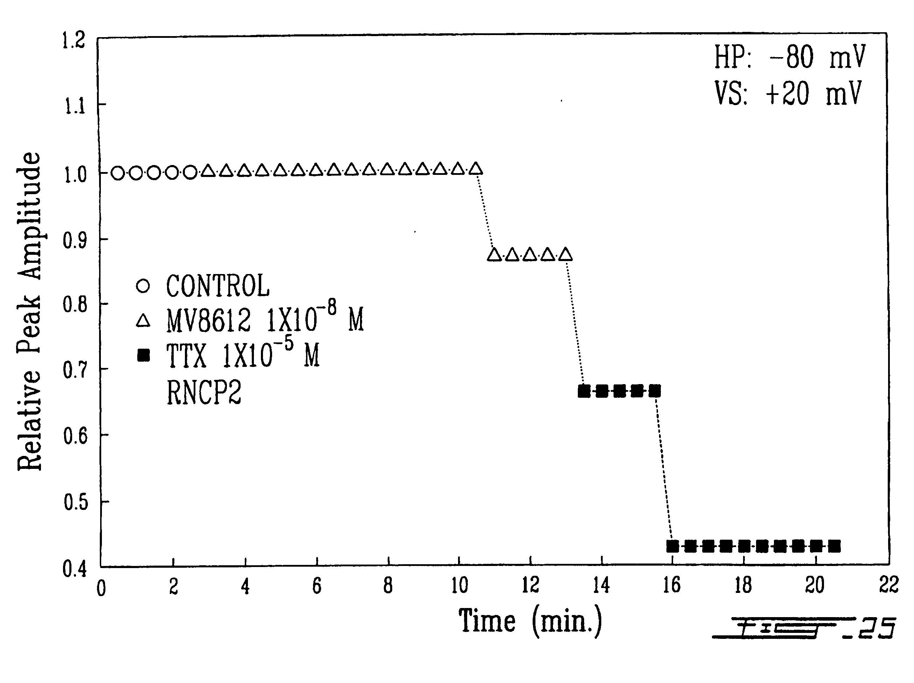

In another series of experiments using the double-perfused bed of the rat (Claing A. et al., 1994, *supra),* MV8608 was found to induce a concentration-dependent inhibition of the PAF-induced responses (Figure 24). The inhibitory properties of MV8608 are specific for PAF as the response to ACh (arterial) and Angll (venous) are unaffected by the pregnane-containing moiety.

In summary, the potency of blockade of the R-type Ca²⁺ channel by MV8608 depends on the degree of the sustained Ca²⁺ overload at the cytosolic and nuclear Ca²⁺, thus it depends on the degree of overstimulation of the R-type Ca²⁺ channel. Consequently, a blockade of the R-type Ca²⁺ channel by MV8608 will be beneficial in pathological situation where sustained cytosolic and nuclear Ca²⁺ take place (such as those described above).

### EXAMPLE 12

### Effect of MV8612 on TTX-sensitive fast Na²⁺ current

In a series of experiments (n=5), we tested the effect of another MV compound MV8612 on the TTX-sensitive fast Na⁺ current (I_{Na}) of embryonic chick heart cells using the whole-cell voltage clamp technique described above. Superfusion with 10⁻⁹M of MV8612 had no effect on the fast Na⁺ current. However, increasing the concentration up to 10⁻⁸M decreased the I_{Na} amplitude by 15% and no further decrease was found at 10⁻⁷M. Figure 26 shows a typical example of the time course effect of 10⁻⁸M MV8612 on the peak amplitude of the fast I_{Na}.

These results show that unlike MV8608, MV8612 depresses the fast Na⁺ current in heart cells. This compound could be used in situations where depressing of the fast Na⁺ channels is beneficial. Non-limiting examples of such situations include anythmia, local anaesthetic, and pain. The compound can also br used in combination with drugs acting on fast sodium channel such as lidocaine.

### EXAMPLE 13

### Effect of MV8612 on L-type Ca²⁺ current

In another series of experiments (n=4), we tested the effect of MV8612 on the L-type Ca²⁺ current (I_{Ca}) in chick embryonic heart cells using the whole-cell voltage clamp technique referred to above. Superfusion with 10⁻⁹M of MV8612 decreased I_{Ca} amplitude by 10% and increasing the concentration of the compound up to 10⁻⁸M further decreased the current amplitude by 25%. Higher concentration of MV8612 (10⁻⁷M) decreased the I_{Ca} amplitude by 62%. These results showed that MV8612 possesses a more potent L-type Ca²⁺ channel antagonist properties than that of MV8608.

The relatively high depressing effect of the L-type Ca²⁺ channel by MV8612 would be beneficial where L-type Ca²⁺ blockade are usually used such as, for example, ventricular tachycardia and hypertension.

### EXAMPLE 14

### Effect of MV8612 on R-type Ca²⁺ channel

In another series of experiments (n=7), the effect of 10⁻⁷M of MV8612 was tested on the R-type Ca²⁺ in human aortic VSM cells, using the single channel cell attached recording technique and intra and extra patch pipette application of drugs. As for MV8608, the R-type Ca²⁺ channel was recorded in the presence of 10⁻⁶M of the L-type Ca²⁺ channel blocker nifedipine (in the patch pipette) and using extra-patch pipette solution (containing 140mM KCI) that mimics the intracellular ionic concentration (to zero the extra-pipette cell membrane potential).

In one series of experiments (n=3), MV8612 (total concentration, 10⁻⁷M) was only applied to the patch pipette. As shown in Figures 27A-C, application of 10⁻⁷M MV8612, via the patch pipette, significantly decreased the R-type single Ca²⁺ channel current amplitude (by about 75% of the control value panel A) and the probability and the opening time of the R-type Ca²⁺ channel (Panel B and C). As for MV8608, in one series of experiments (n=4), MV8612 (10⁻⁷M) was only applied to the extra-patch pipette, in order to verify if the compound penetrates into the cytosol and if so, how it affects the R-type Ca²⁺ channel activity. As shown in Figures 27D and E, extra-patch pipette application of MV8612 rapidly increased the R-type Ca²⁺ opening frequence and was accompanied by a small decrease in the single channel current at all sustained membrane potential (HP) levels used (Figure 27 shows example at HP of -30 where control channel opening is high and a +10mV where control channel opening is low).

These results demonstrate that as for MV8608, intra-patch pipette application of MV8612, equivalent to extracellular application in normal working single cells or muscle, decreased both R-type Ca²⁺ channel amplitude as well as the probability and duration of opening thereof. However, the extend of blocking of the R-type Ca²⁺ channel amplitude and probability of opening by MV8612 was superior to that of MV8608. Also, these results demonstrate that as MV8608, MV8612 did penetrate the cytosol and did increase the frequency of opening (but not the opening-time) of the channel. However, unlike MV8608, intracellular MV8612 permanently depressed the amplitude of the R-type Ca²⁺ channel. The increase of frequency of opening of the R-type channel by intracellular MV8612 would allow extracellular MV8612 to block the channel activity at the opening state. The large decrease of the R-type Ca²⁺ channel amplitude and activities by intra-patch pipette application of MV8612 when compared to the effect with MV8608 could be due to the permanent decrease of the R-type Ca²⁺ channel by intracellular MV8612 but not by MV8608.

In summary, MV8612 seems to be a more effective blocker of the R-type Ca²⁺ channel than that of MV8608 and this difference is mainly due to the permanent depressing effect of the R-type Ca²⁺ channel by intracellular MV8612. Experiments using [Ca]ᵢ, [Ca]_{c} and [Ca]ₙ as well as *in vivo* work (see below) confirm these results and show a more potent effect of MV8612 on the R-type Ca²⁺ channel when compared to that of MV8608.

Thus, the high potency blockade of the R-type Ca²⁺ channel by MV8612 will be more effective than MV8608 in reducing Ca²⁺ overload that occurred during many abnormal cell function such as those described for MV8608 in example 10. Also, since MV8612 (but not MV8608) depressed the fast Na⁺ channel and the L-type Ca²⁺ channel, this compound will be highly effective not only in acute but also in chronic diseases such as those described in examples 10 to 13.

### EXAMPLE 15

### Effect of MV8612 on [Ca]ᵢ [Ca]_{c}, [Ca]ₙ in the presence of extracellular L-type Ca²⁺ blocker

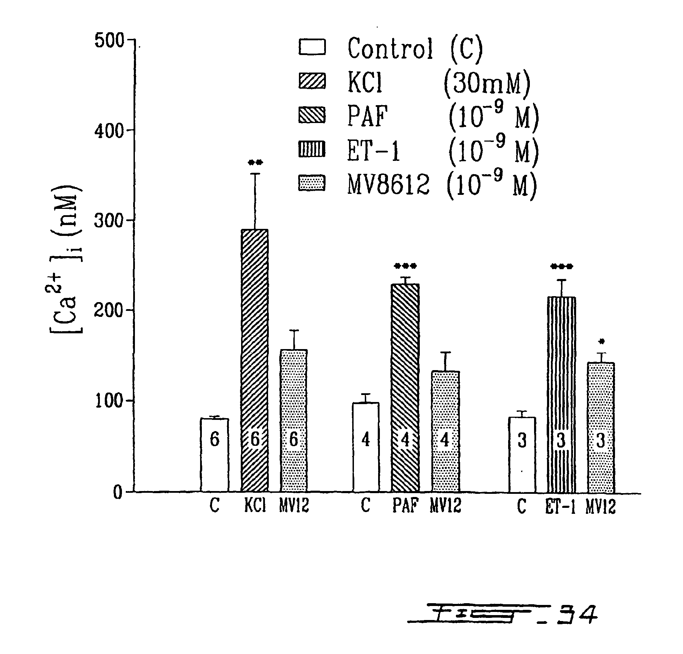

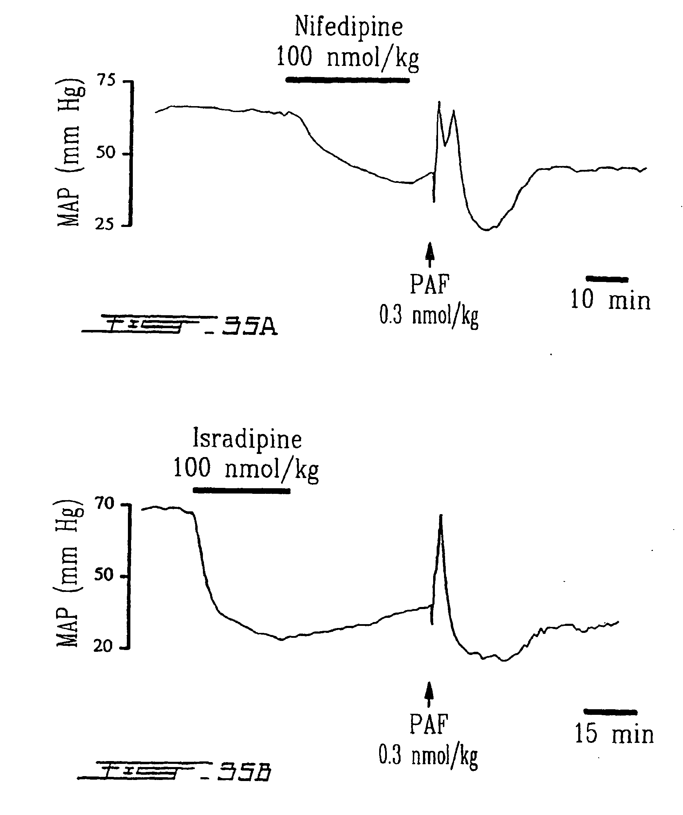

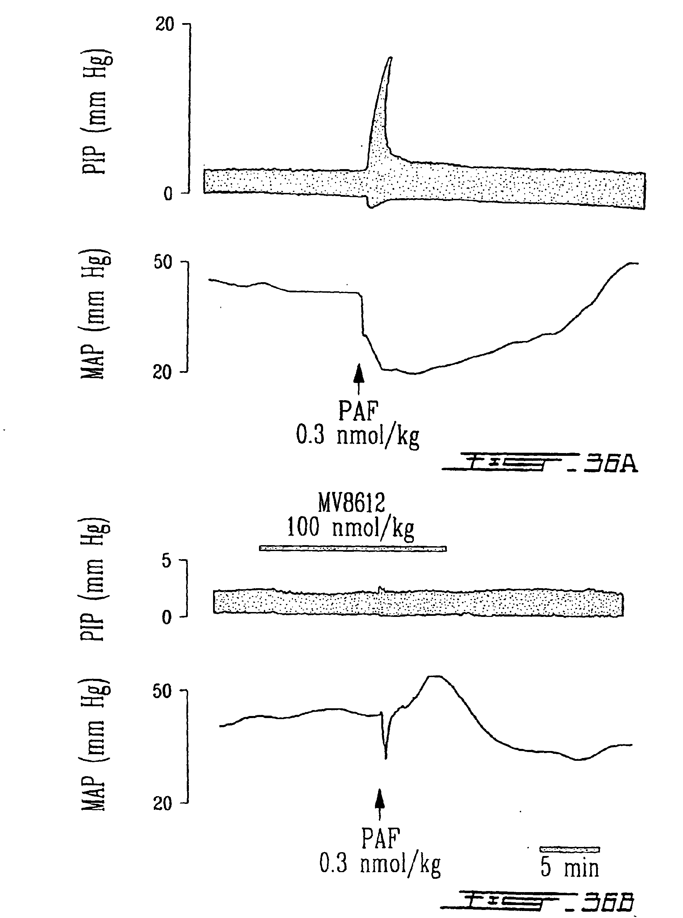

In another series of experiments, as for MV8608, the effect of MV8612 was tested on [Ca]ᵢ, [Ca]_{c}, [Ca]ₙ (in the presence of extracellular L-type Ca²⁺ blocker, nifedipine, 10⁻⁶M) of embryonic chick heart (Figures 29 to 31 and 34) and human aortic VSM cells (Figures 29 to 33) as well as *in vivo* anaesthetized guinea pig (Figure 36).

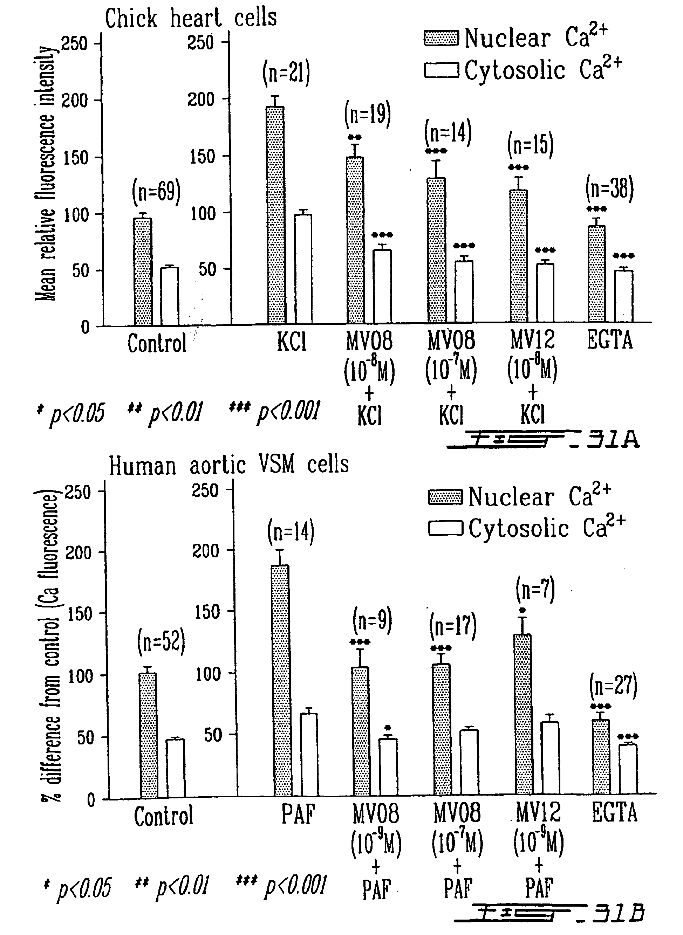

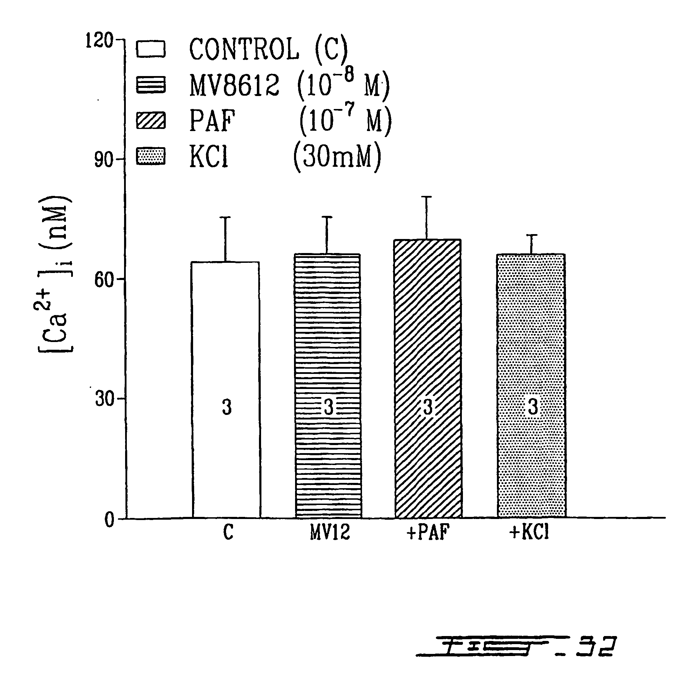

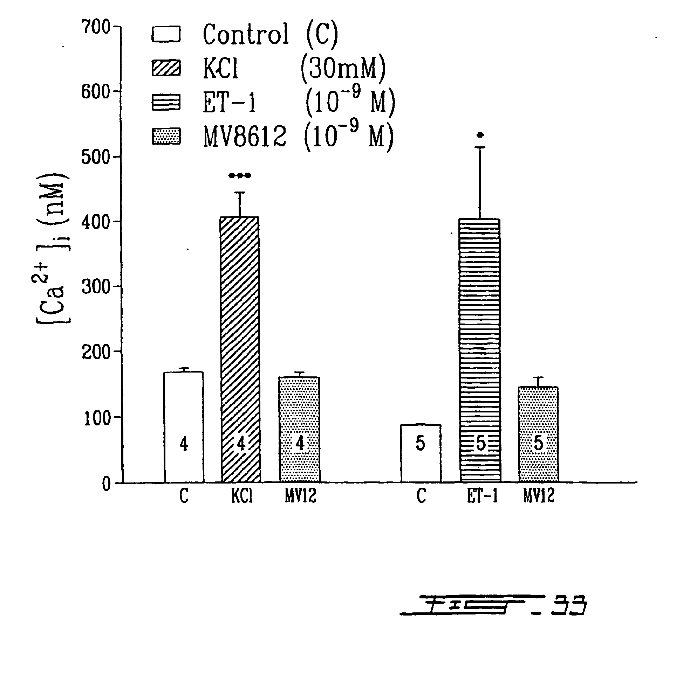

Using Fura-2 [Ca]ᵢ measurement technique, in one series of experiments we tested the effect of MV8612 (10⁻⁹M) on the stimulation of R-type Ca²⁺-induced sustained increase of [Ca]ᵢ by sustained depolarization (Figures 26 and 27), PAF (10⁻⁹M, Figure 34) and ET-1 (Figures 33 and 34). These experiments showed that 10⁻⁹M of MV8612 significantly decreased sustained increase of [Ca]ᵢ induced by sustained depolarization and hormones. Also, MV8612 (10⁻⁸M) was found to prevent stimulation of the R-type Ca²⁺ channel induced sustained increase of [Ca]ᵢ by sustained depolarization and chronic concentration of PAF (10⁻⁷M) (Figure 32). Using 3-dimension Fluo-3 Ca²⁺ measurement of [Ca]_{c} and [Ca]ₙ, MV8612 was found to be a more potent blocker than MV8608 in the overstimulation of R-type Ca²⁺ channel induced sustained increase of [Ca]_{c} and [Ca]ₙ by sustained depolarization and high concentration of PAF (10⁻⁷M) (Figures 29 and 30). MV8612 was also found to be equipotent in preventing the stimulation of the R-type Ca²⁺ channel by sustained depolarization and high concentration of PAF (10⁻⁷M) (Figure 31).

On the other hand, *in vivo* administered PAF, induced a marked hypotension in the anaesthetized rat and guinea pig. In addition, the pro-inflammatory mediator also induced an important bronchoconstriction in the guinea pig, where PAF induced its hypotensive effects through the release of EDRF, its bronchoconstrictive properties are solely mediated by the release of thromboxane A₂.

Characteristically, standard Ca²⁺ blockers such as nifedipine and the dual L and R-type blocker isradipine have marked intrinsic hypotensive properties in the rat (results not shown) and in the guinea pig (Figure 35).

Interestingly, the R-type blocker, MV8612, is devoid of marked intrinsic effect in the guinea pig. As shown in Figure 36, pretreatment of the guinea pig with MV8612 abolished the bronchoconstrictive responses and very significantly reduced the hypotensive effects of PAF. Following withdrawal of the treatment with MV8612, the hypotensive effect of PAF is fully restored in the same animal (for methodology, please refer to Gratton et al., 1995a, Am. J. Hyper. 8:1121-1127). Identical inhibition of MV8612 has been observed on the hypotensive effect of PAF in the anaesthetized rat (for methodology, please refer to D'Orleans-Juste et al., 1996, Can. J. Physiol. Pharmacol. 74:811-817; Gratton et al., 1995a, *supra;* 1995b, Br. J. Pharmacol. 114:720-726).

These results again confirm the more potent effect of MV8612 (when compared to MV8608) on blocking the R-type Ca²⁺ channel and related cytosolic and nuclear Ca²⁺ overload. The high potency effect of MV8612 of the R-type Ca²⁺ combined to its depressing effect of the fast Na⁺ and the L-type Ca²⁺ channels give this compound a higher spectrum of action than that of MV8608 and isradipine. The MV8612 as well as MV8608 are unique compounds that block both the cytosolic and nuclear Ca²⁺ overload. This later effect of MV8608 and especially MV8612 gives these compounds a new target other than that of the cytosolic membrane channels but also a nuclear and a perinuclear ionic channel target blockers. The MV8612 will be beneficial in cell disorders that implicate abnormal Ca²⁺ and Na⁺ transport and preventing cytosolic Ca²⁺ overload such as in diseases cited in examples 7 to 14.

In conclusion, the pharmacological results presented herein support the unique R-type Ca²⁺ channel blocking properties of the MV8608 and MV8612 and their derivatives.

### EXAMPLE 16

### "In vivo" results with compounds MV8608 and MV8612

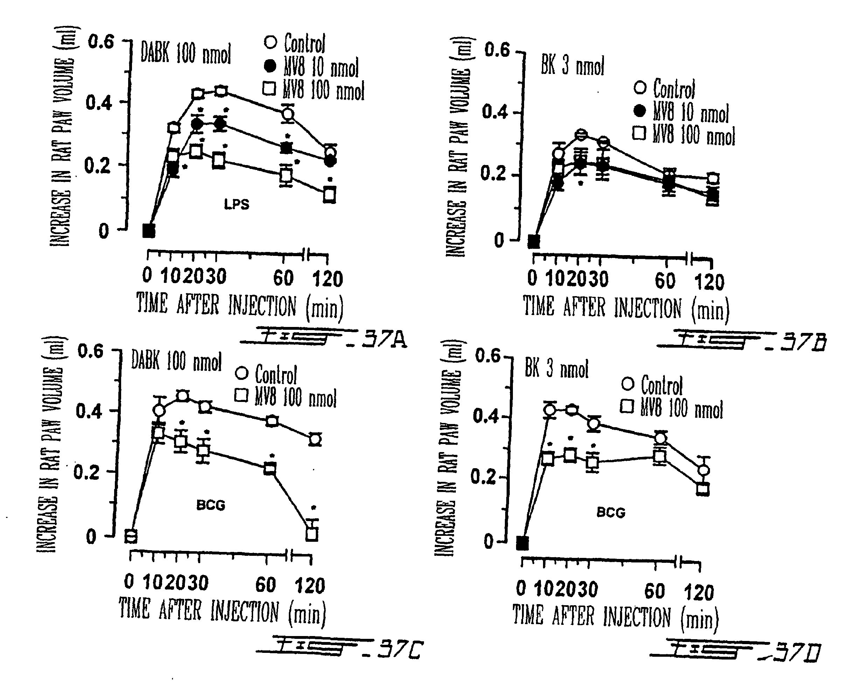

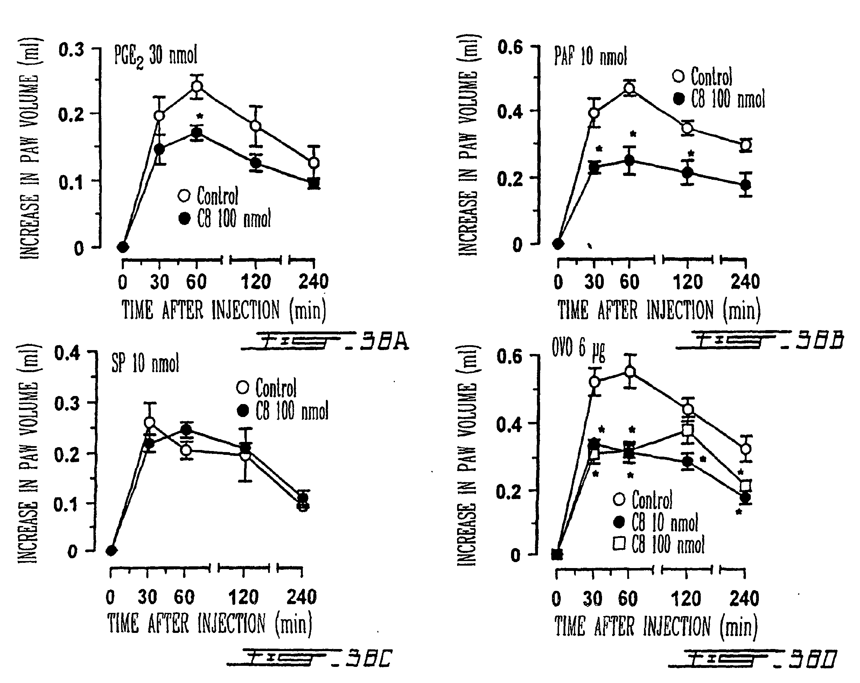

In this experiment (N =5 to 6 animals per group), the anti-oedemagenic action of compounds MV 8608 and MV 8612 against bradykinin and several mediators which are reported to be involved in the inflammatory processes, was evaluated. The procedures used to perform these experiments have been reported elsewhere (Neves et al., 1993, Eur. J. Pharmacol. 243:213-219; Campos et al., 1995, Br. J. Pharmacol. 114:1005-1013; Campos et al., 1996, Br. J. Pharmacol. 117:793-798; Eur. J. Pharmacol. 316:227-286). As can be seen in Figure 37A, MV 8608 (10 and 100 nmol/paw), when co-injected with des-Arg⁹-bradykinin, caused a dose-related inhibition of paw oedema induced by this peptide. In contrast, at similar doses, MV 8608 had no significant effect against bradykinin-induced hindpaw oedema in animals treated with LPS (Campos et al., 1996, *supra)* (Figure 37B). As reported previously, in rats treated 30 days prior to a systemic injection of LPS, both B₁ and B₂ kinin selective agonists caused marked oedema formation (Figures 37C and D (Campos et al., 1996, *supra*)). Under such experimental conditions, compound MV 8608 (100 nmol/paw) significantly inhibited both des-Arg⁹-BK and bradykinin-induced rat paw oedema (Figures 37C and D). However, MV8608 was more effective against B, agonist-mediated oedema formation. In addition, at 100 nmol/paw, compound MV 8608 consistently inhibited the paw oedema induced by PAF (Figure 38B), and partially inhibited the oedema induced by prostaglandin E₂ (PGE₂) (Figure 38A), leaving oedema induced by substance P unaffected (Figure 38C). Interestingly, MV 8608 (10 and 100 nmol/paw) also inhibited significantly oedema formation induced by subplantar injection of ovalbumin in animals that had been actively sensitised to this antigen (Figure 38D).

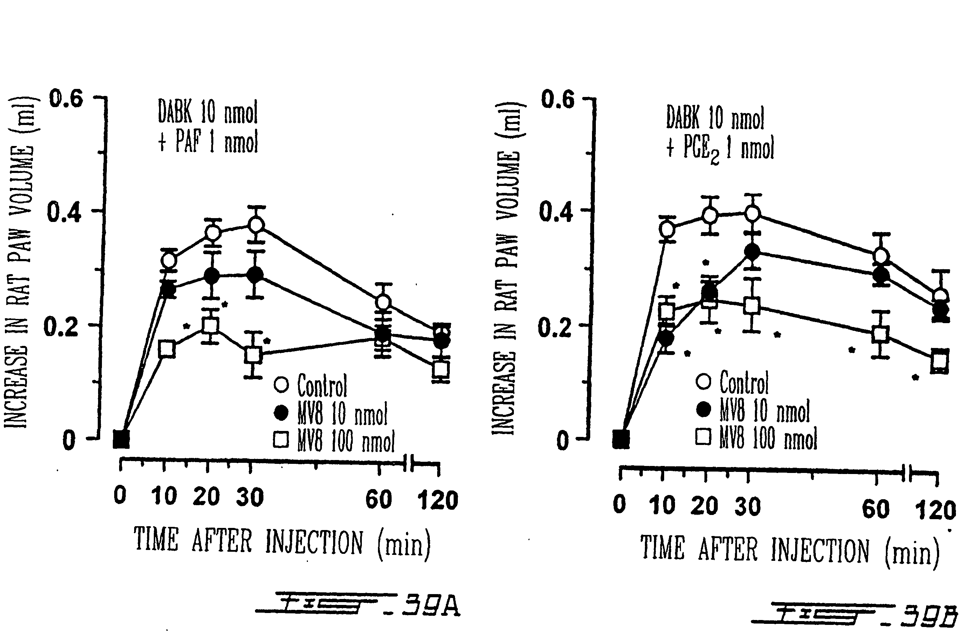

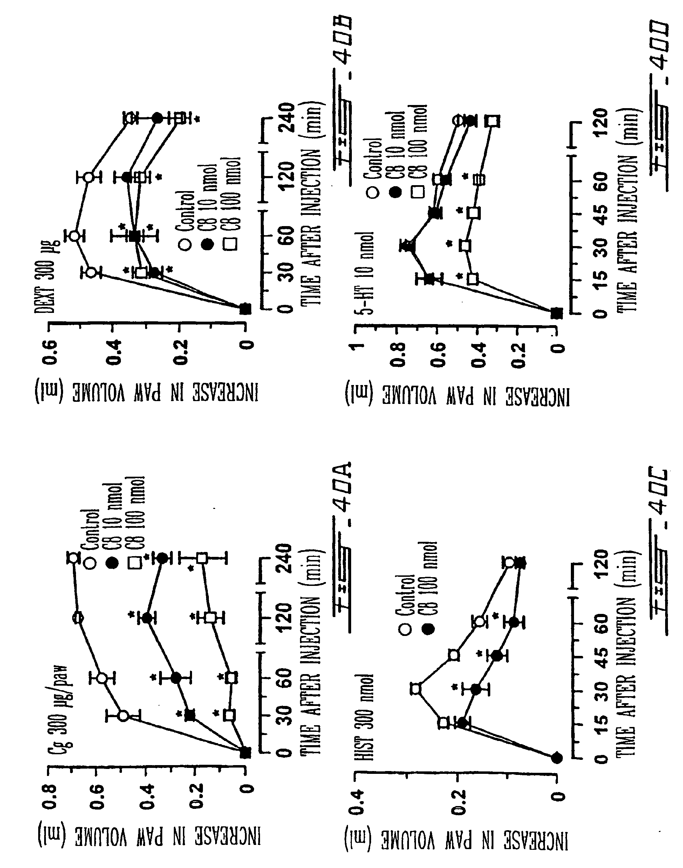

Results of Figure 39 (A and B) demonstrate that MV 8608 (10 and 100 nmol/paw) in a dose-dependant fashion prevented the potentiation of paw oedema caused by association of low dose of des-Arg⁹-BK plus PAF (Figure 39A) or with PGE₂ (Figure 39B). MV 8608 (10 and 100 nmol/paw) when co-injected in association with carrageenan (Figure 40A), dextran (Figure 40B), histamine (Figure 40C) or with serotonin (Figure 40D), significantly prevented the oedemagenic response caused by these substances. However, MV 8608 was much more effective in inhibiting paw oedema induced by carrageenan, an effect which has been reported to be dependent on the release of several mediators, including kinins, histamine, serotonin and PAF (Hargreaves et al., 1988, Clin. Pharmacol. Ther., 44:613-621; Burch et al., 1990, Naunyn-Schimiedeberg's Arch. Pharmacol., 342:189 -193; Damas et al., 1992, Eur. J. Pharmacol., 211:81-86; Damas et al., 1996, Naunyn-Schmiedeberg's Arch. Pharmacol., 354:670-676; De Campos et al., 1996, Eur. J. Pharmacol. 316:227-286).

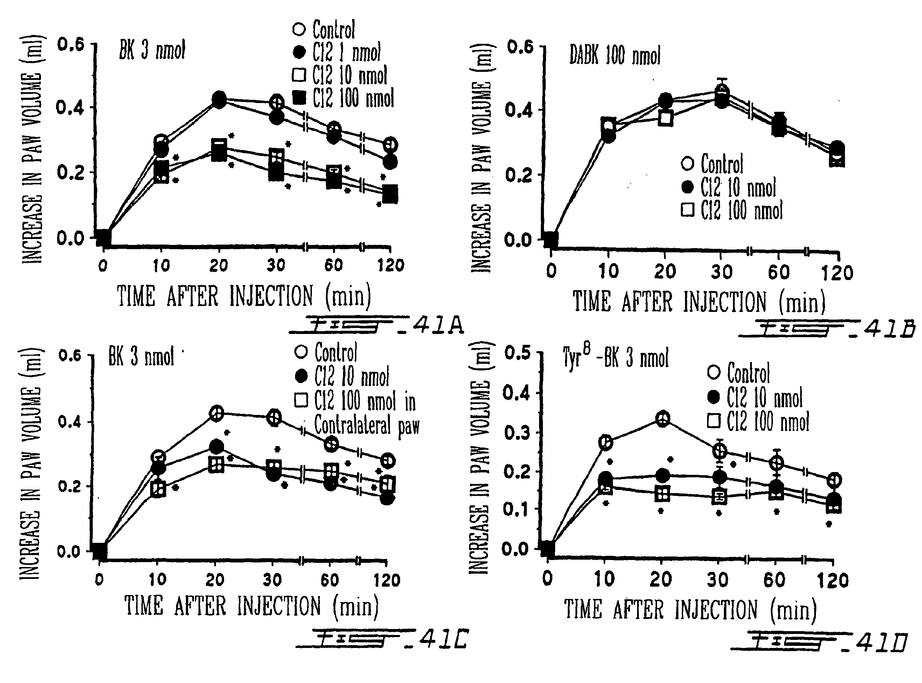

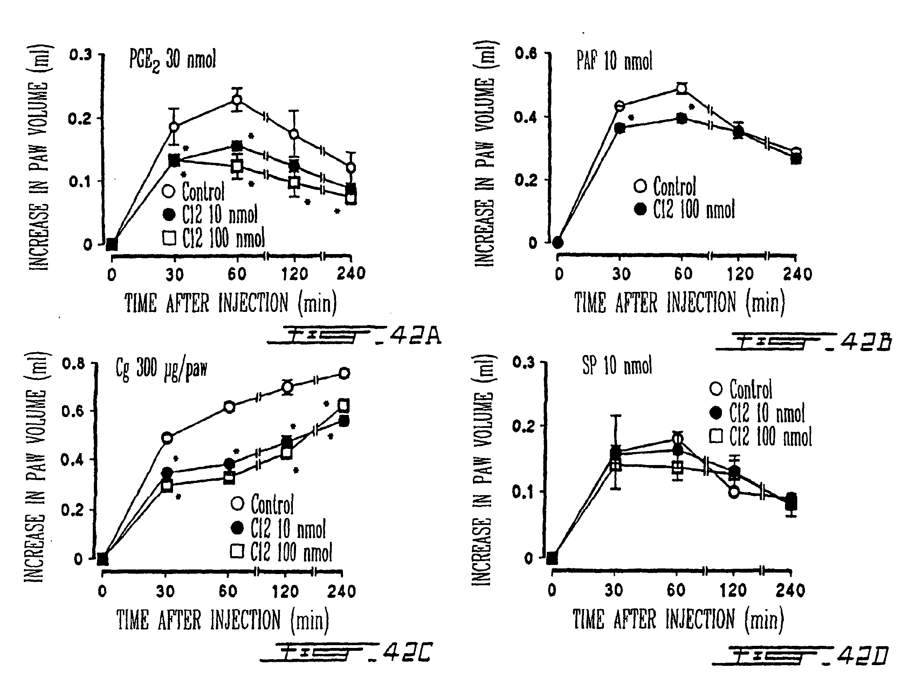

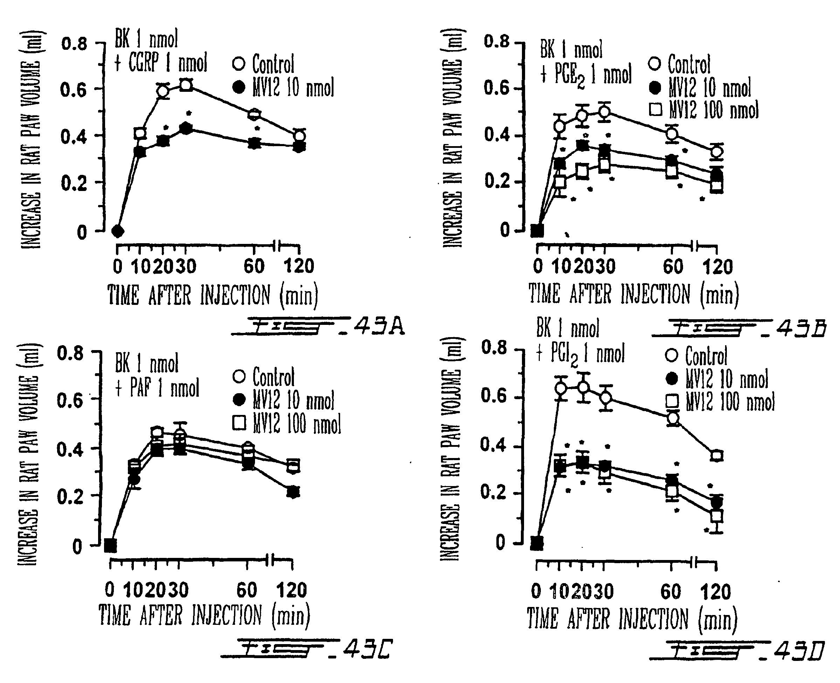

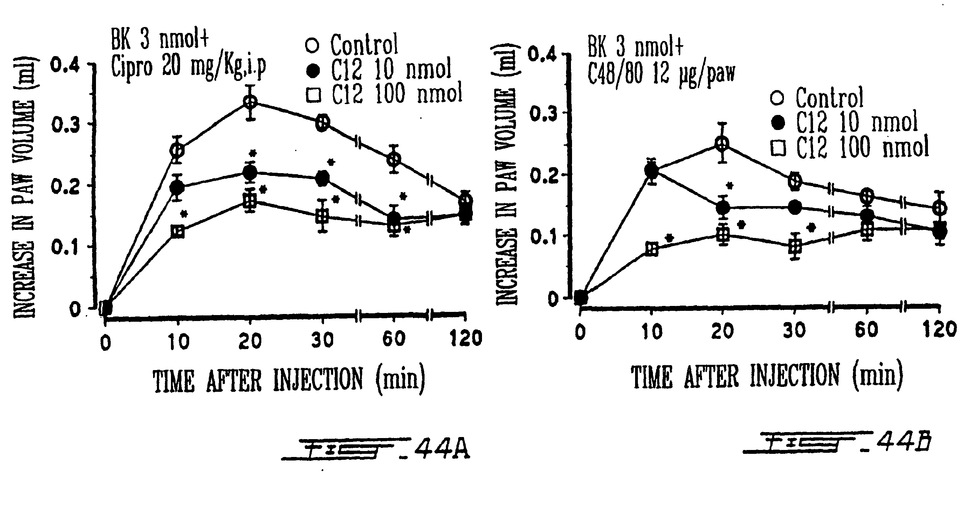

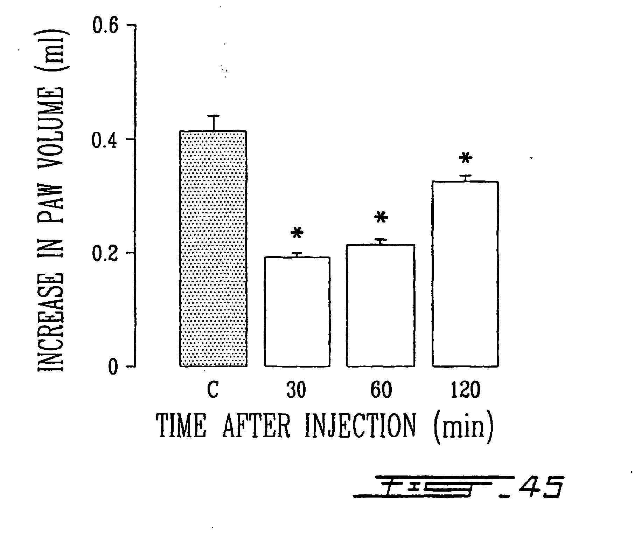

In marked contrast, and confirming previous "in vitro" and "in vivo" results (Calixto et al., 1986, Br. J. Pharmacol. 88:937-941; Calixto et al., 1985, Br. J. Pharmacol., 85:729- 731; 1987, *supra;* 1988, *supra;* 1991a, Prostaglandins, 41:515-526; 1991b, In: Bradykinin Antagonists: Basic and Clinical Research. Ed. by Ronald M. Burch, Marcel Dekker Inc, New York, pp.97-129), MV 8612 (10 and 100 nmol/paw) significantly inhibited bradykinin and the selective B₂ agonist tyr⁸-bradykinin-induced paw oedema (Figures 41A and D), while having no significant effect against oedema-induced by des-Arg⁹-BK in animals treated with LPS (Figure 41B) (Campos et al., 1996, *supra).* The anti-oedematogenic effect caused by MV 8612 seems to be systemic, because the controlateral paw treated with saline also revealed an significant anti-oedemagenic action (Figure 41C). On the other hand, MV 8612 (10 and 100 nmol/paw) dose-dependently inhibited PGE₂ and carrageenan-induced oedema formation (Figure 42A, C), but caused only a minimal inhibition of PAF and substance P-mediated paw oedemas (Figures 42B and D). Compound MV 8612 (10 nmol/paw) significantly inhibited the oedema formation caused by association of low dose of bradykinin plus the calcitonin gene related peptide (Figure 43A), PGE₂ (Figure 43B) and prostacyclin (Figure 43D), but did not interfere with oedema-induced by the association of bradykinin plus PAF (Figure 43C). The anti-oedematogenic action of MV 8612 against bradykinin-induced oedema was independent on the release of histamine, since a dose-related inhibition was still observed in animals treated with cyprohetadine or with compound 48/80 (Figures 44A and B). The inhibition of MV 8612 against oedema caused by bradykinin installs rapidly (30 min) and lasted for at least 2 h (Figure 45).

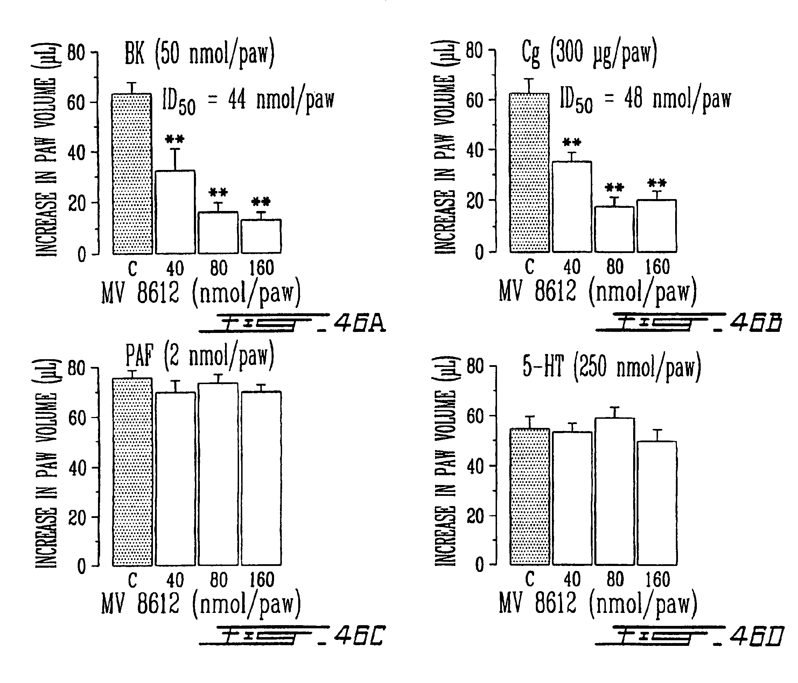

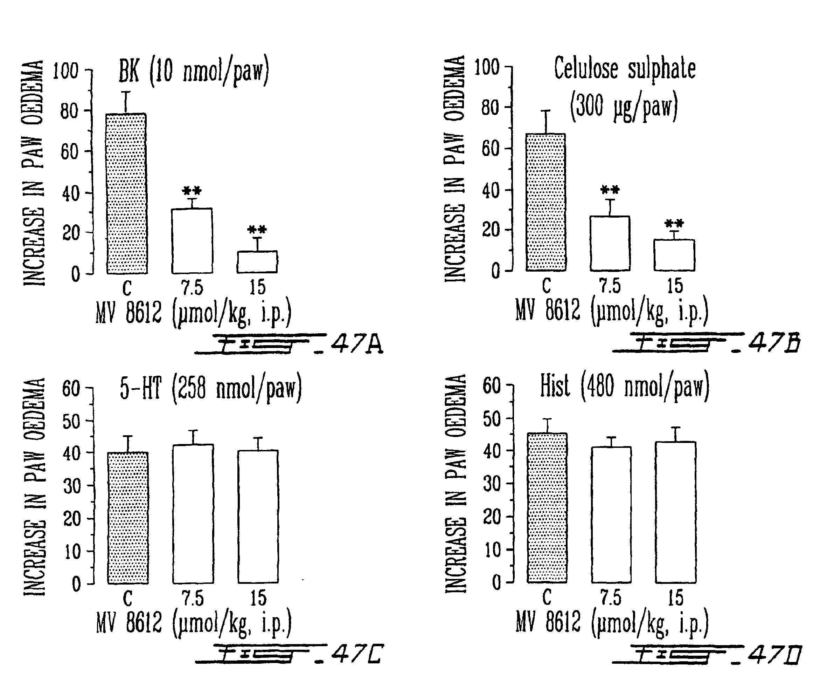

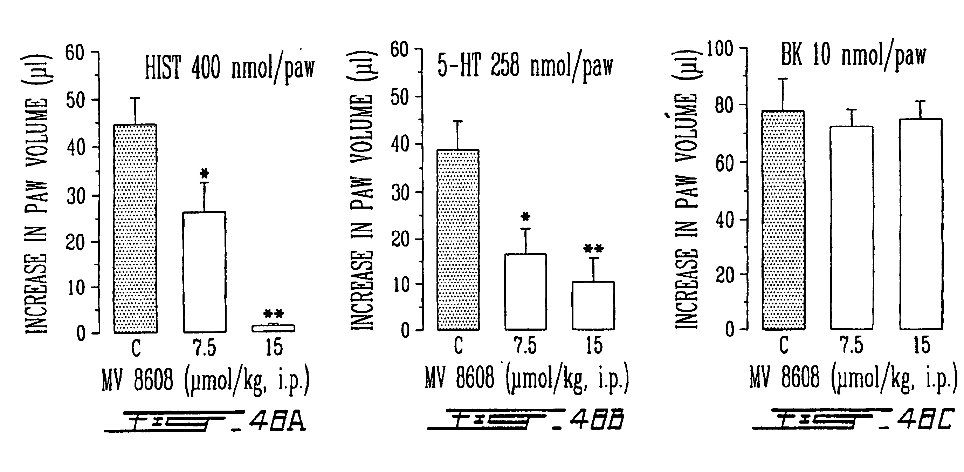

When tested in mice, MV 8612 (40 to 160 nmol/paw) inhibited bradykinin and carrageenan-induced paw oedema in mice in a dose-dependent manner (Figures 46A and B). At the same dose, MV 8612 failed to affect significantly PAF-acether or serotonin-induced oedema formation (Figures 46C and D). Confirming the effect observed with crude extract of *Mandevilla velutina,* when MV 8612 was injected systemically, MV 8612 (7.5 and 15 mol/kg, i.p.), given 30 min prior, produced a dose-dependent inhibition of bradykinin and cellulose sulphate-induced paw oedema (Figure 47A and B). However, at the same range of dose, MV 8612 had no significant effect against paw oedema induced by serotonin and histamine (Figure 47C and D). In marked contrast, compound MV 8608 (7.5 and 15 mol/kg, i.p) caused a dose-related inhibition of histamine and serotonin-induced oedema formation, leaving paw oedema to bradykinin unaffected (Figure 48A ,B and C).

Taken together, these results indicate that both MV 8612 and MV 8608 compounds show potent topical and systemic anti-inflammatory properties through a distinct mechanism of action, albeit through an overstimulation of R-type Ca²⁺ channels. While MV 8612 was more active against inflammatory response caused by bradykinin via stimulation of B₂ receptors, MV 8608 was effective in preventing oedemas elicited by histamine, PAF-acether and by the B, selective agonist des-Arg⁹-BK.

### EXAMPLE 17

### Anti-inflammatory action of MV8612 and MV8608

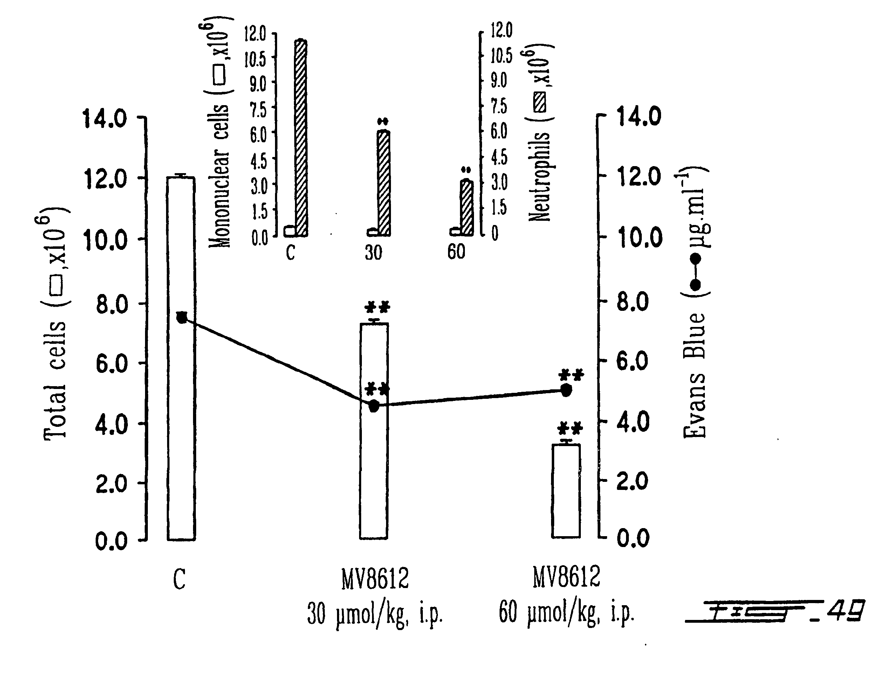

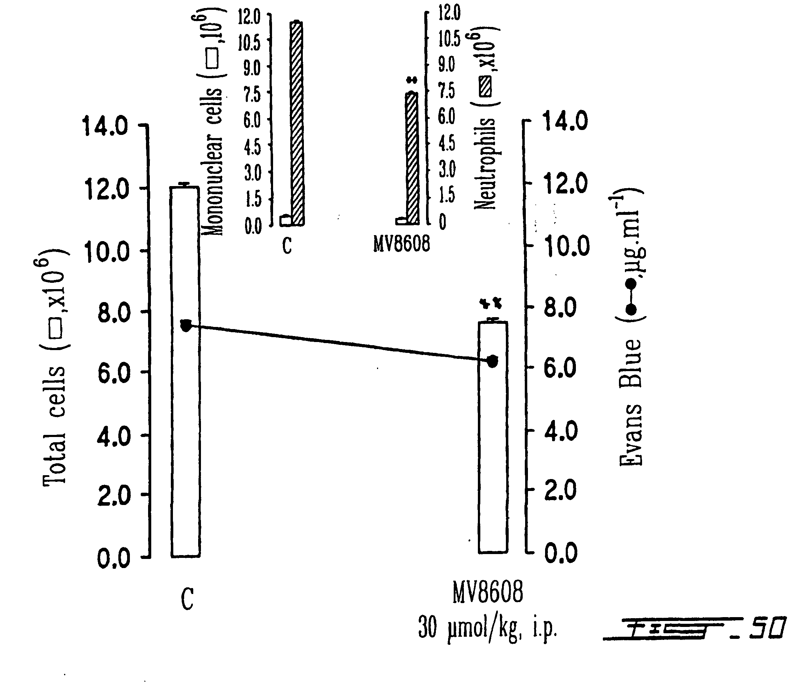

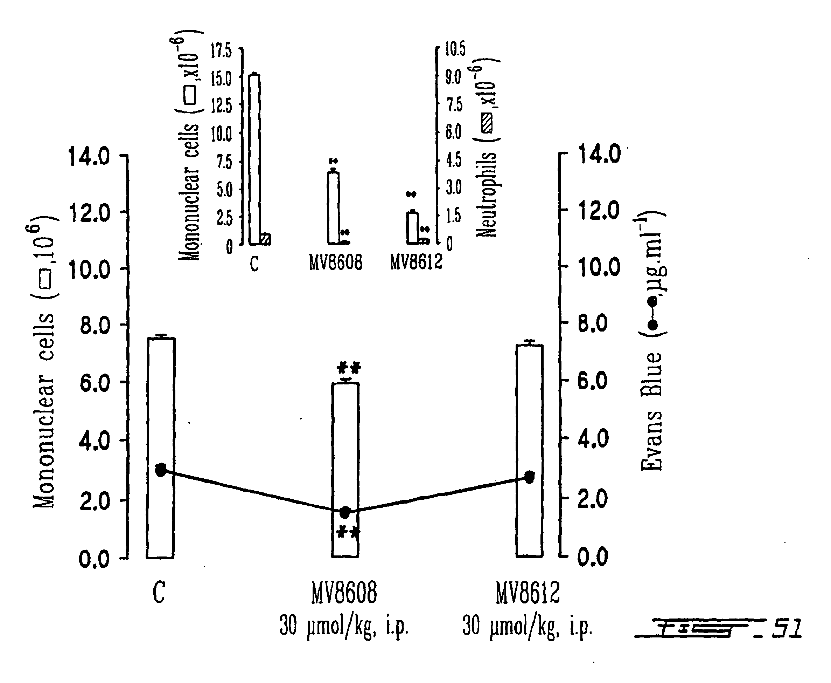

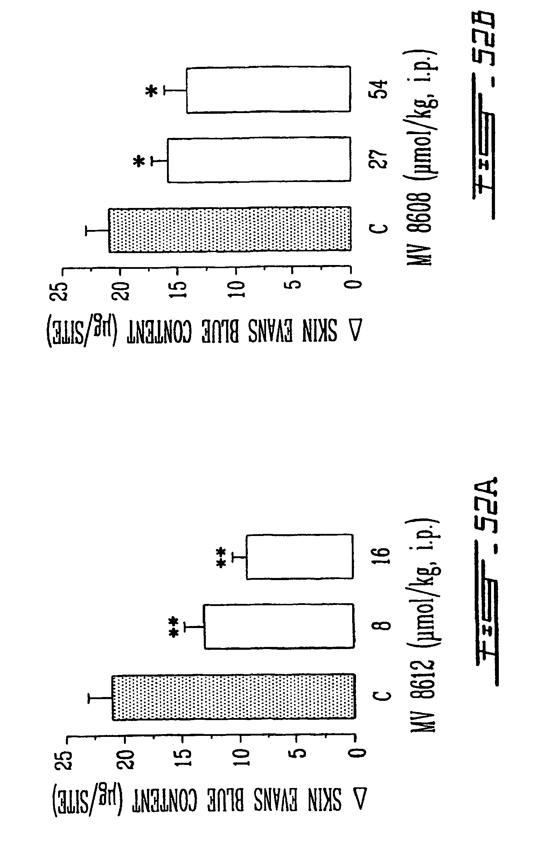

To assess further the anti-inflammatory action of MV 8612 and MV 8608, their effects against the inflammatory responses caused by several mediators of inflammation in a murine model of pleurisy were tested (Saleh et al., 1996, Br. J. Pharmacol 118:811-819). In addition, both compounds were tested against the increase of vascular permeability caused by bradykinin in the rat skin (Neves et al., 1993b, Phytotherapy Research. 7:356-362). Figure 49 shows that compound MV 8612 (30 and 60 mol/kg, i.p.) given 1 h prior, significantly inhibited plasma extravasation (A) as well as the total (B) and neutrophils cells (C) in response to intrapleural injection of carrageenan. Compound MV 8608 (30 mol/kg. i.p) also inhibited significantly the total and neutrophil cells migration caused by intrapleural injection of carrageenan (Figure 50). Confirming the previous results, compound MV 8608 (30 mol/kg, i.p.) also antagonised significantly the plasma extravasation and the mononuclear cells influx caused by intrapleural injection of PAF (Figure 51). In contrast, at the same range of dose, MV 8612 did not affect significantly the inflammatory response caused by PAF acether in a murine model of pleurisy (Figure 51). However, both MV 8612 (8 and 16 mol/kg, i.p) and MV 8608 (27 and 54 mol/kg, i.p.) antagonised in a dose-related manner, the increase of vascular permeability caused by bradykinin in the rat skin (Figure 52A and B). Compound MV 8612 was more active than MV 8608.

This data extend our previous results (Calixto et al., 1991a, *supra;* Zanini et al., 1992, Phytotherapy Research, 6:1-5; Neves et al., 1993b, *supra)* supportting the view that both MV 8612 and MV 8608 exhibit powerful anti-inflammatory properties.

### EXAMPLE 18

### Effect of MV8612 and MV8608 on human lymphocyte proliferation in vivo

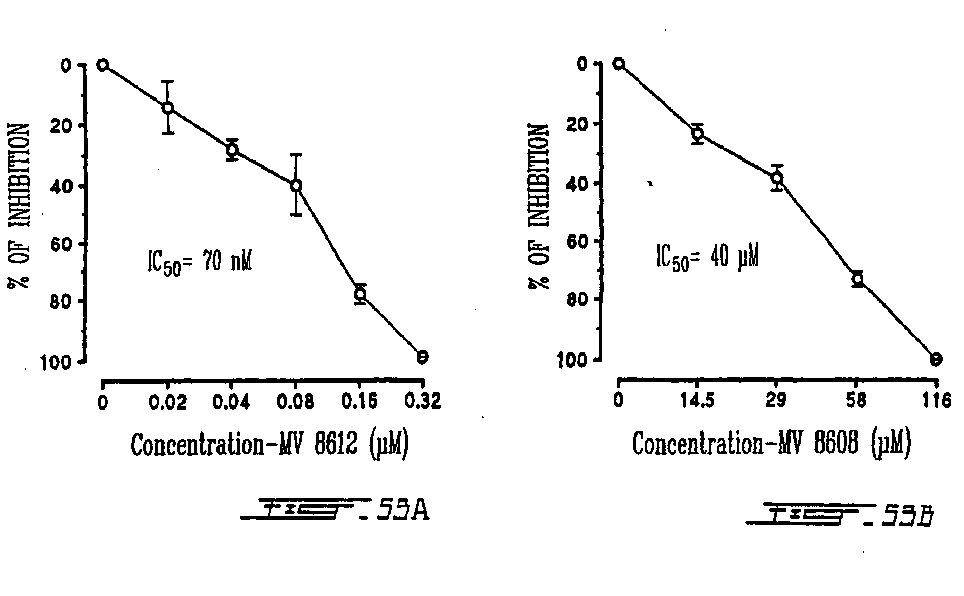

In a separate series of experiments, compounds MV 8612 and MV 8608 were tested to analyze whether they interfered with human lymphocyte proliferation "in vitro". These experiments were carried out as reported previously (Moraes et al., 1996, Eur. J. Pharmacol. 312:333-339). The results of Figure 53(A and B) show that compound MV 8612 (0.02 to 0.32 M) and, to a lesser extent, MV 8608 (14.5 to 116 M) caused graded inhibition of human lymphocyte proliferation, with MV 8612 being about 570 fold more potent. These results may be explained by their above-demonstrated anti-inflammatory properties.

### EXAMPLE 19

### Antinociceptive actions of MV8612 and MV8608

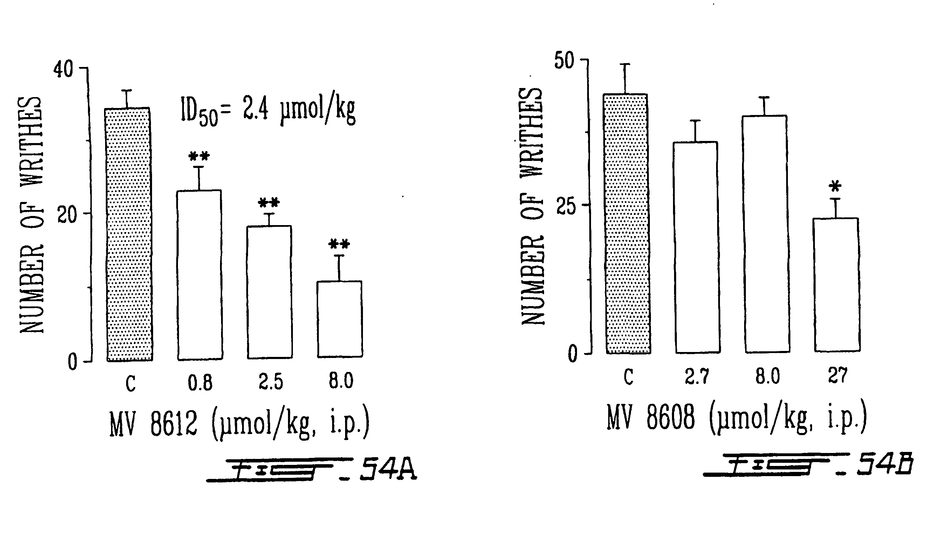

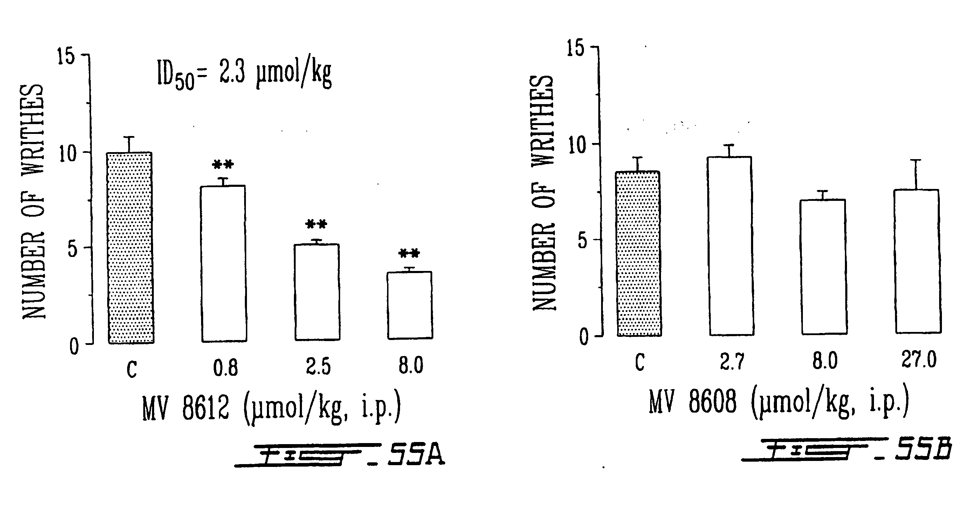

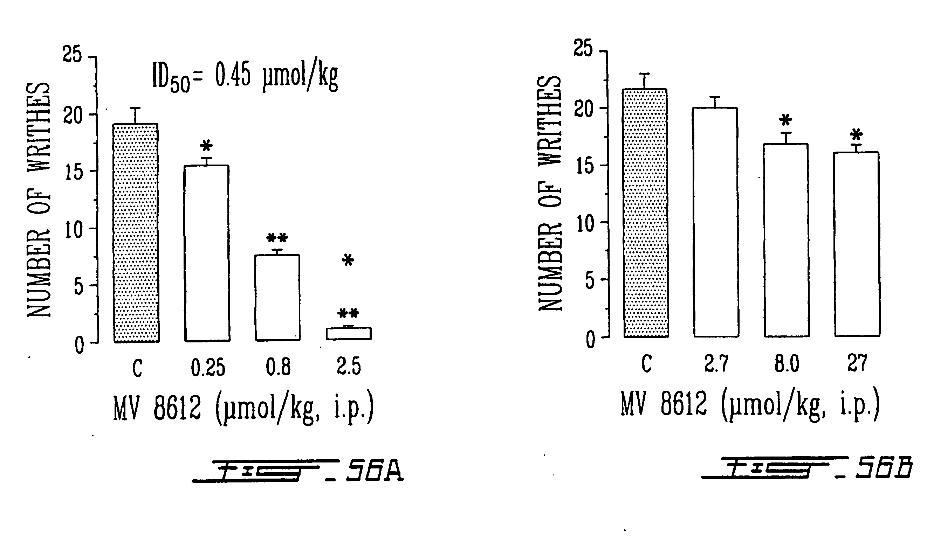

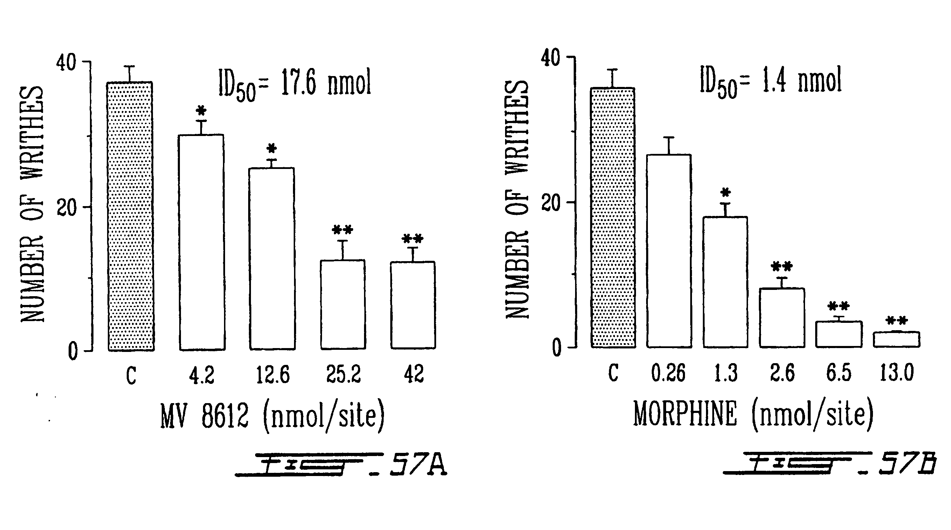

The antinociceptive actions of compound MV 8612 and MV 8608 were investigated in different models of nociception in mice as reported previously (Vaz et al., 1996, J. Pharmacol. Exp. Ther. 278:304-312). Compound MV 8612 (0.25 to 2.5 mol/kg, i.p.), given 30 min prior, caused dose-dependent inhibition of acetic acid (Figure 54A) acetylcholine (Figure 55A) and kaolin (Figure 56A)-induced writhing response in mice. However, MV 8612 was about 2-fold more potent against kaolin-induced pain. In contrast, compound MV 8608 (2.7 to 27 mol/kg, i.p.) caused only partial or even no antinociceptive action when tested in the same model of pain. When compared with morphine and indomethacin (Table 6), MV 8612 was about 2-fold more potent when assessed in the kaolin-induced writhes. Given intracerebroventricularly (i.c.v) MV 8612 (4.2 to 42 nmol/site), like morphine (0.26 to 13 nmol/site), caused a dose-related antinociception when assessed against acetic acid-induced writhes (Figure 57A and B). MV 8612 was about 12-fold less potent than morphine.

These results indicate that MV 8612, exhibits potent antinociceptive actions, comparable to those of morphine and indomethacin. Its antinociceptive property is elicited by its R-type Ca²⁺ channel blocking properties and is associated with its anti-bradykinin action, but appears to be unrelated to activation of opioid, β-adrenergic, serotonin or to the interaction with the nitric oxide pathway (results not shown).

**Table 6 - Antinociceptive potencies of MV 8612, morphine and indomethacin in mice.**

| | **ID**_{**50**} **µmol/kg, i.p** | | | |
|---|---|---|---|---|
| **DRUG** | **KAOLIN** | **ACETYLCHOLINE** | **ACETIC ACID** | **TAIL-FLICK** |
| MV 8612 | 0.4 (0.3-0.6) | 2.2 (2.0-2.3) | 2.4 (1.9-2.6) | no effect |
| MORPHINE | 1.4 (1.2-1.7) | 1.4 (1.0-1.8) | 1.3 (1.0-1.6) | 6.0(5.7-6.4) |
| INDOMETHACIN | 1.6 (1.4-1.8) | 0.8 (0.5-1.0) | 1.2 (0.9-1.4) | no effect |

| | | | | |
|---|---|---|---|---|
| each group represents the mean of 6 to 8 animals | | | | |

Although the present invention has been described hereinabove by way of preferred embodiments thereof, it can be modified, without departing from the spirit and nature of the subject invention as defined in the appended claims.

The present description refers to a number of documents, the content of which is herein incorporated by reference.

## Claims

1. A use of an effective amount of a saponin-like compound having the general formula EST, wherein: E and S define a saponin oligosugar portion, with E defining the terminal sugar portion thereof; and T is a pregnane-3β-ol steroid portion, or a pharmaceutically acceptable salt of said compound, together with a pharmaceutically acceptable carrier, for the manufacture of a medicament for specifically inhibiting overstimulation of a R-type Ca²⁺ channel in a warm blooded animal.

2. A use of an effective amount of a saponin-like compound having the general formula EST, wherein: E and S define a saponin oligosugar portion, with E defining the terminal sugar portion thereof; and T is a pregnane-3β-ol steroid portion, or a pharmaceutically acceptable salt of said compound, together with a pharmaceutically acceptable carrier, for the manufacture of a medicament for treating or preventing a disease or condition associated with an overstimulation of a R-type Ca²⁺ channel in a warm blooded animal.

3. A use according to claim 1 or 2, which treats or prevents a disease or condition associated with an overstimulation of R-type Ca²⁺ channels without significantly affecting the basal activity thereof.

4. A use of an effective amount of a saponin-like compound having the general formula EST, wherein: E and S define a saponin oligosugar portion, with E defining the terminal sugar portion thereof; and T is a pregnane-3β-ol steroid portion, or a pharmaceutically acceptable salt of said compound, together with a pharmaceutically acceptable carrier, for the manufacture of a medicament for treating or preventing a disease or condition associated with a sustained elevation of [Ca]_{c}, [Ca]ₙ, R-type Ca²⁺ channel blocking, and/or cytosolic and nuclear Ca²⁺ accumulation.

5. A use of an effective amount of a saponin-like compound having the general formula EST, wherein: E and S define a saponin oligosugar portion, with E defining the terminal sugar portion thereof; and T is a pregnane-3β-ol steroid portion, or a pharmaceutically acceptable salt of said compound, together with a pharmaceutically acceptable carrier, for the manufacture of a medicament for decreasing abnormal cell proliferation.

6. The use according to claim 5, wherein said cell is a cancer or tumor cell.

7. The use according to any one of claims 1 to 6, wherein S is selected from the group consisting of a tetra sugar, a monomeric sugar and an oligomeric sugar.

8. The use according to claim 7, wherein S is selected from the group consisting of α(1-4) (2-deoxy, 3-methoxy) -L-Iyxotetrose, α(1-4) (2-deoxy, 3-methoxy) L-xylotetrose, α(1-4) (2-deoxy, 3-methoxy)-L-arabinotetrose, α(1-4) (2-deoxy, 3-methoxy)-L-xylotetrose, α(1-4) (2-deoxy, 3-methoxy-L-ribopyranotetrose, α(1-4) (2-deoxy, 3 methoxy-L-sorbotetrose, α(1-4)-L-lyxotetrose, α(1-4)-L-xylotetrose, α(1-4)-L-arabinotetrose, α(1-4)-L-xylotetrose, α(1-4)-3, 4 methoxy-L-lyxotetrose, α(1-4)-3, 4 methoxy-L-xylotetrose, α(1-4)-3,4 methoxy-L-arabinotetrose, α(1-4)-3,4 methoxy-L-xylotetrose, α(a-4)-3,4 methoxy-L-ribopyranotetrose, α(1-4)-3,4 methoxy-L-sorbopyranotetrose, α(1-4)-L-lyxotetrose, α(1-4)-L-xylotetrose, α(1-4)-L-arabinotetrose, α(1-4)-L-ribopyranotetrose, oleandrose, and α(1-4)-L-sorbotetrose.

9. The use according to any one of claims 1 to 8, wherein E is selected from the group consisting of 4-acetoxy-3 methoxy-L-α-lyxose, 4-acetoxy-3-methoxy-L-α-xylose, 4-acetoxy-3-methoxy-L-α-arabinose, 4-acetoxy-3-methoxy-L-α-xylose, 4-acetoxy-3-methoxy-L-α-ribopyranose, and 4-acetoxy-3-methoxy-L-α-sorbose-acetoxy.

10. The use according to any one of claims 1 to 9, wherein T is selected from the group consisting of 5-pregnane-3-ol oxytricyclo- 15-ol, illustrol, 5-pregnane-3-ol-20-one, cholesterol, cholic acid, ergosterol, stigmasterol, androstenon, digitoxygenin, β-sitosterol, uvaol, ursolic acid, sarsasapogenin, 18,β -glycyrrhetinic acid, betulin, betulinic acid, oleanoic acid, and padocarpic acid.

11. The use according to any one of claims 1 to 10, wherein said compound comprises the general formula selected from the group consisting of formula I, IA, IB, II, III, IV, V, VI, VIIA and VIIB as set forth in Figs. 1A, 1B, 1C, 1D, 1E, 1F, 1G, 1H, and Figs. 2A and 2B.

12. The use according to claim 11, wherein said compound has the general formula of compound VIIA and compound VIIB, as set forth in Figs 2A and 2B, respectively.

13. The use according to any one of claims 1 to 12, wherein said compound is derivatized by one of alkylation, benzoylation, or glycosidation of the hydroxyl groups, chain of sugar extension or contraction.

14. The use according to claim 12, wherein said compound has the formula : 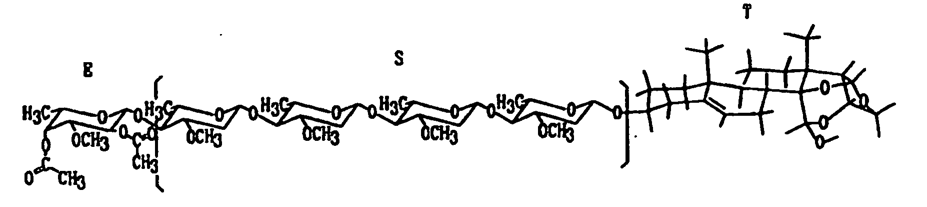 or a pharmaceutically acceptable salt thereof.

15. A use of an effective amount of a saponin-like compound obtained from a *Mandevilla* species, wherein said compound comprises a pregnane-3β-ol steroid portion having the structure selected from the group consisting of the structures set forth in Figure 1A, Figure 1B, Figure 1C, Figure 1D, Figure 1E, Figure 1F, Figure 1G and Figure 1H, or a pharmaceutically acceptable salt of said compound, together with a pharmaceutically acceptable carrier, for one of:
a) the manufacture of a medicament for specifically inhibiting overstimulation of a R-type Ca²⁺ channel in a warm blooded animal;
b) the manufacture of a medicament for treating or preventing a disease or condition associated with an overstimulation of a R-type Ca²⁺ channel in a warm blooded animal;
c) the manufacture of a medicament for treating or preventing a disease or condition associated with a sustained elevation of [Ca]_{c}, [Ca]ₙ, R-type Ca²⁺ channel blocking, and/or cytosolic and nuclear Ca²⁺ accumulation; or
d) the manufacture of a medicament for decreasing abnormal cell proliferation, in particular cancer or tumor cell proliferation.

16. The use according to claim 15, wherein said pregnane-3β-ol steroid portion is defined as a T portion which is part of a compound having a general formula EST, wherein E and S define a saponin oligosugar portion, with E defining the terminal sugar portion thereof.

17. The use according to claim 16, wherein wherein S is selected from the group consisting of a tetra sugar, a monomeric sugar and an oligomeric sugar, and E is selected from the group consisting of 4-acetoxy-3 methoxy-L-α-lyxose, 4-acetoxy-3-methoxy-L-α-xylose, 4-acetoxy-3-methoxy-L-α-arabinose, 4-acetoxy-3-methoxy-L-α-xylose, 4-acetoxy-3-methoxy-L-α-ribopyranose, and 4-acetoxy-3-methoxy-L-α-sorbose-acetoxy.

18. The use according to any one of claims 1 to 17, wherein said compound is isolated or purified.

19. The use according to claim 18, wherein said compound is substantially pure.

20. The use according to any one of claims 1-19, wherein said compound is a specific R-type calcium channel blocker.

## Patentansprüche

1. Verwendung einer wirksamen Menge einer Saponin-ähnlichen Verbindung mit der allgemeinen Formel EST, worin: E und S einen Saponin-Oligozucker-Teil definieren, wobei E den terminalen Zucker-Teil davon definiert; und T ist ein Pregnan-3β-ol-Steroid-Teil, oder ein pharmazeutisch annehmbares Salz dieser Verbindung, zusammen mit einem pharmazeutisch annehmbaren Träger, zur Herstellung eines Medikaments zur spezifischen Inhibierung der Überstimulierung eines Ca²⁺-Kanals vom R-Typ in einem warmblütigen Tier.

2. Verwendung einer wirksamen Menge einer Saponin-ähnlichen Verbindung mit der allgemeinen Formel EST, worin: E und S einen Saponin-Oligozucker-Teil definieren, wobei E den terminalen Zucker-Teil davon definiert; und T ist ein Pregnan-3β-of-Steroid-Teil, oder ein pharmazeutisch annehmbares Salz dieser Verbindung, zusammen mit einem pharmazeutisch annehmbaren Träger, zur Herstellung eines Medikaments zur Behandlung oder Prävention einer Krankheit oder eines Zustands, die mit einer Überstimulierung eines Ca²⁺ -Kanals vom R-Typ in Verbindung stehen in einem warmblütigen Tier.

3. Verwendung nach Anspruch 1 oder 2, welche eine Krankheit oder einen Zustand behandelt oder verhindert, die mit einer Überstimulierung von Ca²⁺-Kanälen vom R-Typ in Verbindung stehen, ohne dass die Grundaktivität desselben signifikant beeinträchtigt wird.

4. Verwendung einer wirksamen Menge einer Saponin-ähnlichen Verbindung mit der allgemeinen Formel EST, worin: E und S einen Saponin-Oligozucker-Teil definieren, wobei E den terminalen Zucker-Teil davon definiert; und T ist ein Pregnan-3β-ol-Sterold-Teil, oder ein pharmzeutisch annehmbares Salz dieser Verbindung, zusammen mit einem pharmazeutisch annehmbaren Träger, zur Herstellung eines Medikamentes zur Behandlung oder Prävention einer Krankheit oder eines Zustands, die mit einer verstärkten Anhebung der [Ca]_{c}, [Ca]ₙ, R-Typ-Ca²⁺-Kanal-Blockierung und/oder cytosolischer und nukleärer Ca²⁺-Akkumulierung verbunden sind.

5. Verwendung einer wirksamen Menge einer Saponin-ähnlichen Verbindung mit der allgemeinen Formel EST, worin: E und S einen Saponin-Oligozucker-Teil definieren, wobei E den terminalen Zucker-Teil davon definiert; und T ist ein Pregnan-3β-ol-Steroid-Teil oder ein pharmazeutisch annehmbares Salz dieser Verbindung, zusammen mit einem pharmazeutisch annehmbaren Träger, zur Herstellung eines Medikaments zur Verminderung abnormaler Zellproliferation.

6. Verwendung nach Anspruch 5, wobei die Zelle eine Krebs- oder Tumor-Zelle ist.

7. Verwendung nach einem der Ansprüche 1 bis 6, worin S ausgewählt ist aus der Gruppe bestehend aus Tetra-Zucker, einem monomeren Zucker und einem oligomeren Zucker.

8. Verwendung nach Anspruch 7, worin S ausgewählt ist aus der Gruppe bestehend aus α(1-4) (2-Deoxy, 3-Methoxy)-L-lyxotetrose, α(1-4) (2-Deoxy, 3-Methoxy) L-xylotetrose, α(1-4) (2-Deoxy, 3-Methoxy)-L-arabinotetrose, α(1-4) (2-Deoxy, 3-Methoxy)-L-xylotetrose, α(1-4) (2-Deoxy, 3-methoxy-L-ribopyranotetrose, α(1-4) (2-Deoxy, 3 methoxy-L-sorbotetrose, α(1-4)-L-lyxotetrose, α(1-4)-L-xylotetrose, α(1-4)-L-arabinotetrose, α(1-4)-L-xylotetrose, α(1-4)-3, 4 Methoxy-L-lyxotetrose, α(1-4)-3, 4 Methoxy-L-xylotetrose, α(1-4)-3,4 Methoxy-L-arabinotetrose, α(1-4)-3,4 Methoxy-L-xylotetrose, α(1-4)-3,4 Methoxy-L-ribopyranotetrose, α(1-4)-3,4 Methoxy-L-sorbopyranotetrose, α(1-4)-L-lyxotetrose, α(1-4)-L-xylotetrose, α(1-4)-L-arabinotetrose, α(1-4)-L-ribopyranotetrose, Oleandrose und α(1-4)-L-sorbotetrose.

9. Verwendung nach einem der Ansprüche 1 bis 8, worin E ausgewählt ist aus der Gruppe bestehend aus 4-Acetoxy-3-methoxy-L-α-lyxose, 4-Acetoxy-3-methoxy-L-α-xylose, 4-Acetoxy-3-methoxy-L-α-arabinose, 4-Acetoxy-3-methoxy-L-α-xylose, 4-Acetoxy-3-methoxy-L-α-ribopyranose und 4-Acetoxy-3-methoxy-L-α-sorbose-acetoxy.

10. Verwendung nach einem der Ansprüche 1 bis 9, worin T ausgewählt ist aus der Gruppe bestehend aus 5-Pregnan-3-ol-oxytricyclo-15-ol, Illustrol, 5-Pregnan-3-ol-20-on, Cholesterol, Cholinsäure, Ergosterol, Stigmasterol, Androstenon, Digitoxygenin, β-Sitosterol, Uvaol, Ursolinsäure, Sarsasapogenin, 18,β-Glycyrrhetininsäure, Betulin, Betulinsäure, Oleansäure und Padocarpinsäure.

11. Verwendung nach einem der Ansprüche 1 bis 10, worin die Verbindung die allgemeine Formel umfasst, ausgewählt aus der Gruppe bestehend aus der Formel I, IA, IB, II, III, IV, V, VI, VIIA und VIIB wie in den Figuren 1A, 1B, 1C, 1D, 1E, 1F, 1G, 1H und Figuren 2A und 2B angegeben.

12. Verwendung nach Anspruch 11, worin die Verbindung die allgemeine Formel der Verbindung VIIA und der Verbindung VIIB hat, wie in den Figuren 1A und 2B jeweils angegeben.

13. Verwendung nach einem der Ansprüche 1 bis 12, worin die Verbindung derivatisiert ist durch mindestens einen aus Alkylierung, Benzoylierung oder Glycosidierung der Hydroxylgruppen, Zuckerketten-Verlängerung oder - Verkürzung.

14. Verwendung nach Anspruch 12, worin die Verbindung die Formel hat 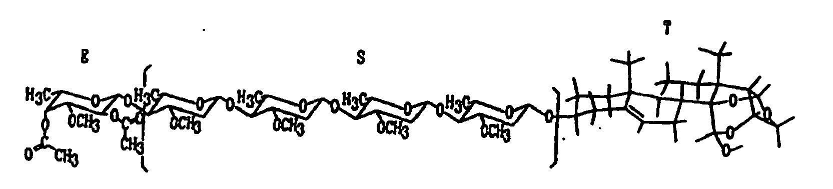 oder ein pharmazeutisch annehmbares Salz davon.

15. Verwendung einer wirksamen Menge einer Saponin-ähnlichen Verbindung, erhalten aus einer *Mandevilla-*Spezies, worin die Verbindung einen Pregnan-3β-ol-Steroid-Teil aufweist mit der Struktur ausgewählt aus der Gruppe bestehend aus den Strukturen wie angegeben in Figur 1A, Figur 1B, Figur 1C, Figur 1D, Figur 1E, Figur 1F, Figur 1G und Figur 1H, oder ein pharmazeutisch annehmbares Salz dieser Verbindung, zusammen mit einem pharmazeutisch annehmbaren Träger, für eines aus:
a) die Herstellung eines Medikamentes zur spezifischen Inhibierung der Überstimulierung eines Ca²⁺-Kanals vom R-Typ in einem warmblütigen Tier;
b) Die Herstellung eines Medikamentes zur Behandlung oder Prävention einer Krankheit oder eines Zustands, die mit einer Überstimulierung eines Ca²⁺-Kanals vom R-Typ in einem warmblütigen Tier in Verbindung stehen;
c) die Herstellung eines Medikamentes zur Behandlung oder Prävention einer Krankheit oder eines Zustandes, die mit einer verstärkten Anhebung von [Ca]_{c}, [Ca]ₙ, R-Typ Ca²⁺-Kanal-Blockierung und/oder cystosolischer und nukleärer Ca²⁺-Akkumulierung in Verbindung stehen; oder
d) die Herstellung eines Medikamentes zur Verminderung abnormaler Zellproliferation, insbesondere Krebs- oder Tumor-Zell-Proliferation.

16. Verwendung nach Anspruch 15, worin der Pregnan-3β-ol-Steroid-Teil definiert ist als ein T-Teil, der Teil einer Verbindung mit der allgemeinen Formel EST ist, worin E und S einen Saponin-Oligozucker-Teil definieren, wobei E den terminalen Zucker-Teil davon definiert.

17. Verwendung nach Anspruch 16, worin S ausgewählt aus der Gruppe bestehend aus einem Tetra-Zucker, einem monomeren Zucker und einem oligomeren Zucker, und E ausgewählt ist aus der Gruppe bestehend aus 4-Acetoxy-3-methoxy-L-α-lyxose, 4-Acetoxy-3-methoxy-L-α-xylose, 4-Acetoxy-3-methoxy-L-α-arabinose, 4-Acetoxy-3-methoxy-L-α-xylose, 4-Acetoxy-3-methoxy-L-α-ribopyranose und 4-Acetoxy-3-methoxy-L-α-sorbose-acetoxy.

18. Verwendung nach einem der Ansprüche 1 bis 17, worin die Verbindung isoliert oder gereinigt ist.

19. Verwendung nach Anspruch 18, worin die Verbindung im wesentlichen rein ist.

20. Die Verwendung nach einem der Ansprüche 1 bis 19, worin die Verbindung ein spezifischer R-Typ-Kalziumkanalblocker ist.

## Revendications

1. Utilisation d'une quantité efficace d'un composé de type saponine ayant la formule générale EST, dans laquelle : E et S définissent une portion oligosucre saponine, avec E définissant sa portion sucre terminale ; et T est une portion prégnane-3β-ol stéroïde, ou un sel pharmaceutiquement acceptable dudit composé, conjointement avec un support pharmaceutiquement acceptable pour la fabrication d'un médicament destiné spécifiquement à inhiber la surstimulation d'un canal de Ca²⁺ de type R dans un animal au sang chaud.

2. Utilisation d'une quantité efficace d'un composé de type saponine ayant la formule générale EST, dans laquelle : E et S définissent une portion oligosucre saponine, avec E définissant sa portion sucre terminale ; et T est une portion prégnane-3β-ol stéroïde, ou un sel pharmaceutiquement acceptable dudit composé, conjointement avec un support pharmaceutiquement acceptable pour la fabrication d'un médicament destiné au traitement ou à la prévention d'une maladie ou condition liée à la surstimulation d'un canal de Ca²⁺ de type R dans un animal au sang chaud.

3. Utilisation selon la revendication 1 ou 2, qui traite ou prévient une maladie ou condition associée à une surstimulation du canal de Ca²⁺ de type R sans affecter sensiblement son activité basale.

4. Utilisation d'une quantité efficace d'un composé de type saponine ayant la formule générale EST, dans laquelle : E et S définissent une portion oligosucre saponine, avec E définissant sa portion sucre terminale ; et T est une portion prégnane-3β-ol stéroïde, ou un sel pharmaceutiquement acceptable dudit composé, conjointement avec un support pharmaceutiquement acceptable pour la fabrication d'un médicament pour le traitement ou la prévention d'une maladie ou condition liée à une élévation soutenue de [Ca]_{c}, [Ca]ₙ, blocage du canal de Ca²⁺ de type R et/ou accumulation cytosolique et nucléaire de Ca²⁺.

5. Utilisation d'une quantité efficace d'un composé de type saponine ayant la formule générale EST, dans laquelle : E et S définissent une portion oligosucre saponine, avec E définissant sa portion sucre terminale ; et T est une portion prégnane-3β-ol stéroïde, ou un sel pharmaceutiquement acceptable dudit composé, conjointement avec un support pharmaceutiquement acceptable pour la fabrication d'un médicament destiné à réduire la prolifération cellulaire anormale.

6. Utilisation selon la revendication 5, dans laquelle ladite cellule est une cellule cancéreuse ou tumorale.

7. Utilisation selon l'une quelconque des revendications 1 à 6, dans laquelle S est choisi dans le groupe consistant en un tétra-sucre, un sucre monomère et un sucre oligomère.

8. Utilisation selon la revendication 7, dans laquelle S est choisi dans le groupe consistant en α(1-4) (2-désoxy, 3-méthoxy)-L-lyxotétrose, α(1-4)(2-désoxy, 3-méthoxy)-L-xylotétrose, α(1-4)(2-désoxy, 3-méthoxy)-L-arabinotétrose, α(1-4)(2-désoxy, 3-méthoxy)-L-xylotétrose, α(1-4)(2-désoxy, 3-méthoxy)-L-ribopyranotétrose, α(1-4)(2-désoxy, 3-méthoxy)-L-sorbotétrose, α(1-4)-L-lyxotétrose, α(1-4)-L-xylotétrose, α(1-4)-L-arabinotétrose, α(1-4)-L-xylotétrose, α(1-4)-3,4-méthoxy-L-lyxotétrose, α(1-4)-3, 4-méthoxy-L-xylotétrose, α(1-4)-3,4-méthoxy-L-arabinotétrose, α(1-4)-3,4-méthoxy-L-xylotétrose, α(1-4)-3,4-méthoxy-L-ribopyranotétrose, α(1-4)-3,4-méthoxy-L-sorbopyranotétrose, α(1-4)-L-lyxotétrose, α(1-4)-L-xylotétrose, α(1-4)-L-arabinotétrose, α(1-4)-L-ribopyranotétrose, oléandrose et α(1-4)-L-sorbotétrose.

9. Utilisation selon l'une quelconque des revendications 1 à 8, dans laquelle E est sélectionné dans le groupe consistant en 4-acétoxy-3-méthoxy-L-α-lyxose, 4-acétoxy-3-méthoxy-L-α-xylose, 4-acétoxy-3-méthoxy-L-α-arabinose, 4-acétoxy-3-méthoxy-L-α-xylose, 4-acétoxy-3-méthoxy-L-α-ribopyranose et 4-acétoxy-3-méthoxy-L-α-sorbose-acétoxy.

10. Utilisation selon l'une quelconque des revendications 1 à 9, dans laquelle T est choisi dans le groupe consistant en 5-prégnane-3-ol oxytricyclo-15-ol, illustrol, 5-prégnane-3-ol-20-one, cholestérol, acide cholique, ergostérol, stigmastérol, androsténone, digitoxygénine, β-sitostérol, uvaol, acide ursolique, sarsasapogénine, acide 18-β-glycyrrhétinique, bétuline, acide bétulinique, acide oléanoïque et acide padocarpique.

11. Utilisation selon l'une quelconque des revendications 1 à 10, dans laquelle ladite composition comprend la formule générale choisie dans le groupe consistant en formules I, IA, IB, II, III, IV, V, VI, VIIA et VIIB telles que définies dans les Figures 1A, 1B, 1C, 1D, 1E, 1F, 1G, 1H et les Figures 2A et 2B.

12. Utilisation selon la revendication 11, dans laquelle ledit composé a la formule générale du composé VIIA et du composé VIIB, telle que définie sur les Figures 1A et 2B respectivement.

13. Utilisation selon l'une quelconque des revendications 1 à 12, dans laquelle ledit composé est dérivé par l'une de l'alkylation, benzoylation ou glycosidation des groupes hydroxyle, chaîne de contraction ou d'extension sucre.

14. Utilisation selon la revendication 12, dans laquelle ledit composé a la formule : 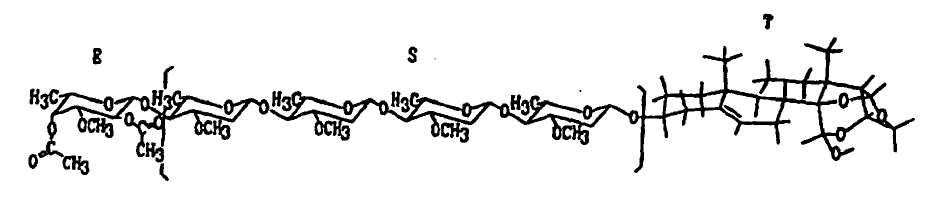 ou son sel pharmaceutiquement acceptable.

15. Utilisation d'une quantité efficace d'un composé de type saponine obtenu à partir d'espèces *Mandevilla,* dans lequel ledit composé comprend une portion de prégnane-3β-ol stéroïde ayant la structure choisie dans le groupe consistant en structures définies sur les Figures 1A, 1B, 1C, 1D, 1E, 1F, 1G et la Figure 1H, ou un sel pharmaceutiquement acceptable dudit composé, conjointement avec un support pharmaceutiquement acceptable pour l'utilisation de :
a) la fabrication d'un médicament pour inhiber spécifiquement la surstimulation d'un canal de Ca²⁺ de type R chez un animal au sang chaud ;
b) la fabrication d'un médicament pour le traitement ou la prévention d'une maladie ou condition associée à une surstimulation d'un canal de Ca²⁺ de type R chez un animal au sang chaud ;
c) la fabrication d'un médicament pour le traitement ou la prévention d'une maladie ou condition liée à une élévation soutenue de [Ca]_{c}, [Ca]ₙ, blocage du canal de Ca²⁺ de type R et/ou accumulation cytosolique et nucléaire de Ca²⁺ ; ou
d) la fabrication d'un médicament pour diminuer la prolifération cellulaire anormale, en particulier la prolifération de cellules cancéreuses ou tumorales.

16. Utilisation selon la revendication 15, dans laquelle ladite portion de prégnane-3β-ol stéroïde est définie en tant que portion T qui fait partie d'un composé ayant une formule générale EST, dans laquelle E et S définissent une portion oligosucre saponine, avec E définissant sa portion sucre terminale.

17. Utilisation selon la revendication 16, dans laquelle S est choisi dans le groupe consistant en un sucre tétra, un sucre monomère et un sucre oligomère, et E est choisi dans le groupe consistant en 4-acétoxy-3-méthoxy-L-α-lyxose, 4-acétoxy-3-méthoxy-L-α-xylose, 4-acétoxy-3-méthoxy-L-α-arabinose, 9-acétoxy-3-méthoxy-L-α-xylose, 4-acétoxy-3-méthoxy-L-α-ribopyranose et 4-acétoxy-3-méthoxy-L-α-sorbose-acétoxy.

18. Utilisation selon l'une quelconque des revendications 1 à 17, dans laquelle le composé est isolé ou purifié.

19. Utilisation selon la revendication 18, dans laquelle ledit composé est sensiblement pur.

20. Utilisation selon l'une quelconque des revendications 1 à 19, dans laquelle le composé est un agent bloquant spécifique du canal de calcium type R.
